# EUROPEAN PATENT SPECIFICATION

(11) **EP 2 627 352 B1**
(45) Date of publication and mention of the grant of the patent: **10.05.2017**
(21) Application number: 11768058.7
(22) Date of filing: 14.10.2011
(51) Int. Cl.: A61K 39/245, C07K 14/045

(54) **CYTOMEGALOVIRUS GB ANTIGEN**
CYTOMEGALOVIRUS GB ANTIGEN
ANTIGÈNE GB DE CYTOMÉGALOVIRUS

(30) Priority: 15.10.2010 US 393413 P
(43) Date of publication of application: 21.08.2013
(73) Proprietor: GlaxoSmithKline Biologicals S.A., 1330 Rixensart (BE)
(72) Inventor: BAUDOUX, Guy, Jean, Marie, Fernand, Pierre, B-1330 Rixensart (BE); BLAIS, Normand, Laval, Québec H7V 3S8 (CA); MARCHAND, Martine, B-1330 Rixensart (BE)
(74) Representative: Furstoss, Olivia Aline
(86) International application number: PCT/EP2011/068040
(87) International publication number: WO 2012/049317

(56) References cited:
- WO-A1-2009/037359
- WO-A2-96/04382
- SINGH J ET AL: "CHARACTERIZATION OF A PANEL OF INSERTION MUTANTS IN HUMAN CYTOMEGALOVIRUS GLYCOPROTEIN B", JOURNAL OF VIROLOGY, THE AMERICAN SOCIETY FOR MICROBIOLOGY, US, vol. 74, no. 3, 1 February 2000 (2000-02-01), pages 1383-1392, XP001118928, ISSN: 0022-538X, DOI: 10.1128/JVI.74.3.1383-1392.2000
- M. BACKOVIC ET AL: "Hydrophobic Residues That Form Putative Fusion Loops of Epstein-Barr Virus Glycoprotein B Are Critical for Fusion Activity", JOURNAL OF VIROLOGY, vol. 81, no. 17, 1 September 2007 (2007-09-01), pages 9596-9600, XP55015633, ISSN: 0022-538X, DOI: 10.1128/JVI.00758-07
- SPECKNER ANDREA ET AL: "The antigenic domain 1 of human cytomegalovirus glycoprotein B contains an intramolecular disulphide bond", JOURNAL OF GENERAL VIROLOGY, vol. 81, no. 11, November 2000 (2000-11), pages 2659-2663, XP002672059, ISSN: 0022-1317
- SCHEFFCZIK<A> H ET AL: "Multimerization potential of the cytoplasmic domain of the human cytomegalovirus glycoprotein B", FEBS LETTERS, ELSEVIER, AMSTERDAM, NL, vol. 506, no. 2, 5 October 2001 (2001-10-05), pages 113-116, XP004308710, ISSN: 0014-5793, DOI: 10.1016/S0014-5793(01)02891-5
- BRITT WILLIAM J ET AL: "Antigenic domain 1 is required for oligomerization of human cytomegalovirus glycoprotein B", JOURNAL OF VIROLOGY, vol. 79, no. 7, April 2005 (2005-04), pages 4066-4079, XP055022639, ISSN: 0022-538X

## Description

### TECHNICAL FIELD

The present invention relates to the field of immunology. In particular, the invention provides compositions, such as for example, vaccines, as well as methods for eliciting an immune response specific for Cytomegalovirus (CMV).

### TECHNICAL BACKGROUND

The human cytomegalovirus (HCMV) is a ubiquitous DNA virus belonging to the Herpes virus family. HCMV is made up of a DNA core, an outer capsid and covered by a lipid membrane which incorporates virus specific glycoproteins.

HCMV is endemic in most part of the world. Primary infection however normally results in subclinical disease after which the virus becomes latent retaining the capacity to reactivate at any later time. Among two populations, HCMV is however responsible for serious medical conditions. HCMV is a major cause of congenital defects in newborns infected in utero. It is the most common cause of congenital infection in the developed world. Congenital infection refers to infection transmitted from mother to foetus prior to birth of the newborn. Among congenitally infected newborns, 5-10% have major clinical symptoms at birth, such as microcephaly, intracranial calcifications, and hepatitis. Many infants with congenital HCMV infection are asymptomatic at birth. However, follow-up studies have shown that 15% of such infants will have sequelae such as hearing loss or central nervous system abnormalities causing, in particular, poor intellectual performance.

The second population at risk are immunocompromised patients, such as those suffering from HIV infection and those undergoing transplantations. In this situation, the virus becomes an opportunistic pathogen and causes severe disease with high morbidity and mortality. The clinical disease causes a variety of symptoms including fever, hepatitis, pneumonitis and infectious mononucleosis.

To address HCMV-associated diseases, candidate vaccines are developed including live attenuated vaccines and subunit vaccines. In particular, recently a subunit vaccine comprising a modified glycoprotein B (gB) deleted from its transmembrane domain combined with the MF59™ emulsion was tested in a phase II clinical trial (WO 2009/037359, N. Engl. J. Med. 2009; 360: 1191-9).

### SUMMARY OF THE INVENTION

In a first aspect of the present invention, there is provided a cytomegalovirus (CMV) gB polypeptide comprising a non-functional transmembrane (TM) domain and at least a portion of a gB protein extracellular domain comprising a fusion loop 1 (FL1) domain defined by amino acids Y.I.Y located at position 155-157 of the sequence set forth in SEQ ID NO:1, or at a corresponding position in other CMV gB polypeptides, and a fusion loop 2 (FL2) domain defined by amino acids W.L.Y located at position 240-242 of the sequence set forth in SEQ ID NO:1, or at a corresponding position in other CMV gB polypeptides, wherein at least one of the FL1 and FL2 domains comprises at least one amino acid deletion or substitution.

In a second aspect, there is provided a CMV gB polypeptide having the sequence selected from the group consisting of the sequences set forth in: SEQ ID NO:3, SEQ ID NO:4, SEQ ID NO:5, SEQ ID NO:10, SEQ ID NO:12, SEQ ID NO:14, SEQ ID NO:16, SEQ ID NO:18, SEQ ID NO:19, SEQ ID NO:20 and SEQ ID NO:21.

In a third aspect, there is provided an immunogenic composition comprising the CMV gB polypeptides of the invention admixed with a suitable pharmaceutical carrier.

In a fourth aspect, there is provided a polynucleotide that encodes the CMV gB polypeptides of the invention.

In a fifth aspect, there is provided a polynucleotide having the sequence selected from the group consisting of the sequences set forth in: SEQ ID NO:6, SEQ ID NO:7, SEQ ID NO:8, SEQ ID NO:9, SEQ ID NO:11, SEQ ID NO:13, SEQ ID NO:15, and SEQ ID NO:17.

In a further aspect, there is provided a recombinant vector comprising the polynucleotides of the invention.

In a still further aspect, there is provided a host cell transformed with the recombinant vector of the invention.

In a still further aspect, there is provided the CMV gB polypeptides of the invention for use in the prevention and/or treatment of CMV infection.

### DESCRIPTION OF DRAWINGS

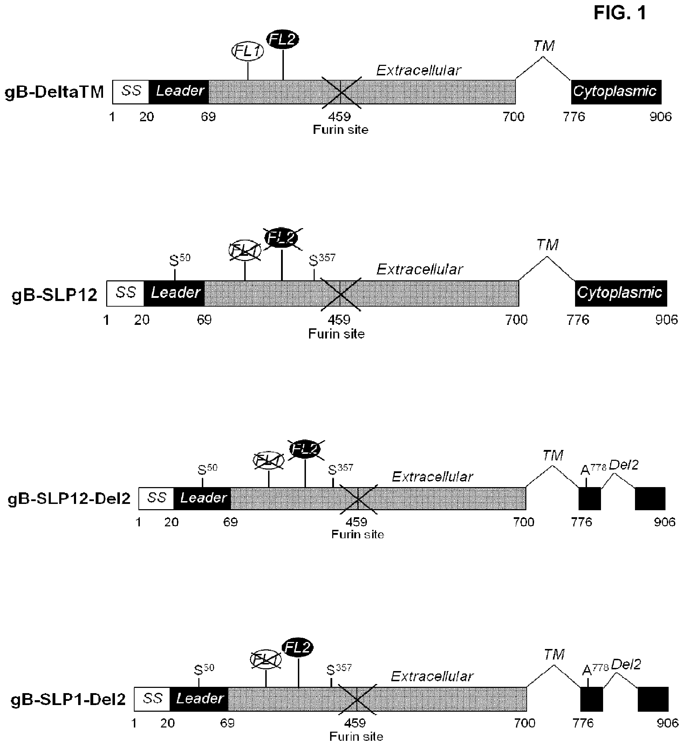

**FIG. 1** is a schematic representation of the CMV gB polypeptide derived from the AD169 strain lacking the transmembrane domain (gB-DeltaTM), as well as three exemplary CMV gB polypeptides disclosed therein : (i) CMV gB deleted from the transmembrane domain wherein both fusion loops FL1 and FL2 are mutated (gB-SLP12) (see SEQ ID NO:3), (ii) CMV gB deleted from the transmembrane domain wherein the mutation of both fusion loops FL1 and FL2 is combined with the deletion of a region (Del2) in the C-terminal cytoplasmic domain which comprises a proline-rich domain, a conserved hydrophobic pattern and a highly hydrophilic, negatively charged region (gB-SLP12-Del2) (see SEQ ID NO:4), and (iii) CMV gB deleted from the transmembrane domain wherein the fusion loop FL1 mutation is combined with the deletion of the above-described region (Del2) in the C-terminal cytoplasmic domain of the polypeptide (gB-SLP1-Del2) (see SEQ ID NO:5). gB-SLP12, gB-SLP12-Del2 and gB-SLP1-Del2 all comprise two additional point mutations substituting arginine (R) with serine (S) at position 50 and 357. gB-SLP12-Del2 and gB-SLP1-Del2 both comprise the further point mutation substituting a cysteine (C) by an alanine (A) at position 778. TM is for transmembrane domain. FL1 is for Fusion Loop 1. FL2 is for Fusion Loop 2. The Del2 deletion starts with amino acid 825 and ends with amino acid 877, *i.e.* these two amino acids are the first one and the last one, respectively, of the region which is deleted. The indicated numbers refer to the position of amino acids of the sequence of CMV gB originating from the AD169 strain and set forth in SEQ ID NO: 1.

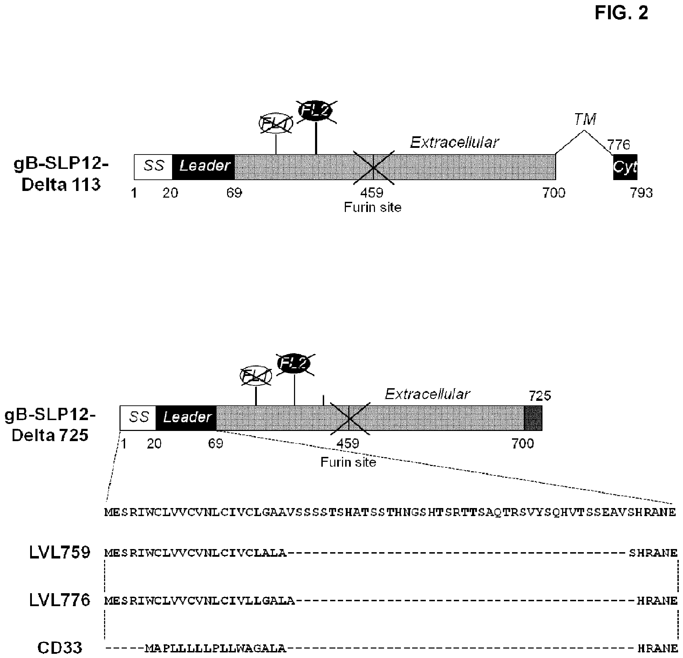

**FIG. 2** is a schematic representation of further exemplary CMV gB polypeptides disclosed therein. All the polypeptides comprise mutated fusion loops FL1 and FL2. gB-SLP12-Delta113 is additionally deleted from both the transmembrane domain and the C-terminal part of the cytoplasmic domain, *i.e.* the amino acids 794 to 906. gB-SLP12-Delta725 is additionally deleted from the entire cytoplasmic domain and retains part of the transmembrane domain. LVL759 (or gB-SLP12-Delta725-LVL759), LVL776 (or gB-SLP12-Delta725-LVL776) and CD33 (or gB-SLP12-Delta725-CD33) have a backbone identical to gB-SLP12-Delta725 except for the N-terminal end, wherein the amino acid sequence varies, as indicated. The indicated numbers refer to the position of amino acids of the sequence of CMV gB originating from the AD169 strain and set forth in SEQ ID NO:1.

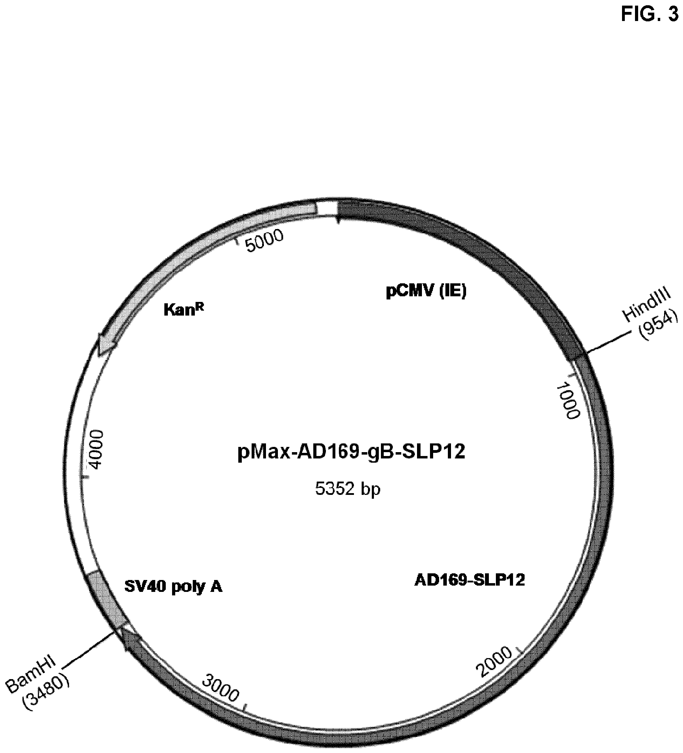

**FIG. 3** shows the pMax plasmid map corresponding to the construct gB-SLP12.

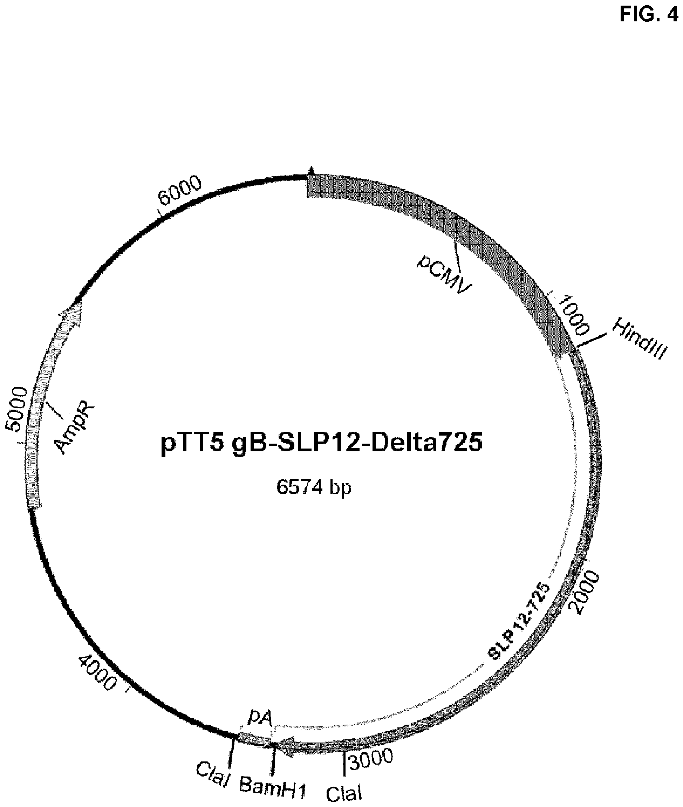

**FIG. 4** shows the pTT5 plasmid map corresponding to the construct gB-SLP12-Delta725.

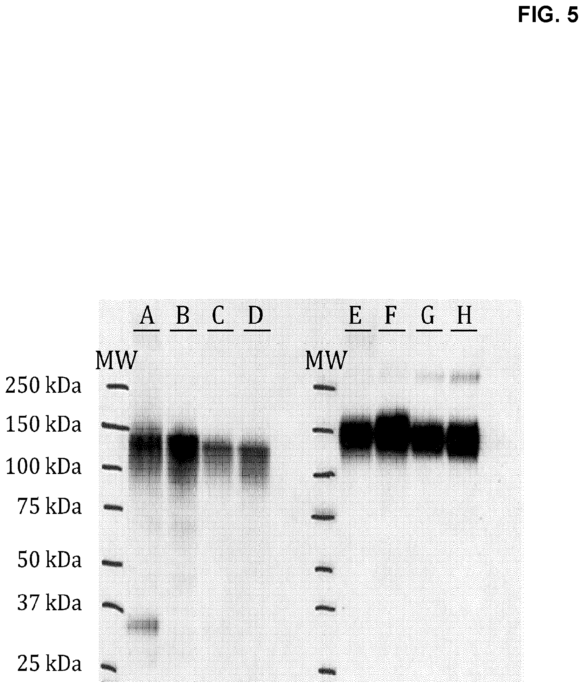

**FIG. 5** shows the expression and secretion level of the three different exemplary gB polypeptides recombinantly expressed in CHO cells, as compared to the expression and secretion level of the CMV gB polypeptide deleted from the transmembrane domain of the prior art. Shown is a Western Blot analysis using an anti-gB antibody indicating the level of gB protein (located on the blot between 150 kDa and 100 kDa) present in both cellular fractions (representing the unsecreted form of gB) and culture supernatants (representing the secreted form of gB) arising from distinct cultures transfected with the indicated gB constructs. **Lane MW:** Molecular Weight ladder, **Lane A:** cellular fraction (C-Fr) of a culture transfected with gB-DeltaTM, **Lane B:** C-Fr of a culture transfected with gB-SLP12, **Lane C:** C-Fr of a culture transfected with gB-SLP1-Del2, **Lane D:** C-Fr of a culture transfected with gB-SLP12-Del2, **Lane E:** culture supernatant (C-Sn) of a culture transfected with gB-DeltaTM, **Lane F:** C-Sn of a culture transfected with gB-SLP12, **Lane G:** C-Sn of a culture transfected with gB-SLP1-Del2, **Lane H:** C-Sn of a culture transfected with gB-SLP12-Del2.

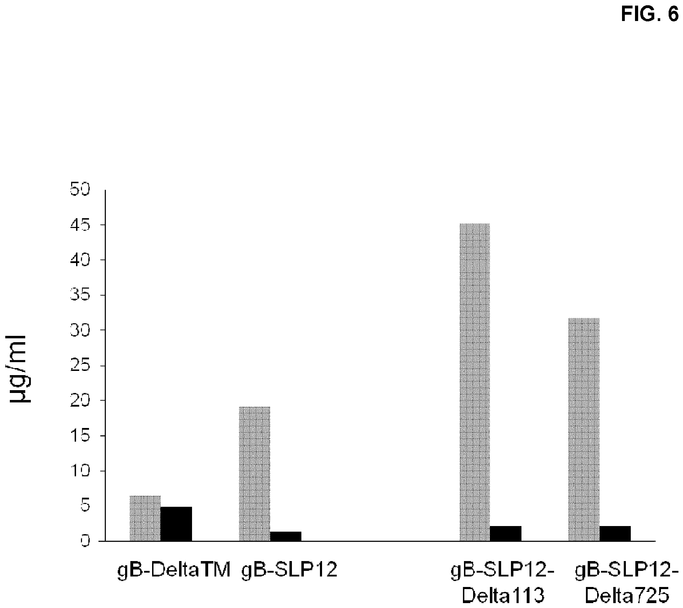

**FIG. 6** shows the expression and secretion level of three different exemplary gB polypeptides in accordance with the present invention, gB-SLP12, gB-SLP12-Delta113, and gB-SLP12-Delta725, which were recombinantly expressed in CHO cells, as compared to the expression and secretion level of the prior art CMV gB polypeptide deleted from the transmembrane domain. Shown is a quantitative Elisa assay using an anti-gB antibody displaying the level of gB protein present in both cellular fractions (black bars - representing the unsecreted form of gB) and culture supernatants (grey bars - representing the secreted form of gB).

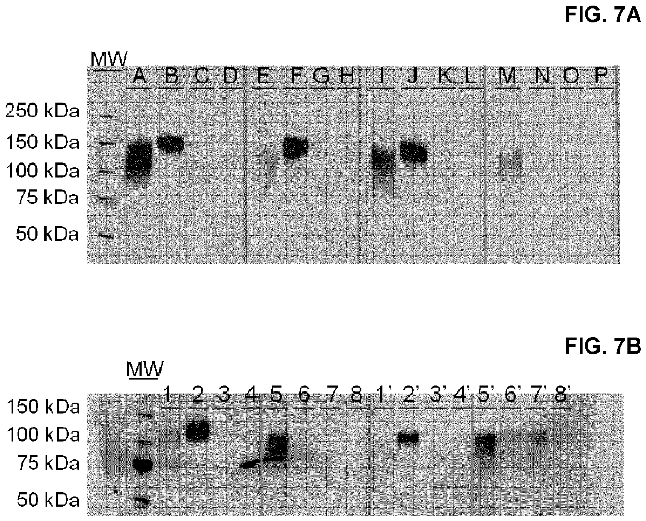

**FIG. 7** shows the expression and secretion level of further exemplary gB polypeptides in accordance with the present invention which were recombinantly expressed in CHO cells. Shown are three Western Blot analysis using an anti-gB antibody displaying the level of gB protein (located on the blot between 150 kDa and 100 kDa) present in both cellular fractions (C-Fr, representing the unsecreted form of gB) and culture supernatants (C-Sn, representing the secreted form of gB) arising from distinct cultures transfected with the indicated gB constructs. **FIG. 7A** **- Lanes A, B, E, F, I, J, M** and **N** represent the amount of protein present in 2600 total cells. **Lanes C, D, G, H, K, L, O** and **P** represent the amount of protein present in 520 total cells. **Lane MW:** Molecular Weight ladder, **Lanes A** and **C:** C-Fr of a culture transfected with gB-DeltaTM, **Lanes B** and **D:** C-Sn of a culture transfected with gB-DeltaTM, **Lanes E** and **G:** C-Fr of a culture transfected with gB-SLP12, **Lanes F** and **H:** C-Sn of a culture transfected with gB-SLP12, **Lanes I** and **K:** C-Fr of a culture transfected with gB-SLP12-Delta 725, **Lanes J** and **L:** C-Sn of a culture transfected with gB-SLP12-Delta725, **Lanes M** and **O:** C-Fr of a culture transfected with gB-Delta725 (having intact Fusion Loops), **Lanes N and P:** C-Sn of a culture transfected with gB-Delta725 (having intact Fusion Loops). **FIG. 7B** **- Lanes 1, 2, 5, 6, 1', 2', 5'** and **6'** represent the amount of protein present in 2600 total cells. **Lanes 3, 4, 7, 8, 3', 4', 7'** and **8'** represent the amount of protein present in 520 total cells. **Lane MW:** Molecular Weight ladder, **Lanes 1** and **3:** C-Fr of a culture transfected with gB-SLP12-Delta725, **Lanes 2** and **4:** C-Sn of a culture transfected with gB-SLP12-Delta725, **Lanes 5** and **7:** C-Fr of a culture transfected with gB-Delta725 (having intact Fusion Loops), **Lanes 6** and **8:** C-Sn of a culture transfected with gB-Delta725 (having intact Fusion Loops), **Lanes 1'** and **3':** C-Fr of a culture transfected with gB-SLP12-Delta113, **Lanes 2'** and **4':** C-Sn of a culture transfected with gB-SLP12-Delta113, **Lanes 5'** and **7':** C-Fr of a culture transfected with gB-Delta113 (having intact Fusion Loops), **Lanes 6'** and **8':** C-Sn of a culture transfected with gB-SLP12-Delta113 (having intact Fusion Loops).

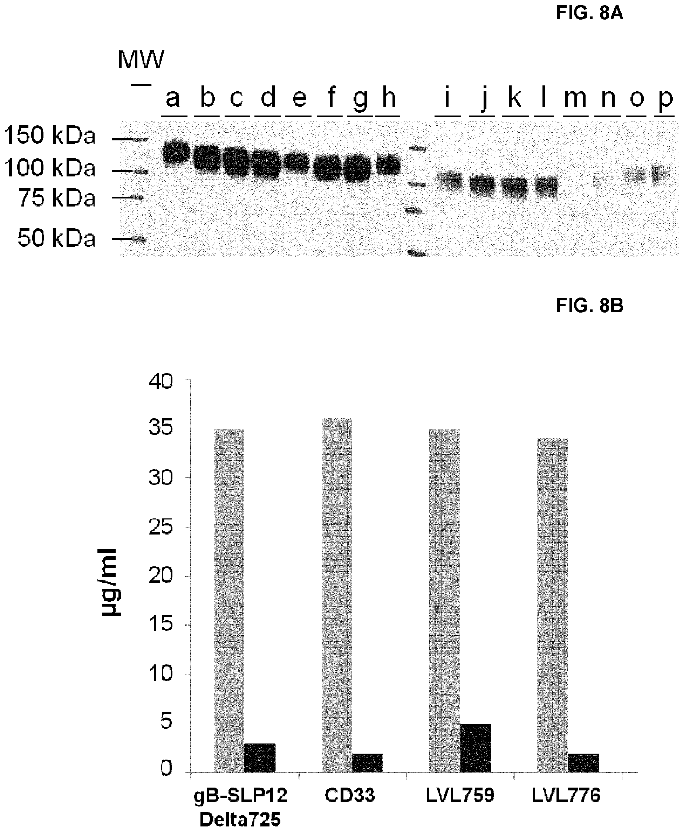

**FIG. 8** shows the expression and secretion level of further exemplary gB polypeptides in accordance with the present invention which were recombinantly expressed in CHO cells. **FIG 8A** **-** Shown is a Western Blot analysis using an anti-gB antibody displaying the level of gB protein (located on the blot between 150 kDa and 100 kDa) present in both cellular fractions (C-Fr, representing the unsecreted form of gB) and culture supernatants (C-Sn, representing the secreted form of gB) arising from distinct cultures transfected with the indicated gB constructs. Lanes a, **b, c, d, i, j, k** and **I** represent the amount of protein present in 11700 total cells, while lanes e, **f, g, h, m, n, o** and **p** represent the amount of protein present in 5100 total cells. **Lane MW:** Molecular Weight ladder, **Lanes a** and **e:** C-Sn of a culture transfected with gB-SLP12-Delta725, **Lanes i** and **m:** C-Fr of a culture transfected with gB-SLP12-Delta725, **Lanes b** and **f:** C-Sn of a culture transfected with CD33, **Lanes j** and **n:** C-Fr of a culture transfected with CD33, **Lanes c** and **g:** C-Sn of a culture transfected with LVL759, **Lanes k** and **o:** C-Fr of a culture transfected with LVL759, **Lanes d** and **h:** C-Sn of a culture transfected with LVL776, **Lanes I** and **p:** C-Fr of a culture transfected with LVL776. **FIG. 8B** **-** Shown is a quantitative Elisa assay using an anti-gB antibody displaying the level of gB protein present in both cellular fractions (black bars - representing the unsecreted form of gB) and culture supernatants (grey bars - representing the secreted form of gB).

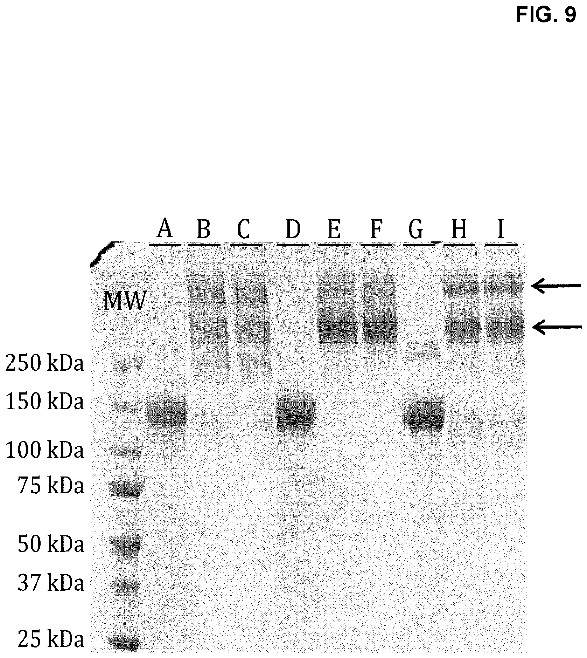

**FIG. 9** shows the product profile of the polypeptides gB-DeltaTM, gB-SLP12 and gB-SLP1-Del2, as analysed by glutaraldehyde-induced crosslinking, upon their recombinant expression in CHO cells. Shown is an image of a polyacrylamide gel. Arrows indicate multimers of different sizes. Increasing doses (0, 0.5, 1%) of glutaraldehyde have been used. **Lane MW:** Molecular Weight ladder, **Lane A:** gB-DeltaTM - 0% glutaraldehyde, **Lane B:** gB-DeltaTM - 0.5% glutaraldehyde, **Lane C:** gB-DeltaTM - 1% glutaraldehyde, **Lane D:** gB-SLP12 - 0% glutaraldehyde, **Lane E:** gB-SLP12 - 0.5% glutaraldehyde, **Lane F:** gB-SLP12 - 1% glutaraldehyde, **Lane G:** gB-SLPl-Del2 - 0% glutaraldehyde, **Lane H:** gB-SLP1-Del2 - 0.5% glutaraldehyde, and **Lane I:** gB-SLP1-Del2 - 1% glutaraldehyde.

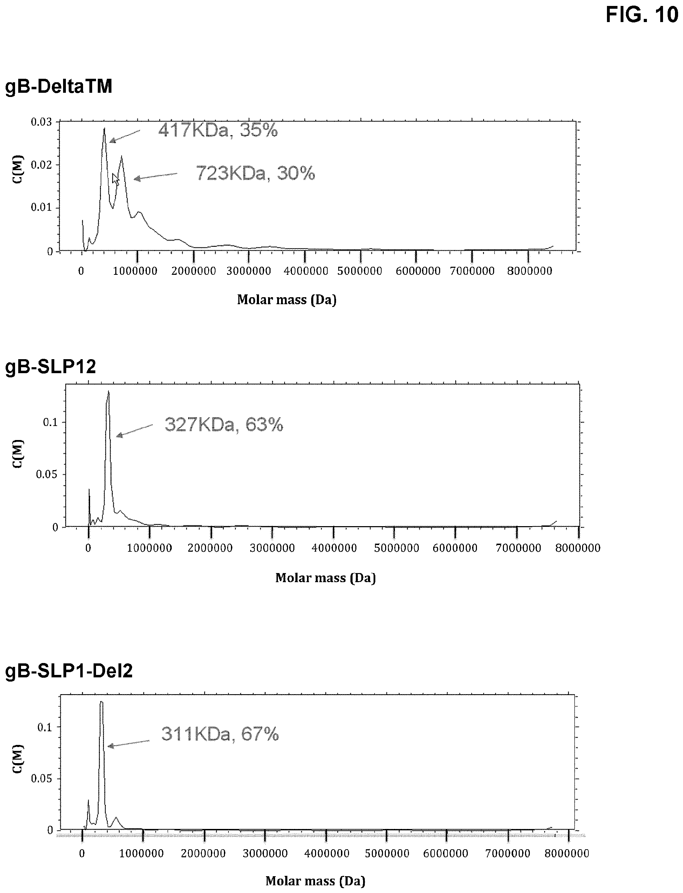

**FIG. 10** shows the product profile of the polypeptides gB-DeltaTM, gB-SLP12 and gB-SLP1-Del2, as analysed by analytical ultracentrifugation (AUC), upon their recombinant expression in CHO cells. Peaks represent multimers of varying size, as indicated. The percentage of identified multimers within each polypeptide population is also specified, as indicated.

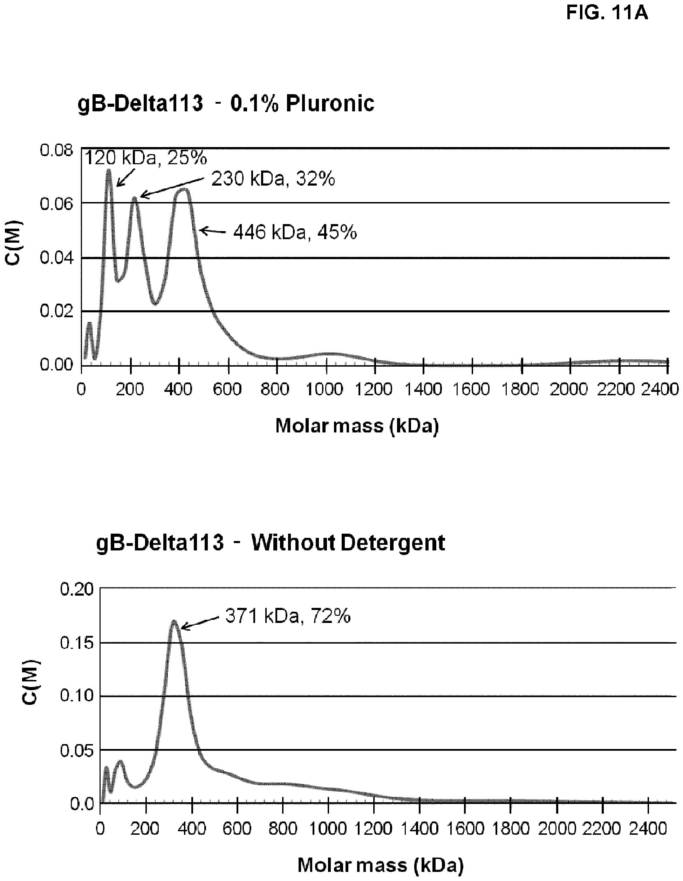

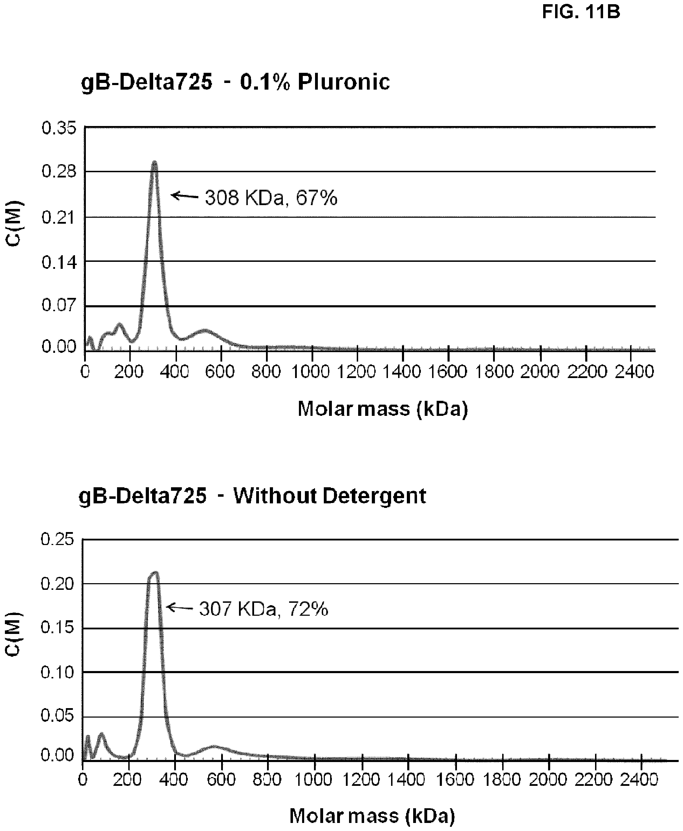

**FIG. 11** shows the product profile of further exemplary CMV gB polypeptides, as indicated and as analysed by analytical ultracentrifugation (AUC), upon their recombinant expression in CHO cells. Peaks represent multimers of varying size, as indicated. The percentage of identified multimers within each polypeptide population is also specified, as indicated. **FIG**. **11A** - displays the product profile of the gB-SLP12-Delta113 polypeptide (indicated as gB-Delta113 in the figure) obtained in the presence or absence of Pluronic. **FIG. 11B** **-** displays the product profile of the gB-SLP12-Delta725 polypeptide (indicated as gB-Delta725 in the figure) obtained in the presence or absence of Pluronic.

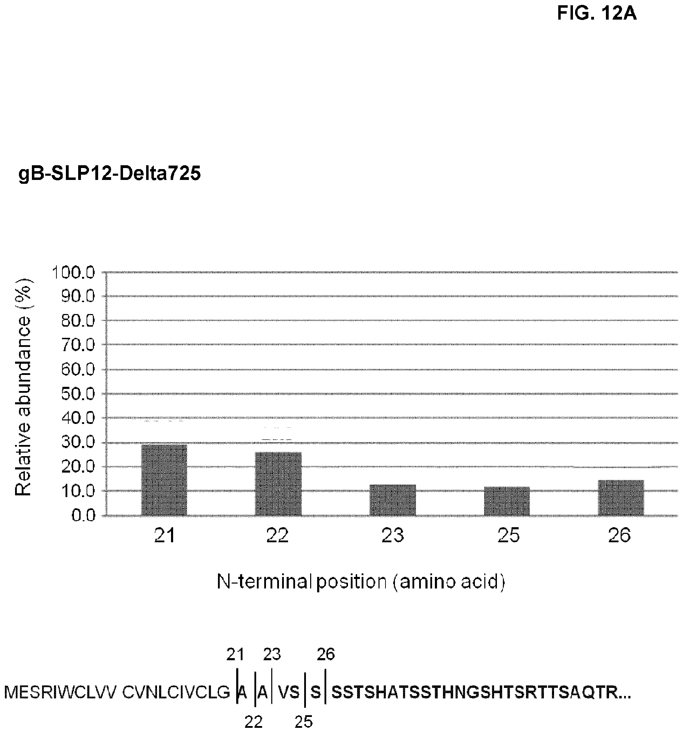

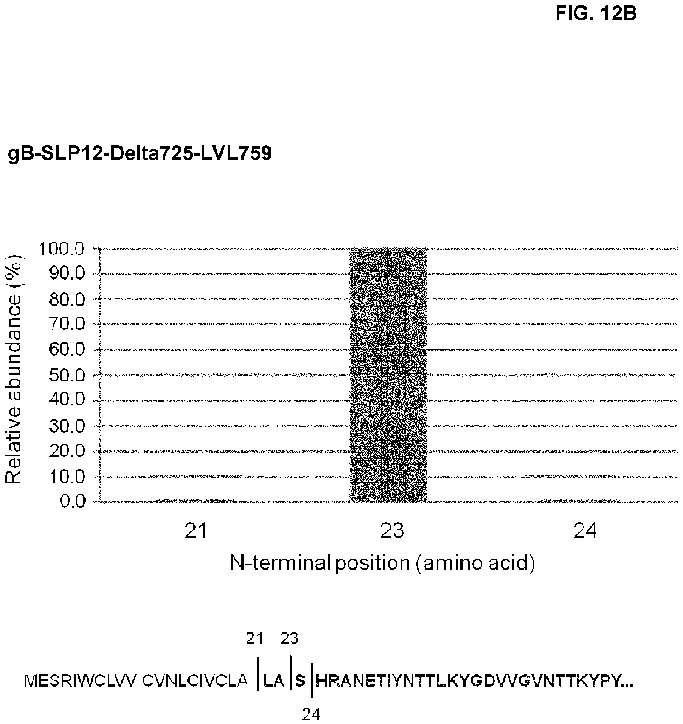

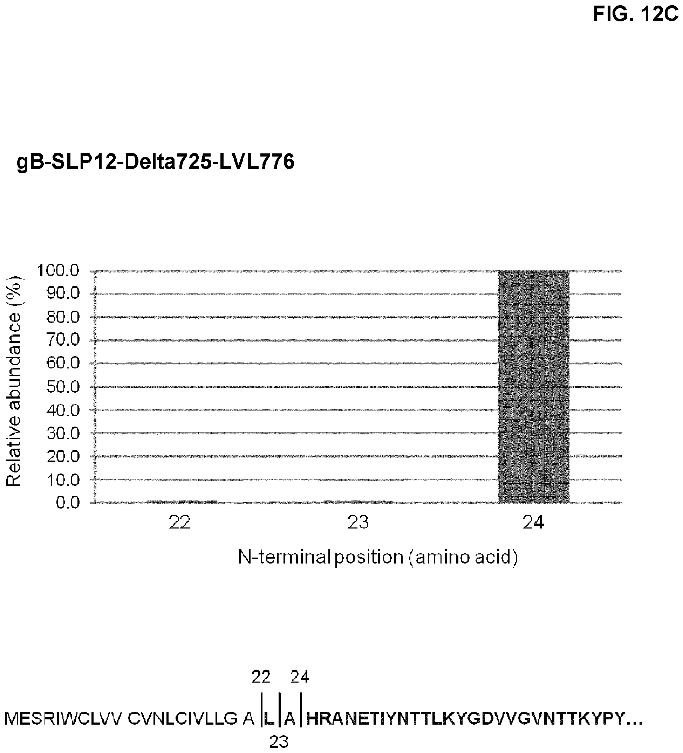

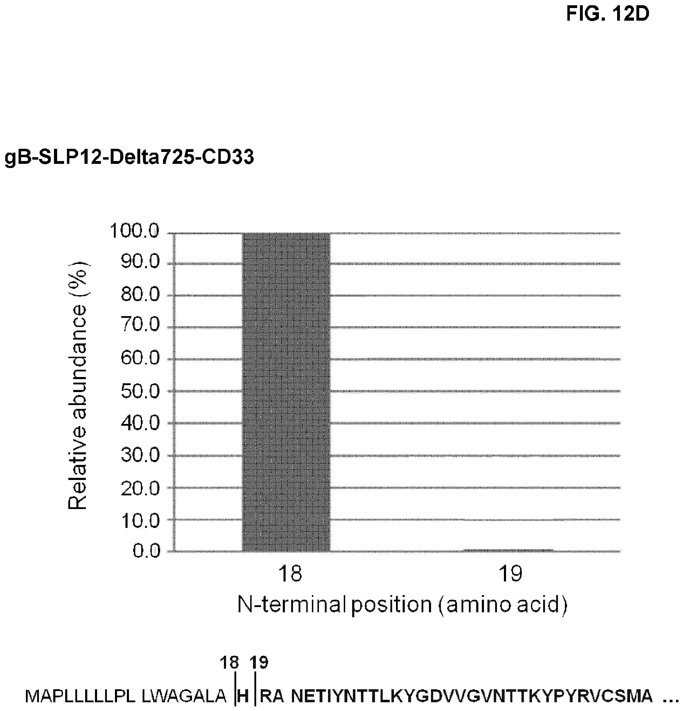

**FIG. 12** shows a peptide analysis by MS/MS mapping the N-terminal end of the indicated polypeptides generated upon their recombinant expression and secretion in CHO cells and after the signal sequence was cleaved off. **FIG. 12A** indicates the relative abundance of the different polypeptides having a different N-terminal amino acid within the population of gB-SLP12-Delta725 recombinantly expressed after the signal sequence was cleaved off. **FIG. 12B** indicates the relative abundance of the different polypeptides having a different N-terminal amino acid present within the population of gB-SLP12-Delta725-LVL759 recombinantly expressed after the signal sequence was cleaved off. **FIG 12C** indicates the relative abundance of the different polypeptides having a different N-terminal amino acid present within the population of gB-SLP12-Delta725-LVL776 recombinantly expressed after the signal sequence was cleaved off. **FIG. 12D** indicates the relative abundance of the different polypeptides having a different N-terminal amino acid present within the population of gB-SLP12-Delta725-CD33 recombinantly expressed after the signal sequence was cleaved off

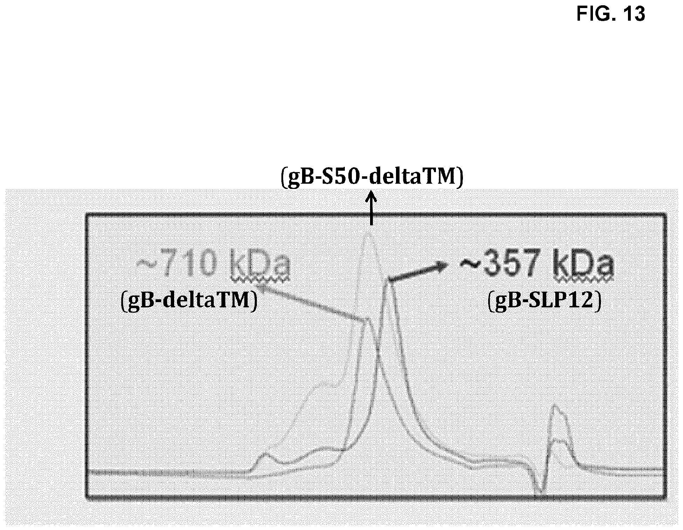

**FIG. 13** shows the product profile of the polypeptides gB-DeltaTM, gB-S50-DeltaTM and gB-SLP12, upon their recombinant expression in CHO cells, as analysed by Size Exclusion Chromatography based on UV (SEC-UV). Peaks represent multimers of the indicated size.

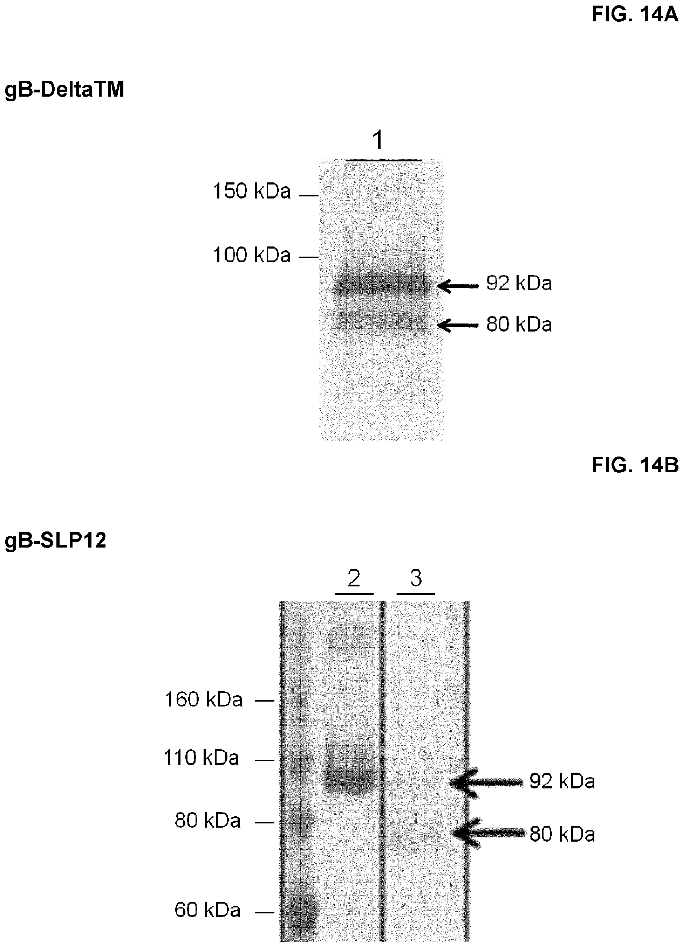

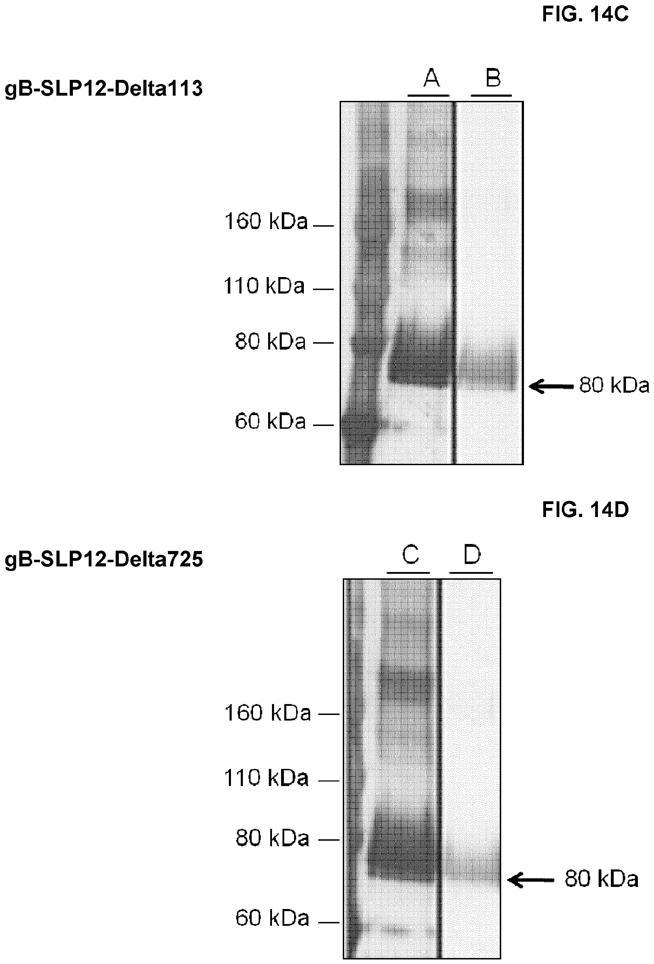

**FIG. 14** shows a Western Blot analysis using either an anti-gB antibody or an anti-His antibody displaying the level of gB protein after the indicated polypeptides were recombinantly expressed, secreted, purified and deglycosylated. **FIG. 14A** corresponds to gB-DeltaTM and shows a Western blot probed with an anti-gB antibody. **FIG. 14B** corresponds to gB-SLP12 and shows a Western blot probed with an anti-His antibody (lane 2) and with an anti-gB antibody (lane 3). **FIG. 14C** corresponds to gB-SLP12-Delta113 and shows a Western blot probed with an anti-His antibody (lane A) and with an anti-gB antibody (lane B). **FIG. 14D** corresponds to gB-SLP12-Delta725 and shows a Western blot probed with an anti-His antibody (lane C) and with an anti-gB antibody (lane D).

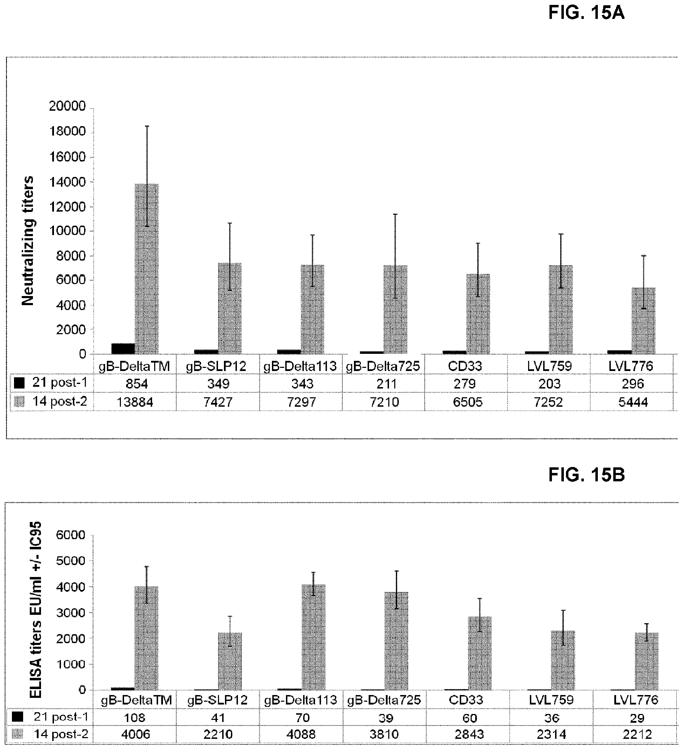

**FIG. 15** presents immunogenicity data. **FIG. 15A** shows neutralizing titers induced after administration of the indicated polypeptides formulated for injection. **FIG. 15B** shows ELISA anti-gB antibody titers induced after administration of the indicated polypeptides formulated for injection.

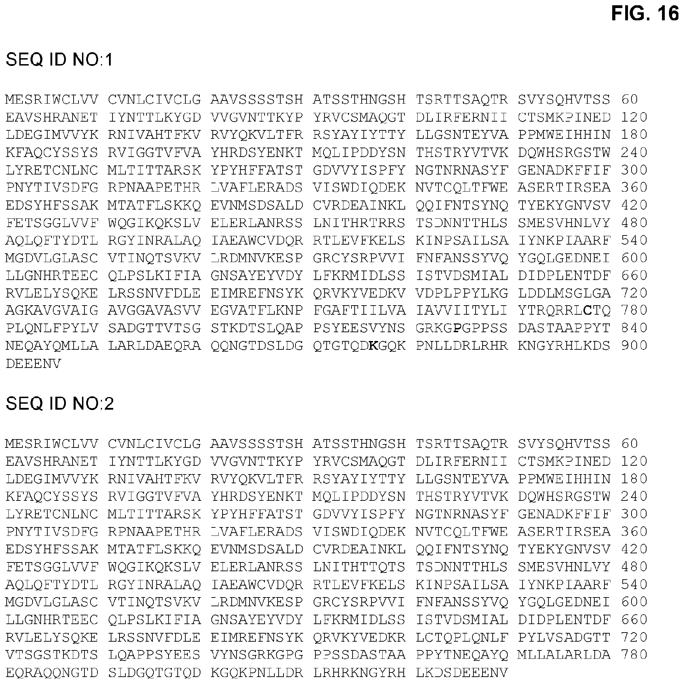

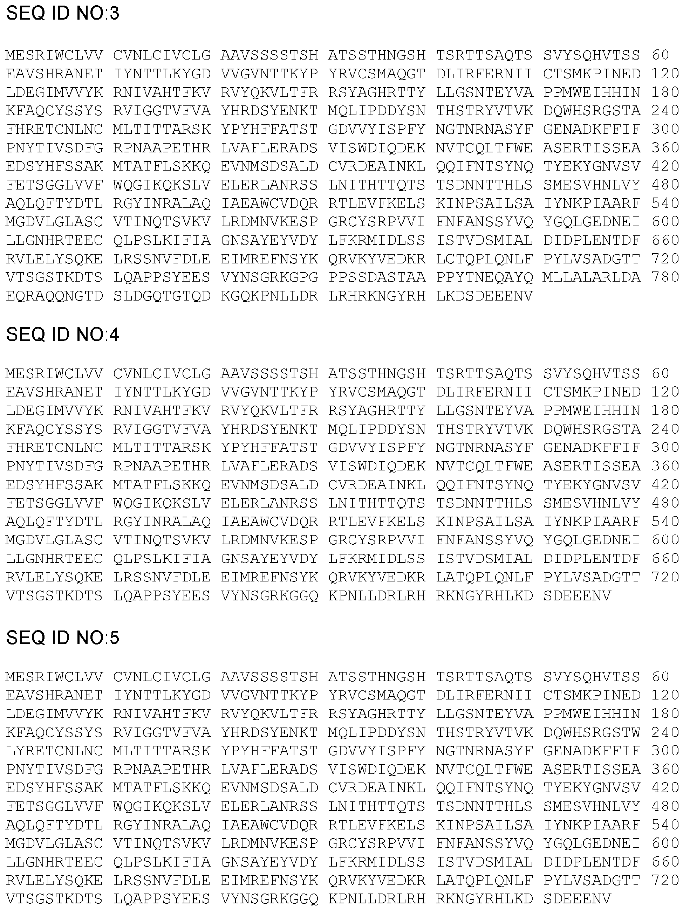

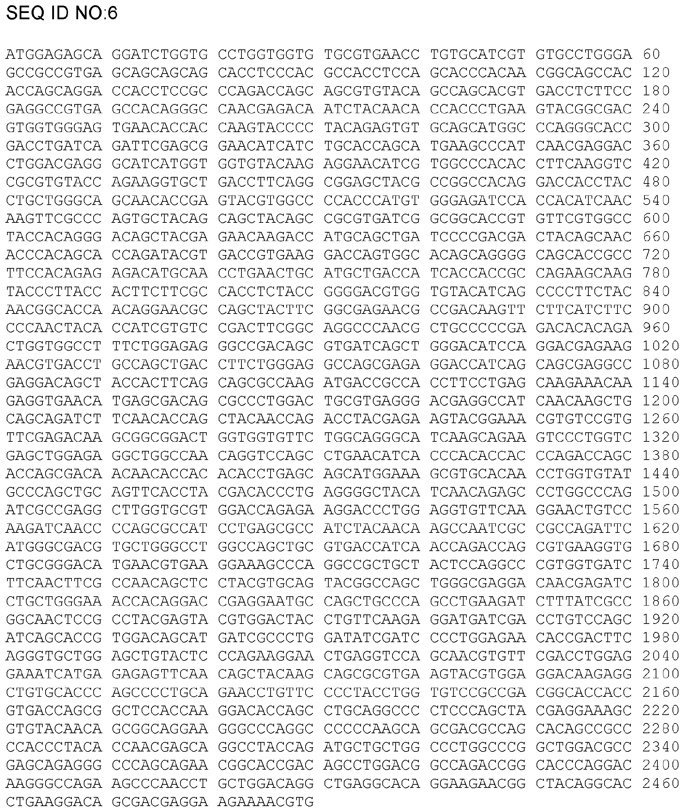

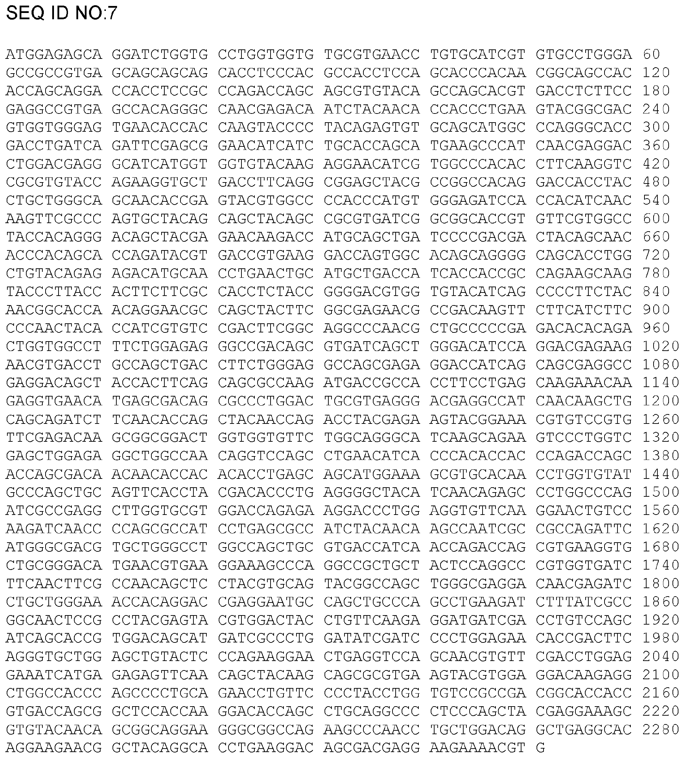

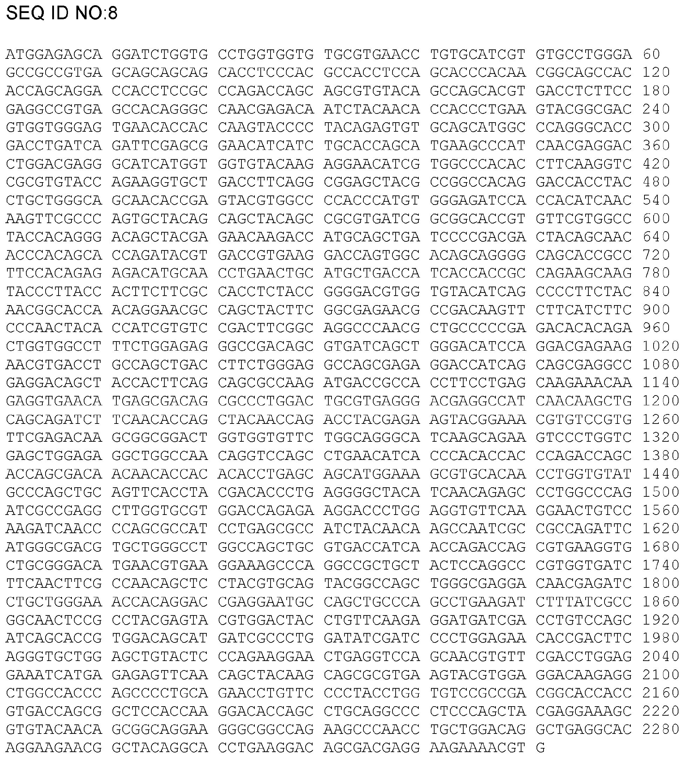

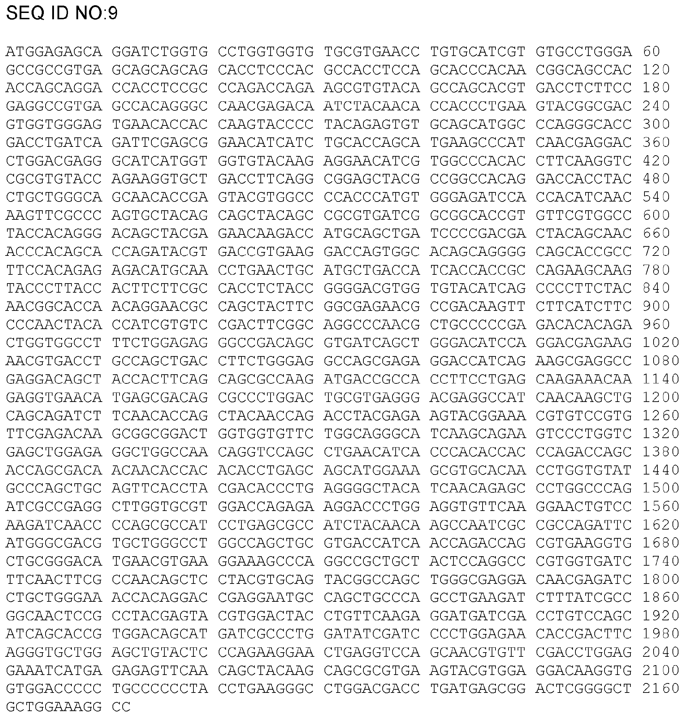

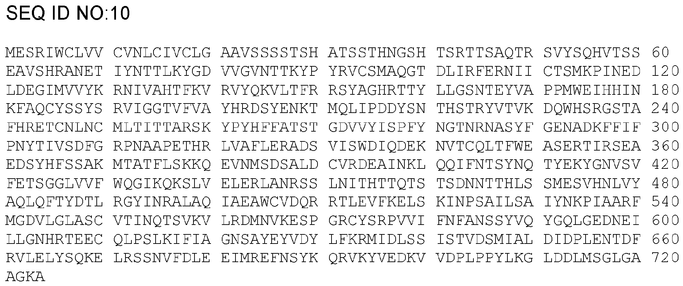

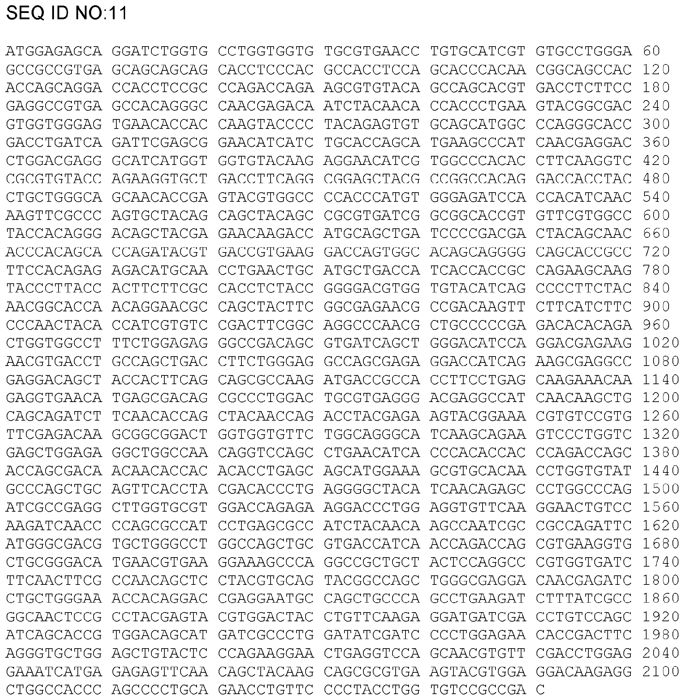

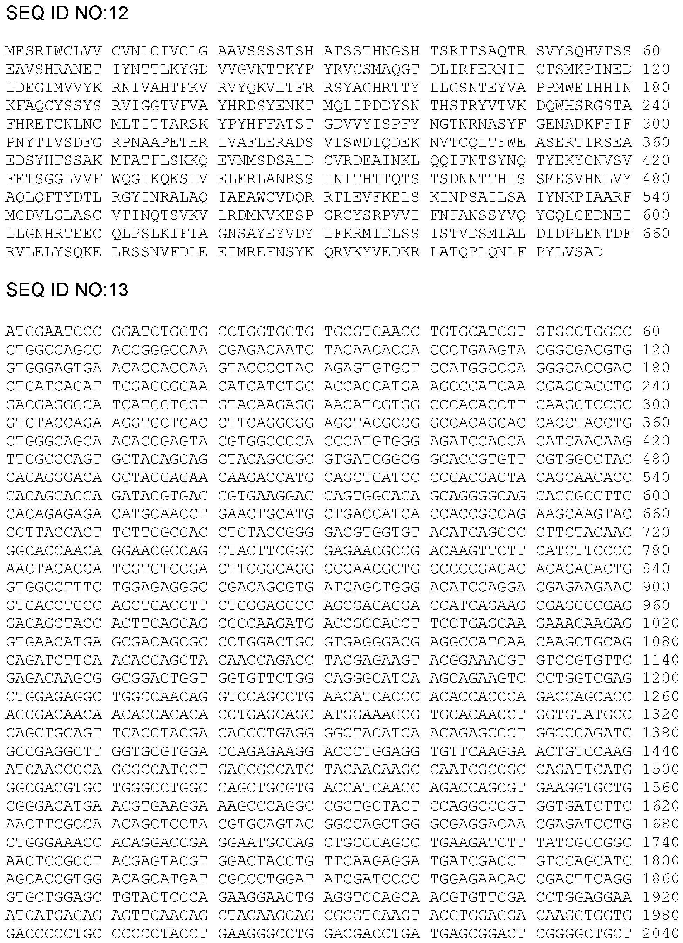

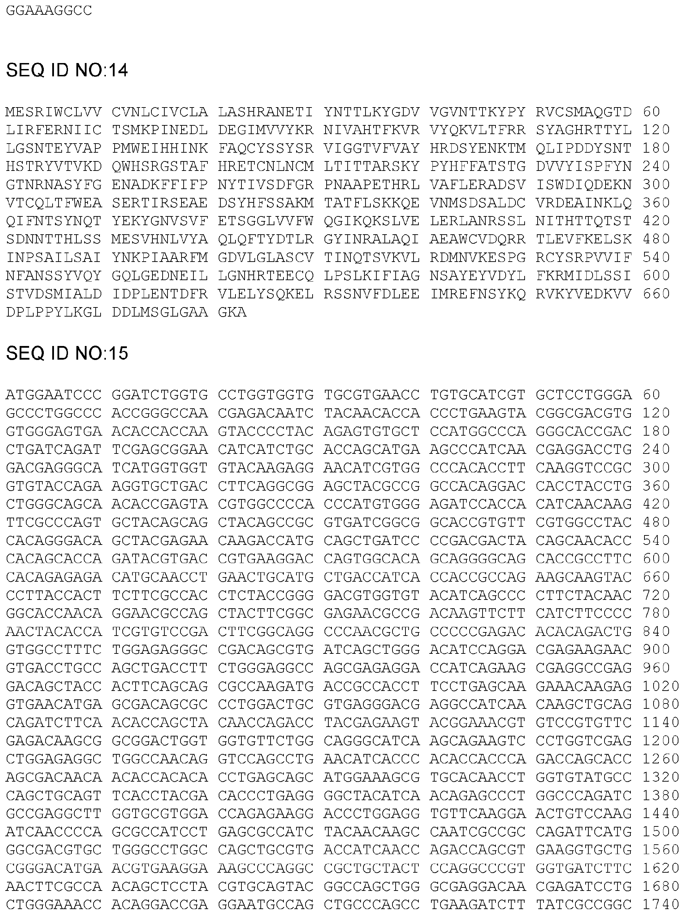

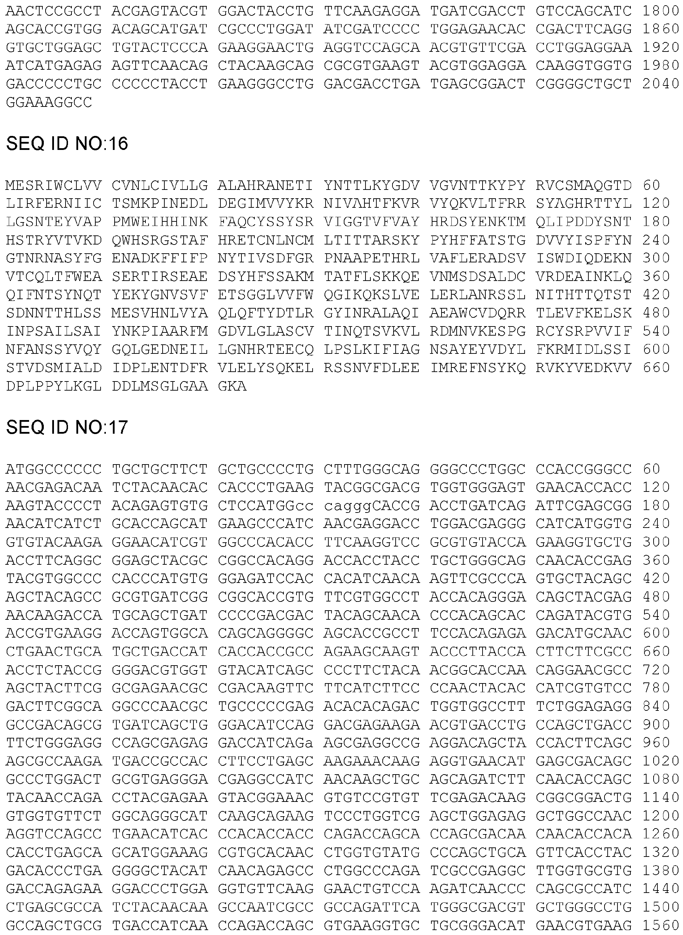

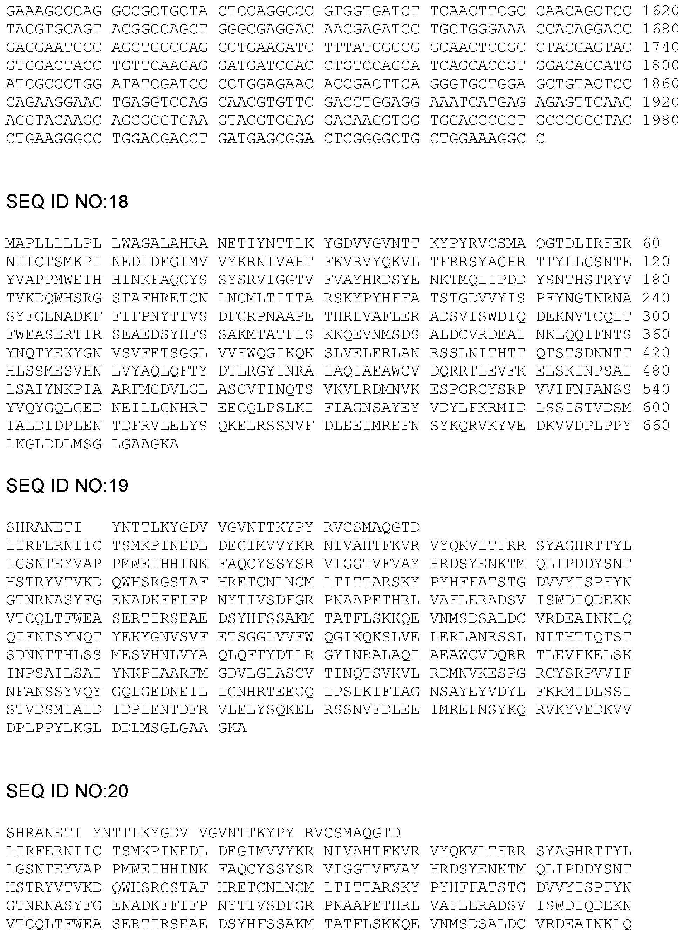

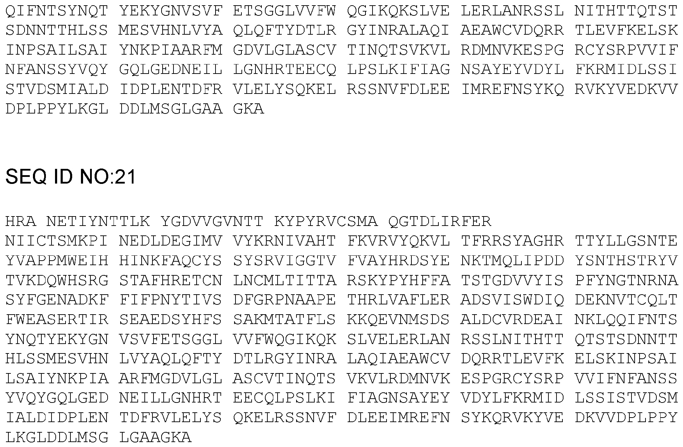

**FIG. 16** shows sequences SEQ ID NO:1 to SEQ ID NO:21 as described below.

### DESCRIPTION OF SEQUENCE LISTING

SEQ ID NO: 1 - Amino acid sequence of full length gB polypeptide from the CMV AD169 strain.
SEQ ID NO:2 - Amino acid sequence of gB-DeltaTM polypeptide from the CMV AD169 strain.
SEQ ID NO:3 - Amino acid sequence of gB-SLP12 polypeptide from the CMV AD169 strain.
SEQ ID NO:4 - Amino acid sequence of gB-SLP12-Del2 polypeptide from the CMV AD169 strain.
SEQ ID NO:5 - Amino acid sequence of gB-SLP1-Del2 polypeptide from the CMV AD169 strain.
SEQ ID NO:6 - Nucleotide sequence encoding the gB-SLP12 polypeptide from the CMV AD169 strain.
SEQ ID NO:7 - Nucleotide sequence encoding the gB-SLP1-Del2 polypeptide from the CMV AD169 strain.
SEQ ID NO:8 - Nucleotide sequence encoding the gB-SLP12-Del2 polypeptide from the CMV AD169 strain.
SEQ ID NO:9 - Nucleotide sequence encoding the gB-SLP12-Delta725 polypeptide from the CMV AD169 strain.
SEQ ID NO:10 Amino acid sequence of gB-SLP12-Delta725 polypeptide from the CMV AD169 strain.
SEQ ID NO:11 - Nucleotide sequence encoding the gB-SLP12-Delta113 polypeptide from the CMV AD169 strain.
SEQ ID NO:12 - Amino acid sequence of gB-SLP12-Delta113 polypeptide from the CMV AD169 strain.
SEQ ID NO:13 - Nucleotide sequence encoding the gB-SLP12-Delta725-LVL759 (or LVL759) polypeptide from the CMV AD169 strain.
SEQ ID NO:14 - Amino acid sequence of gB-SLP12-Delta725-LVL759 (LVL759) polypeptide from the CMV AD169 strain.
SEQ ID NO:15 - Nucleotide sequence encoding the gB-SLP12-Delta725-LVL776 (or LVL776) polypeptide from the CMV AD169 strain.
SEQ ID NO:16 - Amino acid sequence of gB-SLP12-Delta725-LVL776 (or LVL776) polypeptide from the CMV AD169 strain.
SEQ ID NO:17 - Nucleotide sequence encoding the gB-SLP12-Delta725-CD33 (or CD33) polypeptide from the CMV AD169 strain.
SEQ ID NO:18 - Amino acid sequence of gB-SLP12-Delta725-CD33 (or CD33) polypeptide from the CMV AD169 strain.
SEQ ID NO:19 - Amino acid sequence of gB-SLP12-Delta725-LVL759 (or LVL759) polypeptide from the CMV AD169 strain in its mature form.
SEQ ID NO:20 - Amino acid sequence of gB-SLP12-Delta725-LVL776 (or LVL776) polypeptide from the CMV AD169 strain in its mature form.
SEQ ID NO:21 - Amino acid sequence of gB-SLP12-Delta725-CD33 (or CD33) polypeptide from the CMV AD169 strain in its mature form.

### DETAILED DESCRIPTION

The present invention concerns a novel CMV gB polypeptide which presents excellent immunogenicity and superior process characteristics. The gB polypeptide of the invention is particularly suitable as an antigen for inclusion in an immunogenic composition, such as vaccines. In particular, the gB polypeptide of the invention displays an improved homogenous product profile. Indeed, the inventors observed that upon expression, gB polypeptides of the prior art, such as a gB polypeptide lacking the transmembrane domain (gB-DeltaTM), assembled into multimers of different sizes, including high molecular weight multimers, making the population heterogeneous. They also observed that the multimer profile was not consistent, as the proportion of multimers of a given size varied from one experiment to another. This type of variability and inconsistency is not acceptable in terms of vaccine formulations. Therefore, a need remains for a CMV gB polypeptide which presents an improved homogenous and consistent profile product. Also, the aggregation into high molecular weight multimers can have a negative impact on the subsequent process of purification of the recombinant polypeptide, as aggregation may lead, in particular, to a less soluble form of the polypeptide. Therefore, a need remains for a CMV gB polypeptide which presents an improved process characteristics.

A CMV gB polypeptide lacking the transmembrane domain is described in EP 0 802 979.

Surprisingly, the present inventors observed that the introduction of mutations in specific regions of such a CMV gB polypeptide lacking the transmembrane domain results in an improved profile product, such that the population is not only more homogeneous than the corresponding non-mutated polypeptide, but it also displays a profile which is consistent from one experiment to another. In particular, high molecular weight multimers are reduced and the proportion of trimers is increased by a two-fold factor. Typically, trimers represent at least 50%, suitably at least 60% and more suitably at least 70% of the population of CMV gB polypeptides of the present disclosure. These mutations do not impact the expression level, nor the secretion level of the resulting mutants, as compared with the CMV gB polypeptide lacking the transmembrane domain. These mutations are located outside the antigenic epitopes of CMV gB known to be responsible for inducing an immune response. The observed shift from a population where the polypeptides assembled into high molecular weight multimers, such as the polypeptide of the prior art lacking the transmembrane domain, to a population where the polypeptides have an improved trimerisation profile is associated with an easier and better purification of the polypeptides. In particular, the inventors observed that the purification yield of the polypeptides according to the invention is significantly higher than the purification yield obtained with the CMV gB polypeptide of the prior art lacking the transmembrane domain. The yield improvement is at least 2-fold, and possibly at least 3- to 5-fold. Additionally, further mutations in the novel polypeptides in accordance with the invention are shown to result in polypeptides being more efficiently expressed and secreted than the prior art polypeptides. Such a property presents a significant advantage in terms of antigen production for vaccine manufacturing, a field in which the antigen production is typically a limiting factor.

The inventors also observed that prior art gB polypeptides, such as a CMV gB polypeptide lacking the transmembrane domain, presented heterogeneity at the N-terminal and the C-terminal ends of the polypeptide. They indeed observed that a clipping occurred in the C-terminal cytoplasmic domain of gB resulting in at least two different forms of the polypeptide having a different size after recombinant expression in cells and secretion. For the purpose of inclusion in an immunogenic composition, such as in a vaccine, it is preferable to have a homogeneous population of gB polypeptides in the sense that it is made of mainly one single form. The inventors found out that when deleting at least part of the C-terminal cytoplasmic domain, the clipping no longer occurred and the resulting population of polypeptides was mainly made of a single form of gB.

Similarly, it was observed that prior art gB polypeptides, such as a CMV gB polypeptide lacking the transmembrane domain, presented heterogeneity in the N-terminal part of the polypeptide. Indeed, the inventors observed that the signal sequence, after recombinant expression and secretion of the polypeptides, was cleaved off by a signal peptidase at different amino acid positions, generating thus a population of gB mature polypeptides having a varying end at the N-terminus, *i.e.* a population made of gB polypeptides having a different amino acid at the N-terminus. This phenomenon is also referred to as signal peptidase "woobling". For efficacy purposes, and in particular for consistency, it is important, in the vaccine field, that an antigen to be included in a vaccine be as homogeneous as possible and as undegraded as possible, so that vaccine lots are reproducible and consistent.

It is to be understood in the sense of the present invention that an improved homogenous product profile means that the population of CMV gB polypeptides of the invention, upon their recombinant expression, is made of at least 50% of trimers, for instance, as measured by analytical ultra centrifugation (See Example 2, section 1.2). Typically, a preparation comprising a population of CMV gB polypeptides of the invention is made of at least 50%, suitably at least 60%, and more suitably, at least 70% of of trimers. Such a profile of at least 50% of trimers will also be referred to "an improved trimerisation profile" or "a trimer-enriched profile" in the present specification. These terms are to be considered as synonymous and interchangeable.

When referring to "homogeneity/heterogeneity" in the C-terminal part of polypeptides, a heterogeneous population means that the population is made of at least two polypeptides having different sizes, while a homogenous population is to be understood as a population mainly made of a single polypeptide of a given size.

When referring to "homogeneity/heterogeneity" in the N-terminal part of polypeptides, a heterogeneous population means that the population is made of different mature polypeptides starting each with a different amino acid at the N-terminus. On the contrary, in accordance with the invention, a homogeneous population is to be understood as a population mainly made of a single mature polypeptide, which polypeptide starting unvaryingly with an identical given amino acid at the N-terminal end. In the present invention, a "mature" polypeptide refers to a polypeptide wherein the signal sequence has been cleaved off. Typically, in a N-terminal homogeneous population in the sense of the present invention, more than 30%, suitably at least 80%, more suitably from 80% to 90%, more suitably at least 99% of the mature polypeptides produced after cleavage of the signal sequence start with the same amino acid at the N-terminal end. Accordingly, in one aspect of the disclosure, it is described a preparation comprising a population of mature CMV gB polypeptides produced after cleavage of the signal sequence, wherein at least 30%, at least 80%, from 80% to 90% of the mature gB polypeptides comprise the same amino acid at the N-terminal end. In particular, the mature polypeptides described in the disclosure suitably start with a serine or a histidine at the N-terminal position.

gB is an envelope glycoprotein B having numerous roles, one of which is the involvement in the fusion of the Cytomegalovirus with host cells. It is encoded by the UL55 gene of HCMV genome. The size of the native form of gB depends on the size of the open reading frame (ORF) which may vary a little according to the strain. For example, the ORF of AD169 strain, which is 2717 bp long, encodes a full length gB of 906 amino acids whereas the ORF of Towne strain encodes a full length gB of 907 amino acids. Although the present invention is applicable to gB proteins originating from any CMV strain, in order to facilitate its understanding, when referring to amino acid positions in the present specification, the numbering is given in relation to the amino acid sequence of the gB polypeptide of SEQ ID NO:1 originating from the AD169 strain, unless otherwise stated. The present invention is not, however, limited to the AD169 strain. Comparable amino acid positions in a gB polypeptide of any other CMV strain can be determined by those of ordinary skill in the art by aligning the amino acid sequences using readily available and well-known alignment algorithms (such as BLAST). Accordingly, when referring to "other CMV gB polypeptides", it is to be understood as CMV gB polypeptides of any strain different from AD169. The native form of AD169 gB contains in the N-terminal to C-terminal direction of the protein (see also FIG. 1) (i) an amino acid signal sequence, or signal peptide, known to be involved in the polypeptide intracellular trafficking including targeting the polypeptide towards secretion, followed by (ii) a region called the leader sequence, (iii) an extracellular domain containing an endoproteolytic cleavage site of a furin type between amino acids 456 and 460 of the sequence set forth in SEQ ID NO:1, (iv) a transmembrane domain and (v) a C-terminal cytoplasmic domain. Moreover, amino acid stretches have been identified in the amino acid sequence of CMV gB as putative fusion loops based on sequence alignment with gB proteins originating from distinct herpesviruses (Backovic et al. 2007. J. Virol. 81(17): 9596-600), such as HSV. HSV fusion loops were experimentally determined (Hannah, B.P. et al. 2009. J. Virol. 83(13): 6825-6836). Said stretches were so called owing to their involvement in fusion of the viruses with host cells. According to the above literature, two putative fusion loops were identified by sequence alignment in CMV gB, which are called FL1 and FL2, in the rest of the specification. Said fusion loops are located in the extracellular domain of the protein upstream to the transmembrane domain.

Surprisingly, the present inventors observed that disrupting the fusion loops FL1 and FL2 of a CMV gB polypeptide having a non-functional transmembrane domain, by mutating specific amino acids, resulted in a product which upon expression presented an improved trimerisation profile, as compared to a CMV gB polypeptide having a non-functional transmembrane domain, but having intact fusion loops. Accordingly, in one aspect of the disclosure, the CMV gB polypeptide comprises the mutation, such as for instance amino acid substitution, or deletion, of at least one of the fusion loops FL1 or FL2, possibly both. Suitably, the polypeptide of the disclosure comprises at least one amino acid substitution, or deletion, in the fusion loop FL1. Suitably, the polypeptide of the disclosure comprises at least one amino acid substitution, or deletion, in the fusion loop FL2. More suitably, the CMV gB polypeptide of the disclosure comprises the mutation of both fusion loops FL1 and FL2. For instance, the gB polypeptide of the disclosure comprises at least one amino acid substitution, or deletion, in the fusion loop FL1 and at least one amino acid substitution, or deletion, in the fusion loop FL2.

In the sense of the present invention, the amino acids encompassing the fusion loop FL1 of CMV AD169 gB, *i.e.* the amino acid sequence of FL1, are defined as ¹⁵⁵Y.I.Y¹⁵⁷ of the sequence set forth in SEQ ID NO:1. Likewise, the amino acids encompassing the fusion loop FL2, *i.e.* the amino acid sequence of FL2, within the meaning of the present invention, are defined as ²⁴⁰W.L.Y²⁴² of the sequence set forth in SEQ ID NO:1. Corresponding fusion loops FL1 and FL2 sequences can be identified within gB polypeptides from other CMV strains, for instance, by sequence alignment. Disruption of said fusion loops includes any type of mutation in relation to these amino acid triplets, whether by deletion or amino acid substitutions, which results in a product, upon recombinant expression, having a trimer-enriched, improved profile, that is the product has a greater proportion of polypeptides in a trimeric form as compared with the non-mutated form

A non-limiting suitable mutation concerning FL1 is the deletion, or substitution, of at least one, suitably at least two, amino acids selected from ¹⁵⁵Y.I.Y¹⁵⁷ amino acids of the sequence set forth in SEQ ID NO:1, or at a corresponding position in other CMV gB polypeptides originating from different strains, with a polar amino acid, in particular, a non-aromatic polar amino acid, more particularly, an amino acid selected from the group of positively charged amino acids consisting of lysine (K), histidine (H) and arginine (R). Accordingly, in some aspects of the disclosure, the CMV gB polypeptidehaving a non-functional transmembrane domain, has a mutated fusion loop FL1 wherein at least the isoleucine (I¹⁵⁶) suitably at least the tyrosine (Y¹⁵⁵), suitably at least the tyrosine (Y¹⁵⁷), is substituted with a histidine (H), or is deleted. Alternatively, the CMV gB polypeptide of the disclosure has a mutated fusion loop FL1 wherein at least the tyrosine (Y¹⁵⁷) is substituted with an arginine (R). In one aspect of the disclosure, the CMV gB polypeptide having a non-functional transmembrane domain, has a mutated fusion loop FL1 wherein at least the isoleucine (I¹⁵⁶) and tyrosine (Y¹⁵⁷) are substituted with histidine (H¹⁵⁶) and arginine (R¹⁵⁷), respectively, or deleted. In a further aspect of the disclosure, the CMV gB polypeptide having a non-functional transmembrane domain has a mutated fusion loop FL1 wherein at least the tyrosine (Y¹⁵⁵) is substituted with a glycine (G), or is deleted, optionally, in combination with the substitution, or deletion, of the isoleucine (I¹⁵⁶) with a histidine (H) or with the substitution, or deletion, of the tyrosine (Y¹⁵⁷) with an arginine (R), or in combination with both substitutions, or deletions. In a specific embodiment, the CMV gB polypeptide of the invention having a non-functional transmembrane domain has a mutated fusion loop FL1 wherein the three amino acids ¹⁵⁵Y.I.Y¹⁵⁷ of the sequence set forth in SEQ ID NO:1, or at a corresponding position in other CMV gB polypeptides originating from different strains, are substituted with amino acids ¹⁵⁵G.H.R¹⁵⁷. The mutation of the FL1 domain can be defined in an alternative way. As described below, the hydrophobicity of a particular amino acid sequence can be determined using a hydrophobicity scale, such as the Kyte and Dolittle scale (Kyte et al. 1982. J. Mol. Bio. 157: 105-132). The FL1 domain which encompasses the amino acids ¹⁵⁵Y.I.Y¹⁵⁷ achieves a score of +1.9 on that scale. In one embodiment, in the CMV gB polypeptide of the invention, the stretch of amino acids Y.I.Y located at position 155-157 of the sequence set forth in SEQ ID NO:1, or at a corresponding position in other CMV gB polypeptides, is substituted with a stretch of three amino acids having a hydrophobicity score of less than -3, less than - 7, or less than -8, as measured using the Kyte and Doolittle scale. All three amino acids may not be substituted simultaneously, so long as the global score of the amino acid stretch reaches the above-specified values. For example, only one or two amino acids of the three amino acid long stretch may be substituted, the scoring being then calculated based on the identity of the substituted amino acids, *i.e.* one or two, plus the identity of the native ones, two or one, respectively.

A non-limiting suitable mutation concerning the fusion loop FL2 is the deletion, or substitution of at least one, suitably at least two, amino acids selected from ²⁴⁰W.L.Y²⁴² of the sequence set forth in SEQ ID NO:1, or at a corresponding position in other CMV gB polypeptides originating from different strains, with a positively charged amino acid selected from the group consisting of lysine (K), histidine (H) and arginine (R), suitably a histidine (H). Accordingly, in some aspects of the disclosure, the CMV gB polypeptide of the invention has a mutated fusion loop FL2 wherein at least the tyrosine (Y²⁴²), suitably at least the leucine (L²⁴¹), suitably at least the tryptophan (W²⁴⁰), is substituted with a histidine (H), or deleted, optionally, in combination with a mutated fusion loop FL1 as described above. In particular embodiments, the CMV gB polypeptide of the invention has a mutated fusion loop FL2 wherein the three amino acids ²⁴⁰W.L.Y²⁴² of the sequence set forth in SEQ ID NO:1, or at a corresponding position in other CMV gB polypeptides originating from different strains, are substituted with amino acids ²⁴⁰A.F.H ²⁴², optionally, in combination with FL1 mutations as described above.

The mutations relating to FL1 and/or FL2 fusion loops are not limited to the above described mutations. Further mutations, in addition to the ones described above, may be performed, such as, for instance, mutating amino acids surrounding the above triplets ¹⁵⁵Y.I.Y¹⁵⁷ and ²⁴⁰W.L.Y²⁴² of the sequence set forth in SEQ ID NO:1, or at a corresponding position in other CMV gB polypeptides originating from different strains, so long as the resulting mutant polypeptide presents an improved, trimer-enriched, profile, and said further mutations do not interfere with production (expression and secretion), processing or stability of the polypeptide when recombinantly expressed in host cells.

The present inventors also observed that combining the mutation of at least one fusion loop (FL1 and/or FL2), such as any of the above described mutations, with an additional mutation in the C-terminal cytoplasmic domain of the polypeptide also provided a CMV gB polypeptide with an improved trimerisation profile. In the sense of the present invention, the C-terminal cytoplasmic domain of a gB CMV polypeptide is to be understood as the domain located downstream to the transmembrane domain (see also Fig. 1). In particular, the additional mutation in the C-terminal cytoplasmic domain suitably concerns the deletion of a hydrophobic region, suitably a proline-rich region, suitably a highly hydrophilic, negatively charged region, possibly all of them. As a non-limiting example, a suitable mutation corresponds to the deletion of the amino acids 825 to 877 of the sequence set forth in SEQ ID NO:1 which is called Del2. The delineation of the above sequence which is deleted is not strictly limited to the amino acid position 825 to 877 and may vary, so long as the resulting mutant polypeptide presents an improved, trimer-enriched, profile, and is not affected in terms of production, processing or stability when recombinantly expressed in host cells. Accordingly, in some aspects of the disclosure the CMV gB polypeptide comprises both the mutation of at least one fusion loop (FL1 and/or FL2), suitably FL1, and the deletion of at least one hydrophobic region, one proline-rich region and one highly hydrophilic region in the C-terminal cytoplasmic domain, in particular, the deletion of amino acids 825 to 877 (Del2) of the sequence set forth in SEQ ID NO:1.

Hydrophobicity of an amino acid sequence or a fragment thereof is dictated by the type of amino acids composing this sequence or a fragment thereof. Amino acids are commonly classified into distinct groups according to their side chains. For example, some side chains are considered non-polar, *i.e.* hydrophobic, while some others are considered polar. In the sense of the present invention, alanine (A), glycine (G), valine (V), leucine (L), isoleucine (I), methionine (M), proline (P), phenylalanine (F) and tryptophan (W) are considered to be part of hydrophobic amino acids, while serine (S), threonine (T), asparagine (N), glutamine (Q), tyrosine (Y), cysteine (C), lysine (K), arginine (R), histidine (H), aspartic acid (D) and glutamic acid (E) are considered to be part of polar amino acids. Regardless of their hydrophobicity, amino acids are also classified into subgroups based on common properties shared by their side chains. For example, phenylalanine, tryptophan and tyrosine are jointly classified as aromatic amino acids and will be considered as aromatic amino acids within the meaning of the present invention. Aspartate (D) and glutamate (E) are part of the acidic or negatively charged amino acids, while lysine (K), arginine (R) and histidine (H) are part of the basic or positively charged amino acids, and they will be considered as such in the sense of the present invention. Hydrophobicity scales are available which utilize the hydrophobic and hydrophilic properties of each of the 20 amino acids and allocate a hydrophobic score to each amino acid, creating thus a hydrophobicity ranking. As an illustrative example only, may the Kyte and Dolittle scale previously discussed, be cited (Kyte et al. 1982. J. Mol. Bio. 157: 105-132). This scale allows to calculate the average hydrophobicity within a segment of predetermined length. Accordingly, hydrophobic regions in an amino acid sequence may be identified by the skilled person as potential targets for mutation in accordance with the present invention. The ability of the mutation of said regions to induce an improved product profile of the resulting mutant protein, *i.e.* favouring the trimers proportion within the population, may then be tested as described below. The mutation of a hydrophobic region may consist in a deletion of said region as indicated above, or part of it, or in point mutations substituting hydrophobic amino acids with polar amino acids within said region. The relevance of the mutation will be determined by analysing the resulting effect of said mutation on the product profile of the mutated polypeptide, upon recombinant expression in host cells, that is, a relevant mutation is to induce an improved, trimer-enriched, profile wherein at least 50%, suitably at least 60% and more suitably, at least 70% of trimers are present within the population of the mutated polypeptide. The inventors observed that the C-terminal cytoplasmic domain could be deleted to a varying extent while maintaining the improved product profile, as described above. Accordingly, the present disclosure describes CMV gB polypeptides wherein suitably 10%, more suitably 20%, more suitably 60%, more suitably 80%, more suitably 90% of amino acids of the cytoplasmic domain is deleted. Possibly, the entire cytoplasmic domain can be deleted. In particular, the inventors observed that when deleting at least 70%, suitably at least 80%, or more of the cytoplasmic domain, the resulting CMV gB polypeptides showed a better expression and secretion level when recombinantly expressed in host cells. Accordingly, in some embodiments the CMV gB polypeptides of the invention comprise both the mutation of at least one fusion loop (FL1 and/or FL2), suitably FL1, or possibly both, and the deletion of the entire cytoplasmic domain, for example, amino acids 776 to 906 of the sequence set forth in SEQ ID NO:1, or at a corresponding position in other CMV gB polypeptides.

Any method allowing to discriminate the constituents within a population, such as purified polypeptides possibly aggregating to each other, according to their size or density can be used for monitoring the product profile and, thus, for determining whether given mutations are conferring an improved profile product as described above. Suitable methods may be based on sedimentation, such as analytical ultracentrifugation (AUC), or on chromatography, in particular, size exclusion chromatography, such as size exclusion chromatography based on UV (SEC-UV). Alternatively, a method for evaluating the aggregation level within a protein or polypeptide sample can consist in treating the sample with a cross-linking agent, so as to form covalent bonds between two proteins, or polypeptides, or more. Glutaraldehyde may suitably be used as a cross-linker. After cross-linking, loading the sample on a gel in denaturing conditions, such as SDS-PAGE, and staining the gel for the presence of proteins, for example with Coomassie blue or silver nitrate, will display aggregates, if any, which are separated according to their molecular weight. Loading in parallel a ladder made of proteins with known molecular weight will allow determining the molecular weight of the different aggregates. Provided that the molecular weight of the minimal unit, such as a polypeptide in its monomeric form, is known, then the observed molecular weight of the aggregates will be indicative of the multimerisation level within aggregates. For instance, the CMV AD169 gB polypeptide deleted from the transmembrane domain is 838 amino acids long. If considering that the average molecular weight of an amino acid is 110 kDa, then the expected molecular weight of this polypeptide is 92 kDa. Therefore, should such a polypeptide aggregate, then the average molecular weight of the resulting multimers would be expected to be a multiple of 92. In particular, if forming trimers, the expected average molecular weight of such trimers, made of 3 molecules of gB deleted from the transmembrane domain, is 276 kDa.

Any of the above-described mutations in fusion loops FL1 and/or FL2 may be combined with further mutations. CMV gB polypeptides according to the present invention are to be understood as having a non-functional transmembrane domain and comprising further specific mutations, such as the fusion loops mutations. The location of a transmembrane domain within a polypeptide can be predicted through the use of computer programs able to formulate a hydropathy scale from the amino acid sequence, using the hydrophobic and hydrophilic properties of the 20 amino acids (Kyte et al. 1982. J. Mol. Bio. 157: 105-132). The average hydropathy within a segment of predetermined length of sequence is calculated continuously as the program moves through the sequence. These consecutive hydropathy scores are then plotted from the N-terminus to the C-terminus, and a midpoint line is printed corresponding to a grand hydropathy average of amino acid compositions found in most known sequenced proteins. Membrane-bound proteins exhibit large uninterrupted regions on the hydrophobic side of the line corresponding to a portion of sequence which is embedded in the lipid bilayer of the membrane. A transmembrane domain is responsible for anchoring a polypeptide to the cell membrane. In viral envelope glycoproteins, the transmembrane domain typically contains stretches of 20-27 uncharged, primarily, hydrophobic amino acids near the C-terminal part of the polypeptide. As an example of the application of the hydropathy analysis as described above, regarding CMV gB, may the publication by Spaete *et al.* (Spaete et al., 1988, Virology 167: 207-225) cited. Indeed, the authors identified in the CMV gB polypeptide from the strains AD169 and Towne a putative hydrophobic transmembrane domain readily apparent as two broad, adjacent hydrophobic peaks near the C-terminus of the gB polypeptide. The first one comprises amino acids 715 to 748 and the second one amino acids 752 to 772 (the numbering corresponds to the amino acid sequence of the gB polypeptide from the AD169 strain as depicted in SEQ ID NO:1). A publication by Reschke *et al.* (Reschke et al., 1995, J. of Gen. Virology 76: 113-122) alternatively identified the second stretch of amino acids 751 to 771 as the putative transmembrane domain of AD169 CMV gB. These two publications suggest thus that the putative transmembrane domain of AD169 CMV gB includes at least the amino acids 751 to 771, and possibly the amino acids 714 to 771. In the sense of the present invention, the transmembrane domain of the CMV gB polypeptide encompasses at least the amino acids 701 to 775 of the sequence set forth in SEQ ID NO:1, or at a corresponding position in other CMV gB polypeptides, and is thus 74 amino acid long.

By "a non-functional transmembrane domain", it is to be understood within the meaning of the present invention that at least part of the putative transmembrane domain is altered to enable secretion of the resulting polypeptide from a host cell upon expression in said cell. In one aspect of the disclosure, the gB polypeptide is deleted from the entire transmembrane domain. In alternative aspects of the disclosure, the CMV gB polypeptides retain a portion of the transmembrane domain. In particular, the CMV gB polypeptides retain at least 5%, suitably at least 10%, more suitably at least 20%, more suitably at least 30%, or at least 50% of the amino acids of the transmembrane domain. Accordingly, in some aspects of the disclosure, at least 50% of the amino acid number of the transmembrane domain is deleted in the CMV gB polypeptides of the invention, suitably at least 70%, more suitably at least 80%, more suitably at least 90% and even 95% of the amino acid number of the transmembrane domain is deleted. According to one embodiment, the transmembrane domain of the CMV gB polypeptide of the invention is made non-functional by deleting amino acid amino acids 701 to 775 of the sequence set forth in SEQ ID NO:1, or at a corresponding position in other CMV gB polypeptides. Based on the above-described prediction to determine the location of a transmembrane domain within a polypeptide, the extent of the deletion may further vary, so long as the resulting deleted CMV gB polypeptide is secreted from host cells upon expression in said cells. The functional alteration of the transmembrane domain is not limited to deletion of amino acids. The alteration may consist in any other type of amino acid mutations, such as insertion and/or substitutions. Deletions, insertions and substitutions of amino acids may also be combined in any way, so long as the resulting mutated CMV gB polypeptide is secreted from host cells upon expression in said cells. It is within the skilled person's abilities to assess the impact of a given mutation, such as for instance the deletion of a given portion of the transmembrane domain, on the ability of the resulting mutated polypeptide to be secreted from cells. The secretion of a polypeptide can be detected and the secretion level be assessed by any technique known in the art. As a non-limiting illustrative example, it is possible to separately measure the content of the expressed polypeptide which is retained within the cells and which is secreted into the cell culture supernatant or cell culture medium upon expression. Typically, upon expression the cell culture supernatant is collected before the cells are lysed according to any common lysis method, providing thus two fractions, a cellular one and a supernatant one. The content of the polypeptide can then be measured in each fraction by any protein detection technique known in the art. For example, the polypeptide can be detected by Western-blot or by ELISA assay.

By "comprising at least a portion of the extracellular domain", it is to be understood in the sense of the present invention as at least the part of the extracellular domain comprising or encompassing the fusion loops FL1 and FL2. The number of additional amino acids outside the fusion loops in the extracellular domain may vary. The appropriate number of amino acids, or the percentage of amino acids of the extracellular domain present in the polypeptides of the invention should be such that the resulting polypeptides comprise the antigenic epitopes and present an improved trimerisation profile. Suitably, at least 50%, more suitably at least 70%, more suitably at least 80% and even 90% of the amino acids of the extracellular domain is retained in polypeptides of the disclosure, which percentage necessarily encompasses the fusion loops FL1 and FL2. In one aspect of the disclosure, the CMV gB polypeptide retains the entire extracellular domain, which domain comprises at least one mutation in one of the two fusion loops FL1 and FL2, possibly both.

By retention of "at least a portion of the cytoplasmic domain" is meant the number of amino acids necessary for expression and secretion. Suitably, CMV gB polypeptides of the disclosure comprise at least 5%, more suitably at least 10%, and it can be 20%, or greater, of amino acids of the cytoplasmic domain. Accordingly, in some embodiments at least 80%, at least 90%, or at least 95% of amino acids of the cytoplasmic domain in the CMV gB polypeptides of the invention is deleted. In a further embodiment, the CMV gB polypeptides of the invention are deleted from the entire cytoplamsic domain.

Mutations of the transmembrane domain, such as for example deletions of at least part of it, may be combined with mutations of the cytoplasmic domain, such as for example deletions of at least part of it. Any combination is contemplated in the present disclosure, so long as the resulting mutated CMV gB polypeptide is secreted from host cells upon expression in said cells, as described above and presents an improved trimerisation profile. Accordingly, in particular embodiments, the CMV gB polypeptides of the invention are deleted from both the entire transmembrane domain and the entire cytoplasmic domain. In some aspects of the disclosure, the CMV gB polypeptides are deleted from the entire transmembrane domain and retain at least part of the cytoplasmic domain, such as, for example, 10% of it. In further aspects of the disclosure, the CMV gB polypeptides are deleted from the entire cytoplasmic domain and retains at least 30% of the transmembrane domain.

Surprisingly, the inventors observed that combining mutations of the fusion loops FL1 and/or FL2 with the deletion of at least part of the cytoplasmic domain, possibly the deletion of all of it, and the deletion of at least part of the transmembrane domain, possibly the deletion of all it, provided polypeptides being more efficiently expressed and secreted than polypeptides of the prior art.

Also, the inventors observed that the signal sequence of the CMV polypeptides of the prior art, after recombinant expression and secretion of the polypeptides, was cleaved off by a signal peptidase at different amino acid positions, generating thus a population of mature gB polypeptides having a varying end at the N-terminus, *i.e.* a population made of gB polypeptides having a different amino acid at the N-terminus. Surprisingly, they observed that introducing mutations into the leader sequence of the gB polypeptide resulted in the signal sequence being cleaved off at mainly one site, generating thus a homogeneous population of mature gB polypeptides having the same amino acid at the N-terminus. Accordingly, in one aspect of the disclosure, there is described a preparation comprising a population of mature CMV gB polypeptides produced after cleavage of the signal sequence, wherein at least 30%, at least 80%, from 80% to 90% of the mature gB polypeptides comprise the same amino acid at the N-terminal end. In particular, the mature polypeptides of the invention suitably start with a serine or a histidine at the N-terminal position. It is an object of the invention to provide a CMV gB polypeptide comprising a deletion of at least 40%, at least 50%, at least 70%, at least 80% or at least 90% of the amino acids of the leader sequence. In one embodiment, there is provided a CMV gB polypeptide comprising the deletion of the entire leader sequence. The inventors also observed that the above deletion of the leader sequence could be combined with mutations occurring in the signal sequence, such as at least one amino acid substitution, suitably in the C-terminal part of the signal peptide. Alternatively, said signal sequence may comprise at least one amino acid insertion, suitably in the C-terminal part of the signal peptide. The extent of the deletion of the leader sequence, as well as the type of mutations in the signal sequence, may vary so long as the resulting gB polypeptide, after recombinant expression and secretion, is cleaved off by a signal peptidase at one major site, generating thus a population of mature gB polypeptides having a the same amino acid at the N-terminus. The identity of the N-terminal amino acid within a population of polypeptides can determined through any known in the art sequencing techniques. Alternatively the different forms of polypeptides within a population of polypeptides may be subjected to a tryptic digestion, and the different peptides generated may be analysed by mass spectroscopy, such as MS/MS. The comparison with standard peptides of known sequences will allow to determine the amino acid composition of the digested peptides.

The inventors noticed that the above deletion of the leader sequence and/or the mutation of the signal sequence could be combined with any of the above-described mutations relating to the Fusion Loops FL1 and FL2 domains and/or to the deletion of at least a portion of the TM domain and/or to the deletion of at least a portion of the cytoplasmic domain, while still maintaining the properties conferred by each type of mutation, such as an improved trimerisation profile, a better expression and secretion, the absence of C-terminal clipping, and homogeneity at the N-terminus after cleavage of the signal sequence, as compared with the CMV gB polypeptides of the prior art, such as gB-DeltaTM. Accordingly, in some aspects of the disclosure, the CMV gB polypeptides comprise mutations in the leader sequence optionally combined with mutations in the signal sequence, any previously described FL1 and/or FL2 domain mutations, and any mutations in the cytoplasmic domain and in the TM domain disclosed therein.

The CMV gB polypeptides of the invention are not "naturally occurring" in the sense that they are not present in the same state as it is in nature, *i.e.* their sequence have been modified artificially. The terms "polypeptide" or "protein" refers to a polymer in which the monomers are amino acids which are joined together through amide bonds. The terms "polypeptide" or "protein" as used herein are intended to encompass any amino acid sequence and include modified sequences such as glycoproteins. The term "polypeptide" is specifically intended to cover both naturally occurring proteins and non-naturally occuring proteins which are recombinantly or synthetically produced. The term "fragment," in reference to a polypeptide, refers to a portion (that is, a subsequence) of a polypeptide. The term "immunogenic fragment" refers to all fragments of a polypeptide that retain at least one predominant immunogenic epitope of the full-length reference protein or polypeptide. Orientation within a polypeptide is generally recited in an N-terminal to C-terminal direction, defined by the orientation of the amino and carboxy moieties of individual amino acids. Polypeptides are translated from the N-terminal or amino-terminus towards the C-terminal or carboxy-terminus.

It is also possible to use in the present invention a gB polypeptide as described above carrying further mutations, such as for instance, mutations at endoproteolytic cleavage sites so that said sites are made ineffectual. For example, the furin cleavage site located around amino acids 456 to 460 of the sequence set forth in SEQ ID NO:1, or at a corresponding position in other CMV gB polypeptides originating from different strains, may be mutated. Suitably, at least one of the amino acid substitutions is performed which is selected from the group consisting of: R⁴⁵⁶ substituted with T⁴⁵⁶, R⁴⁵⁸ substituted with Q⁴⁵⁸, and R⁴⁵⁹ substituted with T⁴⁵⁹, relative to the sequence set forth in SEQ ID NO:1. Accordingly, in some aspects of the disclosure, the CMV gB polypeptides comprise further point mutations wherein the arginine R⁴⁵⁶, R⁴⁵⁸ and R⁴⁵⁹ of the sequence set forth in SEQ ID NO:1, or at a corresponding position in other CMV gB polypeptides originating from different strains, are substituted with a threonine (T⁴⁵⁶), a glutamic acid (Q⁴⁵⁸ ) and a threonine (T⁴⁵⁹), respectively. In some other aspects of the disclosure, the arginine at position 50 and 357 of the sequence as set forth in SEQ ID NO:1 was substituted with a serine. In still further aspects of the disclosure, the gB polypeptides, in particular, the ones comprising a deletion of a hydrophobic region in the C-terminal cytoplasmic domain, comprise a further point mutation wherein the cysteine at position 778 of the sequence set forth in SEQ ID NO:1 is substituted with an alanine.

Optionally, to facilitate expression and recovery, the gB polypeptide of the present disclosure may include a signal peptide at the N-terminus. A signal peptide can be selected from among numerous signal peptides known in the art, and is typically chosen to facilitate production and processing in a system selected for recombinant expression of the gB polypeptide of the present disclosure. In some aspects of the disclosure, the signal peptide is the one naturally present in the native gB protein from the AD169 strain, *i.e.* amino acids 1-20 of the sequence as set forth in SEQ ID NO:1. Alternatively, the signal peptide may be a heterologous sequence in that the sequence arises from a protein distinct from gB. Exemplary signal peptides suitable for use in the context of the CMV gB polypeptides of the disclosure include signal peptides of tissue plasminogen activator (tPA), Herpes Simplex Virus (HSV) gD protein, human endostatin, HIV gp120, CD33, human Her2Neu, or Epstein Barr Virus (EBV) gp350. A "signal peptide" is a short amino acid sequence (e.g., approximately 18-25 amino acids in length) that direct newly synthesized secretory or membrane proteins to and through membranes, e.g., of the endoplasmic reticulum. Signal peptides are frequently but not universally located at the N-terminus of a polypeptide, and are frequently cleaved off by signal peptidases after the protein has crossed the membrane. This cleavage is commonly considered as a step in the process of protein "maturation". In particular, once the signal peptide is cleaved off, the protein is referred to as in its "mature" form. Signal sequences typically contain three common structural features: an N-terminal polar basic region (n-region), a hydrophobic core, and a hydrophilic c-region). According to one aspect of the disclosure, the gB polypeptide comprises the signal peptide naturally present in the native gB. In an alternative aspect of the disclosure, the gB polypeptide comprises a heterologous signal peptide, such as the signal peptide of the protein CD33. The signal sequences can be non native and may comprise mutations, such as substitutions, insertions, or deletions of amino acids. Accordingly, in some aspects of the disclosure, wherein the gB polypeptides comprise a signal peptide, in particular the native signal peptide, said signal peptide comprises at least one amino acid substitution, suitably in the C-terminal part of the signal peptide. Alternatively, said signal peptide may comprise at least one amino acid insertion, suitably in the C-terminal part of the signal peptide.

Optionally, the CMV gB polypeptides of the disclosure can include the addition of an amino acid sequence that constitutes a tag, which facilitates subsequent processing or purification of a recombinantly expressed polypeptide. Such a tag can be an antigenic or epitope tag, an enzymatic tag or a polyhistidine tag. Typically the tag is situated at one or the other end of the polypeptide, such as at the C-terminus or N-terminus of the polypeptide. Accordingly, in some aspects of the disclosure, the CMV gB polypeptides comprise a polyhistidine tag located at the C-terminus of the polypeptides.

In certain embodiments, the CMV gB polypeptide of the invention has an amino acid sequence selected from SEQ ID NO:3, SEQ ID NO:4,SEQ ID NO:5, SEQ ID NO:10, SEQ ID NO:12, SEQ ID NO:14, SEQ ID NO:16 and SEQ ID NO:18. Alternatively, in distinct embodiments, the CMV gB polypeptide of the invention has an amino acid sequence selected from SEQ ID NO:19, SEQ ID NO:20,SEQ ID NO:21.

Another aspect of this disclosure concerns nucleic acids encoding the CMV gB polypeptides of the present invention. Cells into which such nucleic acids or vectors are introduced (*i.e.* host cells) are also part of the invention. The host cells can be bacterial cells, but more commonly will be eukaryotic cells, such a yeast cells (e.g. pichia), plant cells, insect cells, or mammalian cells (e.g. CHO cells). In one aspect of the disclosure, the CMV gB polypeptides are recombinantly expressed in CHO cells.

The terms "polynucleotide" and "nucleic acid sequence" refer to a polymeric form of nucleotides at least 10 bases in length. Nucleotides can be ribonucleotides, deoxyribonucleotides, or modified forms of either nucleotide. The term includes single and double forms of DNA. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. In one aspect of the disclosure, a polynucleotide encodes a polypeptide. The 5' and 3' direction of a nucleic acid is defined by reference to the connectivity of individual nucleotide units, and designated in accordance with the carbon positions of the deoxyribose(or ribose) sugar ring. The informational (coding) content of a polynucleotide sequence is read in a 5' to 3' direction.

A "recombinant" nucleic acid is one that has a sequence that is not naturally occurring or has a sequence that is made by an artificial combination of two otherwise separated segments of sequence. This artificial combination can be accomplished by chemical synthesis or, more commonly, by the artificial manipulation of isolated segments of nucleic acids, e.g., by genetic engineering techniques. A "recombinant" protein is one that is encoded by a heterologous (e.g., recombinant) nucleic acid, which has been introduced into a host cell, such as a bacterial or eukaryotic cell. The nucleic acid can be introduced, on an expression vector having signals capable of expressing the protein encoded by the introduced nucleic acid or the nucleic acid can be integrated into the host cell chromosome.

In some aspects of the disclosure, the recombinant nucleic acids are codon optimized for expression in a selected prokaryotic or eukaryotic host cell, such as a mammalian, plant or insect cell. To facilitate replication and expression, the nucleic acids can be incorporated into a vector, such as a prokaryotic or a eukaryotic expression vector. Although the nucleic acids disclosed herein can be included in any one of a variety of vectors (including, for example, bacterial plasmids; phage DNA; baculovirus; yeast plasmids; vectors derived from combinations of plasmids and phage DNA, viral DNA such as vaccinia, adenovirus, fowl pox virus, pseudorabies, adenovirus, adeno-associated virus, retroviruses and many others), most commonly the vector will be an expression vector suitable for generating polypeptide expression products. In an expression vector, the nucleic acid encoding the CMV gB polypeptide is typically arranged in proximity and orientation to an appropriate transcription control sequence (promoter, and optionally, one or more enhancers) to direct mRNA synthesis. That is, the polynucleotide sequence of interest is operably linked to an appropriate transcription control sequence. Examples of such promoters include: the immediate early promoter of CMV, LTR or SV40 promoter, polyhedron promoter of baculovirus, *E. coli* lac or trp promoter, phage T7 and lambda P_{L} promoter, and other promoters known to control expression of genes in prokaryotic or eukaryotic cells or their viruses. The expression vector typically also contains a ribosome binding site for translation initiation, and a transcription terminator. The vector optionally includes appropriate sequences for amplifying expression. In addition, the expression vectors optionally comprise one or more selectable marker genes to provide a phenotypic trait for selection of transformed host cells, such as dihydrofolate reductase, neomycin resistance or kanamycin resistance for eukaryotic cell culture, or such as tetracycline or ampicillin resistance in *E. coli.*

The expression vector can also include additional expression elements, for example, to improve the efficiency of translation. These signals can include an ATG initiation codon and adjacent sequences. In some cases, for example, a translation initiation codon and associated sequence elements are inserted into the appropriate expression vector simultaneously with the polynucleotide sequence of interest (e.g., a native start codon). In such cases, additional translational control signals are not required. However, in cases where only a polypeptide coding sequence, or a portion thereof, is inserted, exogenous translational control signals, including an ATG initiation codon is provided for expression of the CMV gB sequence. The initiation codon is placed in the correct reading frame to ensure translation of the polynucleotide sequence of interest. Exogenous transcriptional elements and initiation codons can be of various origins, both natural and synthetic. If desired, the efficiency of expression can be further increased by the inclusion of enhancers appropriate to the cell system in use (Scharf et al. (1994) Results Probl Cell Differ 20:125-62; Bitter et al. (1987) Methods in Enzymol 153:516-544).

Exemplary procedures sufficient to guide one of ordinary skill in the art through the production of recombinant CMV gB nucleic acids can be found in Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2003); and Ausubel et al., Short Protocols in Molecular Biology: A Compendium of Methods from Current Protocols in Molecular Biology, 4th ed., Wiley & Sons, 1999.

Exemplary nucleic acids that encode the CMV gB polypeptides of the invention are represented by SEQ ID NOs: 6, 7, 8, 9, 11, 13, 15 and 17. Additional sequence variants that share sequence identity with the exemplary variants can be produced by those of skill in the art. Typically, the nucleic acid variants will encode polypeptides that differ by no more than 1%, or 2%, or 5%, or 10%, or 15%, or 20% of the nucleotide or amino acids. That is, the encoded polypeptides share at least 80%, or 85%, more commonly, at least about 90% or more, such as 95%, or even 98% or 99% sequence identity. It will be immediately understood by those of skill in the art, that the polynucleotide sequences encoding the CMV gB polypeptides, can themselves share less sequence identity due to the redundancy of the genetic code.

It will be understood by those of skill in the art, that the similarity between the CMV gB polypeptides and polynucleotide sequences, as for polypeptide and nucleotide sequences in general, can be expressed in terms of the similarity between the sequences, otherwise referred to as sequence identity. Sequence identity is frequently measured in terms of percentage identity (or similarity); the higher the percentage, the more similar are the primary structures of the two sequences. In general, the more similar the primary structures of two amino acid (or polynucleotide) sequences, the more similar are the higher order structures resulting from folding and assembly. Variants of CMV gB polypeptides and polynucleotide sequences can have one or a small number of amino acid deletions, additions or substitutions but will nonetheless share a very high percentage of their amino acid, and generally their polynucleotide sequence. Methods of determining sequence identity are well known in the art. Various programs and alignment algorithms are described in: Smith and Waterman, Adv. Appl. Math. 2:482, 1981; Needleman and Wunsch, J. Mol. Biol. 48:443, 1970; Higgins and Sharp, Gene 73:237, 1988; Higgins and Sharp, CABIOS 5:151, 1989; Corpet et al., Nucleic Acids Research 16:10881, 1988; and Pearson and Lipman, Proc. Natl. Acad. Sci. USA 85:2444, 1988. Altschul et al., Nature Genet. 6:119, 1994, presents a detailed consideration of sequence alignment methods and homology calculations. The NCBI Basic Local Alignment Search Tool (BLAST) (Altschul et al., J. Mol. Biol. 215:403, 1990) is available from several sources, including the National Center for Biotechnology Information (NCBI, Bethesda, MD) and on the internet, for use in connection with the sequence analysis programs blastp, blastn, blastx, tblastn and tblastx. A description of how to determine sequence identity using this program is available on the NCBI website on the internet.

Another indicia of sequence similarity between two nucleic acids is the ability to hybridize. The more similar are the sequences of the two nucleic acids, the more stringent the conditions at which they will hybridize. The stringency of hybridization conditions are sequence-dependent and are different under different environmental parameters. Thus, hybridization conditions resulting in particular degrees of stringency will vary depending upon the nature of the hybridization method of choice and the composition and length of the hybridizing nucleic acid sequences. Generally, the temperature of hybridization and the ionic strength (especially the Na⁺ and/or Mg⁺⁺ concentration) of the hybridization buffer will determine the stringency of hybridization, though wash times also influence stringency. Generally, stringent conditions are selected to be about 5°C to 20°C lower than the thermal melting point (Tₘ) for the specific sequence at a defined ionic strength and pH. The Tₘ is the temperature (under defined ionic strength and pH) at which 50% of the target sequence hybridizes to a perfectly matched probe. Conditions for nucleic acid hybridization and calculation of stringencies can be found, for example, in Sambrook et al., Molecular Cloning: A Laboratory Manual, Cold Spring Harbor Laboratory Press, Cold Spring Harbor, NY, 2001; Tijssen, Hybridization With Nucleic Acid Probes, Part I: Theory and Nucleic Acid Preparation, Laboratory Techniques in Biochemistry and Molecular Biology, Elsevier Science Ltd., NY, NY, 1993.and Ausubel et al. Short Protocols in Molecular Biology, 4th ed., John Wiley & Sons, Inc., 1999.

For purposes of the present disclosure, "stringent conditions" encompass conditions under which hybridization will only occur if there is less than 25% mismatch between the hybridization molecule and the target sequence. "Stringent conditions" can be broken down into particular levels of stringency for more precise definition. Thus, as used herein, "moderate stringency" conditions are those under which molecules with more than 25% sequence mismatch will not hybridize; conditions of "medium stringency" are those under which molecules with more than 15% mismatch will not hybridize, and conditions of "high stringency" are those under which sequences with more than 10% mismatch will not hybridize. Conditions of "very high stringency" are those under which sequences with more than 6% mismatch will not hybridize. In contrast, nucleic acids that hybridize under "low stringency" conditions include those with much less sequence identity, or with sequence identity over only short subsequences of the nucleic acid. It will, therefore, be understood that the various variants of nucleic acids that are encompassed by this disclosure are able to hybridize to at least one of SEQ ID NOs: 6, 7, 8, 9, 11, 13, 15 and 17, over substantially their entire length.

The CMV gB polypeptides disclosed herein are produced using well established procedures for the expression and purification of recombinant proteins. Procedures sufficient to guide one of skill in the art can be found in, for example, Sambrook and the Ausubel references cited above. Additional and specific details are provided hereinbelow. Recombinant nucleic acids that encode the CMV gB polypeptides, such as (but not limited to) the exemplary nucleic acids represented by SEQ ID NOs: 6, 7, 8, 9, 11, 13, 15 and 17, are introduced into host cells by any of a variety of well-known procedures, such as electroporation, liposome mediated transfection, Calcium phosphate precipitation, infection, transfection and the like, depending on the selection of vectors and host cells. Host cells that include recombinant CMV gB polypeptide-encoding nucleic acids are, thus, also a feature of this disclosure. Favorable host cells include prokaryotic (*i.e.,* bacterial) host cells, such as *E. coli,* as well as numerous eukaryotic host cells, including fungal (*e.g*., yeast, such as *Saccharomyces cerevisiae* and *Picchia pastoris)* cells, insect cells, plant cells, and mammalian cells (such as CHO cells). Recombinant CMV gB nucleic acids are introduced (*e.g*., transduced, transformed or transfected) into host cells, for example, *via* a vector, such as an expression vector. As described above, the vector is most typically a plasmid, but such vectors can also be, for example, a viral particle, a phage, etc. Examples of appropriate expression hosts include: bacterial cells, such as *E. coli, Streptomyces,* and *Salmonella typhimurium;* fungal cells, such as *Saccharomyces cerevisiae, Pichia pastoris,* and *Neurospora crassa;* insect cells such as *Drosophila* and *Spodoptera frugiperda;* mammalian cells such as 3T3, COS, CHO, BHK, HEK 293 or Bowes melanoma; plant cells, including algae cells, etc.

The host cells can be cultured in conventional nutrient media modified as appropriate for activating promoters, selecting transformants, or amplifying the inserted polynucleotide sequences. The culture conditions, such as temperature, pH and the like, are typically those previously used with the host cell selected for expression, and will be apparent to those skilled in the art and in the references cited herein, including, *e.g*., Freshney (1994) Culture of Animal Cells, a Manual of Basic Technique, third edition, Wiley- Liss, New York and the references cited therein. Expression products corresponding to the nucleic acids of the invention can also be produced in non-animal cells such as plants, yeast, fungi, bacteria and the like. In addition to Sambrook, Berger and Ausubel, details regarding cell culture can be found in Payne et al. (1992) Plant Cell and Tissue Culture in Liquid Systems John Wiley & Sons, Inc. New York, NY; Gamborg and Phillips (eds) (1995) Plant Cell. Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL. In bacterial systems, a number of expression vectors can be selected depending upon the use intended for the expressed product. For example, when large quantities of a polypeptide or fragments thereof are needed for the production of antibodies, vectors which direct high level expression of proteins that are readily purified are favorably employed. Such vectors include, but are not limited to, multifunctional *E. coli* cloning and expression vectors such as BLUESCRIPT (Stratagene), in which the coding sequence of interest, *e.g*., a polynucleotide of the invention as described above, can be ligated into the vector in-frame with sequences for the amino-terminal translation initiating Methionine and the subsequent 7 residues of beta-galactosidase producing a catalytically active beta galactosidase fusion protein; pIN vectors (Van Heeke & Schuster (1989) J Biol Chem 264:5503-5509); pET vectors (Novagen, Madison WI), in which the amino-terminal methionine is ligated in frame with a histidine tag; and the like. Similarly, in yeast, such as *Saccharomyces cerevisiae,* a number of vectors containing constitutive or inducible promoters such as alpha factor, alcohol oxidase and PGH can be used for production of the desired expression products. For reviews, see Berger, Ausubel, and, *e.g*., Grant et al. (1987; Methods in Enzymolog 153:516-544). In mammalian host cells, a number of expression systems, including both plasmids and viral-based systems, can be utilized.

A host cell is optionally chosen for its ability to modulate the expression of the inserted sequences or to process the expressed protein in the desired fashion. Such modifications of the protein include, but are not limited to, glycosylation, (as well as, *e.g*., acetylation, carboxylation, phosphorylation, lipidation and acylation). Post-translational processing for example, which cleaves a precursor form into a mature form of the protein (for example, by a furin protease) is optionally performed in the context of the host cell. Different host cells such as 3T3, COS, CHO, HeLa, BHK, MDCK, 293, WI38, etc. have specific cellular machinery and characteristic mechanisms for such post-translational activities and can be chosen to ensure the correct modification and processing of the introduced, foreign protein. For long-term, high-yield production of recombinant CMV gB polypeptide encoded by the nucleic acids disclosed herein, stable expression systems are typically used. For example, cell lines which stably express a CMV gB polypeptide of the invention are obtained by introducing into the host cell expression vectors which contain viral origins of replication or endogenous expression elements and a selectable marker gene. Following the introduction of the vector, cells are allowed to grow for 1-2 days in an enriched media before they are switched to selective media. The purpose of the selectable marker is to confer resistance to selection, and its presence allows growth and recovery of cells which successfully express the introduced sequences. For example, resistant groups or colonies of stably transformed cells can be proliferated using tissue culture techniques appropriate to the cell type. Host cells transformed with a nucleic acid encoding a CMV gB polypeptide are optionally cultured under conditions suitable for the expression and recovery of the encoded protein from cell culture.

Following transduction of a suitable host cell line and growth of the host cells to an appropriate cell density, the selected promoter is induced by appropriate means (*e.g*., temperature shift or chemical induction) and cells are cultured for an additional period. The secreted polypeptide product is then recovered from the culture medium and purified. Alternatively, cells can be harvested by centrifugation, disrupted by physical or chemical means, and the resulting crude extract retained for further purification. Eukaryotic or microbial cells employed in expression of proteins can be disrupted by any convenient method, including freeze-thaw cycling, sonication, mechanical disruption, or use of cell CMV gB polypeptides can be recovered and purified from recombinant cell cultures by any of a number of methods well known in the art, including ammonium sulfate or ethanol precipitation, acid extraction, anion or cation exchange chromatography, phosphocellulose chromatography, hydrophobic interaction chromatography, affinity chromatography (*e.g*., using any of the tagging systems noted herein), hydroxyapatite chromatography, and lectin chromatography. Protein refolding steps can be used, as desired, in completing configuration of the mature protein. Finally, high performance liquid chromatography (HPLC) can be employed in the final purification steps. In addition to the references noted above, a variety of purification methods are well known in the art, including, *e.g*., those set forth in Sandana (1997) Bioseparation of Proteins, Academic Press, Inc.; and Bollag et al. (1996) Protein Methods, 2nd Edition Wiley-Liss, NY; Walker (1996) The Protein Protocols Handbook Humana Press, NJ, Harris and Angal (1990) Protein Purification Applications: A Practical Approach IRL Press at Oxford, Oxford, U.K.; Scopes (1993) Protein Purification: Principles and Practice 3rd Edition Springer Verlag, NY; Janson and Ryden (1998) Protein Purification: Principles. High Resolution Methods and Applications, Second Edition Wiley-VCH, NY; and Walker (1998) Protein Protocols on CD-ROM Humana Press, NJ.

In one example, the polynucleotides that encode the CMV gB polypeptides are cloned into a vector suitable for introduction into mammalian cells (*e.g*., CHO cells). In this exemplary aspect of the disclosure, the polynucleotide sequence that encodes the CMV gB polypeptide is introduced into the pMax vector developed by Amaxa. The polypeptide is expressed under a constitutive promoter, the immediate early CMV promoter. Selection of the stably transfected cells expressing the chimer is made based on the ability of the transfected cells to grow in the presence of kanamycin. Cells that have successfully integrated the pMax are able to grow in the presence of kanamycin, because the pMax vector expresses a kanamycin resistance gene. Selected cells can be clonally expanded and characterized for expression of the CMV gB polypeptides. Alternatively, the polynucleotide sequences that encode the CMV gB polypeptides of the invention may be introduced into the pTT5 vector developed by NRC, which expresses an ampicillin resistance gene.

Following transfection and induction of expression (according to the selected promoter and/or enhancers or other regulatory elements), the expressed polypeptides are recovered (*e.g*., purified or enriched). The following is an exemplary procedure for purifying the CMV gB polypeptides. To facilitate purification, the CMV gB polypeptides include a C-terminal polyhistidine tag. In brief, the cell culture supernatant was collected 6 days post-transfection. After clarification, the supernatants were loaded on nickel columns.

The term "purification" refers to the process of removing components from a composition, the presence of which is not desired. Purification is a relative term, and does not require that all traces of the undesirable component be removed from the composition. In the context of vaccine production, purification includes such processes as centrifugation, dialyzation, ion-exchange chromatography, and size-exclusion chromatography, affinity-purification or precipitation. Thus, the term "purified" does not require absolute purity; rather, it is intended as a relative term. A preparation of substantially pure nucleic acid or protein can be purified such that the desired nucleic acid, or protein, represents at least 50% of the total nucleic acid content of the preparation. In some aspects of the disclosure, a substantially pure nucleic acid, or protein, will represent at least 60%, at least 70%, at least 80%, at least 85%, at least 90%, or at least 95% or more of the total nucleic acid or protein content of the preparation.

In the sense of the present invention, an "isolated" biological component (such as a nucleic acid molecule, or protein) has been substantially separated or purified away from other biological components in the cell of the organism in which the component naturally occurs, such as, other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids and proteins.

The CMV gB polypeptides and nucleic acids of the disclosure are useful in the preparation of medicaments for treating a CMV infection. The CMV gB polypeptides of the invention are particularly suitable as antigens for inclusion in an immunogenic composition, such as vaccines. Accordingly, the present disclosure also describes methods for eliciting an immune response against CMV by administering to a subject an immunologically effective amount of a composition containing any of the CMV gB polypeptides of the disclosure. Subjects may be either HCMV-seropositive or HCMV-seronegative subjects. In some aspects of the disclosure, the subjects are adolescent girls, typically from 12 to 16 years old. Alternatively, the subjects are child bearing-age women. Typically, the group of child bearing-age women represent women who are between 16 and 45 years of age that can become pregnant. In one aspect of the disclosure, it is described a method for eliciting an immune response against CMV comprising the steps of administering to HCMV-seronegative child bearing-age women an immunologically effective amount of a composition comprising a gB polypeptide of the disclosure. In an alternative aspect of the disclosure, the CMV gB polypeptides are used in the preparation of a medicament for preventing and/or treating CMV infection. Suitably, the composition, or medicament, elicits a protective immune response that reduces or prevents infection with CMV and/or reduces or prevents a pathological response following infection with CMV. In particular, the method of the disclosure, wherein child bearing-age women are administered an immunologically effective amount of a composition containing any of the CMV gB polypeptides of the invention, prevents the CMV transmission from mother to foetus, *i.e.* prevents CMV congenital infection. The terms "prevention of HCMV congenital infection in a newborn" means that the newborn is free of HCMV infection at birth. Alternatively, suitable subjects to receive the immunogenic composition of the disclosure are immunocompromised subjects, such as for example, transplanted patients. Accordingly, in some aspects of the disclosure, it is described the use of CMV gB polypeptides of the invention for reducing and/or preventing CMV infection in immunocompromised subjects. In further aspects of the disclosure, it is described methods for eliciting an immune response against CMV comprising the steps of administering CMV gB polypeptides of the invention to immunocompromised subjects.

A "subject" is a living multi-cellular vertebrate organism. In the context of this disclosure, the subject can be an experimental subject, such as a non-human animal, *e.g.*, a mouse, a cotton rat, or a non-human primate. Alternatively, the subject can be a human subject, as indicated above.

An "antigen" is a compound, composition, or substance that can stimulate an immune response by producing antibodies and/or a T cell response in an animal, including compositions that are injected, absorbed or otherwise introduced into an animal. The term "antigen" includes all related antigenic epitopes. The term "epitope" or "antigenic determinant" refers to a site on an antigen to which B and/or T cells respond. The "predominant antigenic epitopes" are those epitopes to which a functionally significant host immune response, *e.g*., an antibody response or a T-cell response, is made. Thus, with respect to a protective immune response against a pathogen, the predominant antigenic epitopes are those antigenic moieties that when recognized by the host immune system result in protection from disease caused by the pathogen. The term "T-cell epitope" refers to an epitope that when bound to an appropriate MHC molecule is specifically bound by a T cell (via a T cell receptor). A "B-cell epitope" is an epitope that is specifically bound by an antibody (or B cell receptor molecule).

An "immune response" is a response of a cell of the immune system, such as a B cell, T cell, or monocyte, to a stimulus. An immune response can be a B cell response, which results in the production of specific antibodies, such as antigen specific neutralizing antibodies. An immune response can also be a T cell response, such as a CD4+ response or a CD8+ response. In some cases, the response is specific for a particular antigen (that is, an "antigen-specific response"), in particular, CMV. If the antigen is derived from a pathogen, the antigen-specific response is a "pathogen-specific response." A "protective immune response" is an immune response that inhibits a detrimental function or activity of a pathogen, reduces infection by a pathogen, or decreases symptoms (including death) that result from infection by the pathogen. A protective immune response can be measured, for example, by the inhibition of viral replication or plaque formation in a plaque reduction assay or ELISA-neutralization assay, or by measuring resistance to pathogen challenge *in vivo.*

Typically, the immune response elicits an immune response characterized by the production of Th1 type cytokines, *e.g.* a Th1-type immune response. A "Th1" type immune response is characterized CD4+ T helper cells that produce IL-2 and IFN-y. In contrast, a "Th2" type immune response is characterized by CD4+ helper cells that produce IL-4, IL-5, and IL-13.

An "immunologically effective amount" is a quantity of a composition (typically, an immunogenic composition) used to elicit an immune response in a subject. Commonly, the desired result is the production of an antigen (*e.g*., pathogen)-specific immune response that is capable of or contributes to protecting the subject against the pathogen, such as CMV. However, to obtain a protective immune response against a pathogen can require multiple administrations of the immunogenic composition. Thus, in the context of this disclosure, the term immunologically effective amount encompasses a fractional dose that contributes in combination with previous or subsequent administrations to attaining a protective immune response.

Also provided are immunogenic compositions, such as vaccines, including a CMV gB polypeptide and a pharmaceutically acceptable diluent, carrier or excipient. An "immunogenic composition" is a composition of matter suitable for administration to a human or animal subject that is capable of eliciting a specific immune response, *e.g.,* against a pathogen, such as CMV. As such, an immunogenic composition includes one or more antigens (for example, polypeptide antigens) or antigenic epitopes, such as for instance, the CMV gB polypeptides of the inventions. An immunogenic composition can also include one or more additional components capable of eliciting or enhancing an immune response, such as an excipient, carrier, and/or adjuvant. In certain instances, immunogenic compositions are administered to elicit an immune response that protects the subject against symptoms or conditions induced by a pathogen. In some cases, symptoms or disease caused by a pathogen is prevented (or reduced or ameliorated) by inhibiting replication of the pathogen (*e.g*., CMV) following exposure of the subject to the pathogen. In the context of this disclosure, the term immunogenic composition will be understood to encompass compositions that are intended for administration to a subject or population of subjects for the purpose of eliciting a protective or palliative immune response against CMV (that is, vaccine compositions or vaccines). The immunogenic compositions according to the disclosure are not limited to compositions comprising CMV gB polypeptides. The present disclosure also describes immunogenic compositions, such as vaccines, comprising the CMV gB polypeptides of the invention and at least one or more CMV antigens selected from the group consisting of pp65, IE1, pUL131, gL, gH, pUL128, pUL130, or any combination thereof. The present disclosure also describes immunogenic compositions, such as vaccines, comprising the CMV gB polypeptides of the invention and at least one or more antigens selected from the group consisting of HPV, Chlamydia, RSV, *Toxoplasma gondii.* Zoster, and Aspergillus.

Numerous pharmaceutically acceptable diluents and carriers and/or pharmaceutically acceptable excipients are known in the art and are described, *e.g.,* in Remington's Pharmaceutical Sciences, by E. W. Martin, Mack Publishing Co., Easton, PA, 15th Edition (1975). The adjective "pharmaceutically acceptable" indicates that the diluent, or carrier, or excipient, is suitable for administration to a subject (*e.g*., a human or animal subject). In general, the nature of the diluent, carrier and/or excipient will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. In certain formulations (for example, solid compositions, such as powder forms), a liquid diluent is not employed. In such formulations, non-toxic solid carriers can be used, including for example, pharmaceutical grades of trehalose, mannitol, lactose, starch or magnesium stearate..

Accordingly, suitable excipients and carriers can be selected by those of skill in the art to produce a formulation suitable for delivery to a subject by a selected route of administration. Excipients include, without limitation: glycerol, polyethylene glycol (PEG), glass forming polyols (such as, sorbitol, trehalose) N-lauroylsarcosine (*e.g*., sodium salt), L -proline, non detergent sulfobetaine, guanidine hydrochloride, urea, trimethylamine oxide, KCI, Ca²⁺, Mg²⁺ , Mn²⁺ , Zn²⁺ (and other divalent cation related salts), dithiothreitol (DTT), dithioerytrol, ß-mercaptoethanol, Detergents (including, *e.g*., Tween80, Tween20, Triton X-100, NP-40, Empigen BB, Octylglucoside, Lauroyl maltoside, Zwittergent 3-08, Zwittergent 3-10, Zwittergent 3-12, Zwittergent 3-14, Zwittergent 3-16, CHAPS, sodium deoxycholate, sodium dodecyl sulphate, and cetyltrimethylammonium bromide.

In certain examples, the immunogenic composition also includes an adjuvant. Suitable adjuvants for use in immunogenic compositions containing CMV gB polypeptides of the invention are adjuvants that in combination with said polypeptides disclosed herein are safe and minimally reactogenic when administered to a subject.

An "adjuvant" is an agent that enhances the production of an immune response in a non-specific manner. Common adjuvants include suspensions of minerals (alum, aluminum hydroxide, aluminum phosphate) onto which antigen is adsorbed; emulsions, including water-in-oil, and oil-in-water (and variants thereof, including double emulsions and reversible emulsions), liposaccharides, lipopolysaccharides, immunostimulatory nucleic acids (such as CpG oligonucleotides), liposomes, Toll Receptor agonists (particularly, TLR2, TLR4, TLR7/8 and TLR9 agonists), and various combinations of such components.

Suitable adjuvants for use in combination with the CMV gB polypeptides of the invention are saponins. Accordingly, immunogenic compositions of the invention may comprise the saponin QS21 (WO8809336A1; US5057540A). QS21 is well known in the art as a natural saponin derived from the bark of *Quillaja saponaria Molina,* which induces CD8+ cytotoxic T cells (CTLs), Th1 cells and a predominant IgG2a antibody response. For the avoidance of doubt reference to QS21 includes OPT-821. In a suitable form of the present disclosure, the immunogenic compositions of the may comprise QS21 in substantially pure form, that is to say, the QS21 is at least 80%, at least 85%, at least 90% pure, for example at least 95% pure, or at least 98% pure. Compositions of the disclosure may comprise QS21 in an amount of between about 1µg to about 100µg, for example between about 1µg and about 60µg or between 10µg and about 50µg, for example, about 10µg, about 12.5µg, about 15µg, about 20µg, about 25µg, about 30µg, about 40µg or in particular about 50µg. In particular, QS21 is present in an amount between about 40µg and 60µg or between 45 and 55µg or between 47 and 53µg or between 48 and 52µg or between 49 and 51 or about 50µg. Alternatively QS21 is present in an amount between 21µg and 29µg or between about 22µg and about 28µg or between about 23µg and about 27µg or between about 24µg and about 26µg, or about 25µg. In some aspects of the disclosure, immunogenic compositions comprising the CMV gB polypeptides comprise QS21 in an amount of about 10µg, for example between about 5µg and 15µg, about 6µg and about 14µg, about 7µg and about 13µg, about 8µg and about 12µg or about 9µg and about 11µg, or about 10µg. In a further aspects of the disclosure, immunogenic compositionscomprise QS21 in an amount of around about 5µg, for example between about 1µg and 9µg, about 2µg and about 8µg, about 3µg and about 7µg, about 4µg and about 6µg, or about 5µg. A suitable amount of QS21 in the human dose of compositions of the disclosure is for example any of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 µg.

The immunogenic compositions comprising the CMV gB polypeptides of the invention may comprise QS21 and a sterol, cholesterol in particular. Such compositions show a decreased reactogenicity when compared to compositions in which the sterol is absent, while the adjuvant effect is maintained. Reactogenicity studies may be assessed according to the methods disclosed in WO 96/33739. The sterol according to the disclosure is taken to mean an exogenous sterol, *i.e.* a sterol which is not endogenous to the organism from which the β-Amyloid antigen preparation may be taken. Suitably the exogenous sterol is associated to the saponin adjuvant as described in WO 96/33739. In a particular aspect of the disclosure, the cholesterol is present in excess to that of QS21, for example, the ratio of QS21:sterol will typically be in the order of 1:100 to 1:1 (w/w), suitably between 1:10 to 1:1 (w/w), and preferably 1:5 to 1:1 (w/w). In particular, the ratio of QS21:sterol being at least 1:2 (w/w). In a particular aspect of the disclosure, the ratio of QS21:sterol is 1:5 (w/w). Suitable sterols include β-sitosterol, stigmasterol, ergosterol, ergocalciferol and cholesterol. In one aspect of the disclosure, the compositions of the invention comprise cholesterol as sterol. These sterols are well known in the art, for example cholesterol is disclosed in the Merck Index, 11th Edn, page 341, as a naturally occurring sterol found in animal fat. Accordingly, in one aspect of the disclosure, immunogenic compositions comprising the CMV gB polypeptides comprise QS21 in its less reactogenic composition where it is quenched with an exogenous sterol, such as cholesterol for example. Several particular forms of less reactogenic compositions wherein QS21 is quenched with an exogenous cholesterol exist. In a specific aspect of the disclosure, the saponin /sterol is in the form of a liposome structure (WO 96/337391). Thus, for example, CMV gB polypeptides of the invention can suitably be employed in immunogenic compositions with an adjuvant comprising a combination of QS21 and cholesterol.

The term "liposome(s)" generally refers to uni- or multilamellar (particularly 2, 3, 4, 5, 6, 7, 8, 9, or 10 lamellar depending on the number of lipid membranes formed) lipid structures enclosing an aqueous interior. Liposomes and liposome formulations are well known in the art. Lipids, which are capable of forming liposomes include all substances having fatty or fat-like properties. Lipids which can make up the lipids in the liposomes can be selected from the group comprising of glycerides, glycerophospholipides, glycerophosphinolipids, glycerophosphonolipids, sulfolipids, sphingolipids, phospholipids, isoprenolides, steroids, stearines, sterols, archeolipids, synthetic cationic lipids and carbohydrate containing lipids. Liposomes may suitably comprise a phospholipid. Suitable phospholipids include (but are not limited to): phosphocholine (PC) which is an intermediate in the synthesis of phosphatidylcholine; natural phospholipid derivates: egg phosphocholine, egg phosphocholine, soy phosphocholine, hydrogenated soy phosphocholine, sphingomyelin as natural phospholipids; and synthetic phospholipid derivates: phosphocholine (didecanoyl-L-α-phosphatidylcholine [DDPC], dilauroylphosphatidylcholine [DLPC], dimyristoylphosphatidylcholine [DMPC], dipalmitoyl phosphatidylcholine [DPPC], Distearoyl phosphatidylcholine [DSPC], Dioleoyl phosphatidylcholine [DOPC], 1-palmitoyl, 2-oleoylphosphatidylcholine [POPC], Dielaidoyl phosphatidylcholine [DEPC]), phosphoglycerol (1,2-Dimyristoyl-sn-glycero-3-phosphoglycerol [DMPG], 1,2-dipalmitoyl-sn-glycero-3-phosphoglycerol [DPPG], 1,2-distearoyl-sn-glycero-3-phosphoglycerol [DSPG], 1-palmitoyl-2-oleoyl-sn-glycero-3-phosphoglycerol [POPG]), phosphatidic acid (1,2-dimyristoyl-sn-glycero-3-phosphatidic acid [DMPA], dipalmitoyl phosphatidic acid [DPPA], distearoyl-phosphatidic acid [DSPA]), phosphoethanolamine (1,2-dimyristoyl-sn-glycero-3-phosphoethanolamine [DMPE], 1,2-Dipalmitoyl-sn-glycero-3-phosphoethanolamine [DPPE], 1,2-distearoyl-sn-glycero-3-phosphoethanolamine DSPE 1,2-Dioleoyl-sn-Glycero-3-Phosphoethanolamine [DOPE]), phoshoserine, polyethylene glycol [PEG] phospholipid (mPEG-phospholipid, polyglycerin-phospholipid, funcitionilized-phospholipid, terminal activated-phosholipid). In one aspect of the disclosure, the liposomes comprise 1-palmitoyl-2-oleoyl-glycero-3-phosphoethanolamine. In one aspect of the disclosure highly purified phosphatidylcholine is used and can be selected fom the group comprising Phosphatidylcholine (EGG), Phosphatidylcholine Hydrogenated (EGG) Phosphatidylcholine (SOY) Phosphatidylcholine Hydrogenated (SOY). In a further aspect of the disclosure, the liposomes comprise phosphatidylethanolamine [POPE] or a derivative thereof. Liposome size may vary from 30 nm to several µm depending on the phospholipid composition and the method used for their preparation. In particular aspects of the disclosure, the liposome size will be in the range of 50 nm to 500 nm and in further aspects 50 nm to 200 nm. Dynamic laser light scattering is a method used to measure the size of liposomes well known to those skilled in the art. Liposomes of the disclosure may comprise dioleoyl phosphatidylcholine [DOPC] and a sterol, in particular cholesterol. Thus, in a particular aspect of the disclosure, immunogenic compositions comprising the CMV gB polypeptides of the invention, such as gB polypeptides having a non-functional transmembrane domain and at least one of the Fusion Loops FL1 and FL2 mutated, possibly both, comprise QS21 in any amount described herein in the form of a liposome, wherein said liposome comprises dioleoyl phosphatidylcholine [DOPC] and a sterol, in particular cholesterol.

Immunogenic compositions of the invention may comprise one or more further immunostimulants. In one aspect of the disclosure, immunogenic compositions comprising the CMV gB polypeptides of the invention as described herein further comprise a lipopolysaccharide, suitably a non-toxic derivative of lipid A, particularly monophosphoryl lipid A or more particularly 3-Deacylated monophoshoryl lipid A (3D - MPL). 3D-MPL is sold under the name MPL by GlaxoSmithKline Biologicals N.A., and is referred throughout the specification as MPL or 3D-MPL. See, for example, US Patent Nos. 4,436,727; 4,877,611; 4,866,034 and 4,912,094. 3D-MPL primarily promotes CD4+ T cell responses with an IFN-γ (Th1) phenotype. 3D-MPL can be produced according to the methods disclosed in GB2220211 A. Chemically it is a mixture of 3-deacylated monophosphoryl lipid A with 3, 4, 5 or 6 acylated chains. In the compositions of the present disclosure small particle 3D-MPL can be used. Small particle 3D-MPL has a particle size such that it can be sterile-filtered through a 0.22µm filter. Such preparations are described in WO94/21292.

Compositions of the disclosure may comprise 3D-MPL in an amount of between about 1µg to about 100µg, for example between about 1µg and about 60µg or between 10µg and about 50µg, for example, about 10µg, about 12.5µg, about 15µg, about 20µg, about 25µg, about 30µg, about 40µg or in particular about 50µg. In particular, 3D-MPL is present in an amount between about 40µg and 60µg or between 45 and 55µg or between 47 and 53µg or between 48 and 52µg or between 49 and 51 or about 50µg. Alternatively 3D-MPL is present in an amount between 21µg and 29µg or between about 22µg and about 28µg or between about 23µg and about 27µg or between about 24µg and about 26 µg, or about 25µg.

In another aspect of the disclosure, the human dose of the immunogenic composition comprises 3D-MPL at a level of about 10µg, for example between 5 and 15µg, suitably between 6 and 14µg, for example between 7 and 13µg or between 8 and 12µg or between 9 and 11µg, or 10µg. In a further aspect of the disclosure, the human dose of the immunogenic composition comprises 3D-MPL at a level of about 5µg, for example between 1 and 9µg, or between 2 and 8µg or suitably between 3 and 7µg or 4 and 6µg, or 5µg. A suitable amount of 3D-MPL in the compositions of the disclosure is for example any of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50µg. In other aspects of the disclosure, the lipopolysaccharide can be a β1-6) glucosamine disaccharide, as described in US Patent No. 6,005,099 and EP Patent No. 0 729 473 B1. One of skill in the art would be readily able to produce various lipopolysaccharides, such as 3D-MPL, based on the teachings of these references. In addition to the aforementioned immunostimulants (that are similar in structure to that of LPS or MPL or 3D-MPL), acylated monosaccharide and disaccharide derivatives that are a sub-portion to the above structure of MPL are also suitable adjuvants. In other aspects of the disclosure, the adjuvant is a synthetic derivative of lipid A, some of which are described as TLR-4 agonists, and include, but are not limited to:
OM174 (2-deoxy-6-o-[2-deoxy-2-[(R)-3-dodecanoyloxytetra-decanoylamino]-4-o-phosphono-□-D-glucopyranosyl]-2-[(R)-3-hydroxytetradecanoylamino]-□-D-glucopyranosyldihydrogenphosphate), (WO 95/14026)
OM 294 DP (3S, 9 R) -3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO 99/64301 and WO 00/0462)
OM 197 MP-Ac DP (3S-, 9R) -3-□(R)-dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127)

Combinations of different adjuvants, such as those mentioned hereinabove, can also be used in compositions with CMV gB polypeptides. For example, as already noted, QS21 can be formulated together with 3D-MPL. The ratio of QS21 : 3D-MPL will typically be in the order of 1 : 10 to 10 : 1; such as 1:5 to 5 : 1, and often substantially 1 : 1. Typically, the ratio is in the range of 2.5 : 1 to 1 : 1 3D-MPL: QS21. Accordingly, in some aspects of the disclosure, immunogenic compositions comprising CMV gB polypeptides of the invention may comprise at least QS21 and 3D-MPL.

The immunogenic compositions comprising the CMV gB polypeptides of the invention may also be suitably formulated with an oil-in-water emulsion. The oil in water emulsion comprises a metabolisable oil (*i.e.* biodegradable). The oil may be any vegetable oil, fish oil, animal oil or synthetic oil, which is not toxic to the recipient and is capable of being transformed by metabolism. Nuts, seeds, and grains are common sources of vegetable oils. Synthetic oils are also suitable. Accordingly, oil-in-water emulsions used in combination with the CMV gB polypeptides of the invention may comprise a metabolisable oil. In one aspect of the disclosure, oil- in-water emulsions comprise squalene (for example between about 4% and 6% [v/v]). The oil-in-water emulsion may further comprise a surfactant. Oil-in-water emulsions of the disclosure comprise one or more surfactants. Suitable surfactants are well known to the skilled person and include, but are not limited to polyoxyethylene sorbitan monooleate (Tween 80, Polysorbate 80), sorbitan triolate (Span 85), phosphatidylcholine (lecithin), polyoxyethylene (12) cetostearyl ether and octoxynol-9 (Triton X-100). In a particular aspect of the disclosure, oil-in-water emulsions comprise polyoxyethylene sorbitan monooleate (Tween 80, Polysorbate 80). In a further aspect, oil in water emulsions of the disclosure comprise polyoxyethylene sorbitan monooleate (Tween 80) and a further surfactant, in particular sorbitan trioleate (Span 85). Oil-in-water emulsions of the disclosure may also comprise a tocol. Tocols are well known in the art and are described in EP0382271. In particular, the tocol is α-tocopherol or a derivative thereof such as alpha-tocopherol succinate (also known as vitamin E succinate). In a particular aspect of the disclosure, there is described immunogenic compositions comprising CMV gB polypeptides of the invention in combination with an oil-in-water emulsion comprising squalene (for example about 5% [v/v]) and α-tocopherol (for example about 5% [v/v]). In a particular aspect of the disclosure, the oil-in-water emulsion comprises a metabolisable oil (*e.g*. squalene), a tocol (*e.g*. α-tocopherol) and a surfactant (*e.g*. polyoxyethylene sorbitan monooleate [Polysorbate 80]). In a further aspect, oil-in-water emulsions of the disclosure comprise a metabolisable oil (*e.g*. squalene), a surfactant (*e.g*. polyoxyethylene sorbitan monooleate [Polysorbate 80]), and optionally a second surfactant (*e.g*. sorbitan trioleate [Span 85]). In a further aspect, oil-in-water emulsions of the disclosure comprise a metabolisable oil (*e.g*. squalene), a polyoxyethylene alkyl ether hydrophilic non-ionic surfactant (*e.g*. polyoxyethylene (12) cetostearyl ether) and a hydrophobic non-ionic surfactant (*e.g*. polyoxyethylene sorbitan monooleate [Polysorbate 80]), or sorbitan trioleate [Span 85]). In some aspects of the disclosure, immunogenic compositions comprising the CMV gB polypeptides of the invention, such as gB polypeptides having a non-functional transmembrane domain and at least one of the Fusion Loops FL1 and FL2 mutated, possibly both, comprise an oil-in-water emulsion comprising squalene, alpha-tocopherol, and Polysorbate 80.

Suitably, the oil-in-water comprises 11 mg metabolisable oil (such as squalene) or below, for example between 0.5-11 mg, 0.5-10 mg or 0.5-9 mg 1-10 mg, 1-11 mg, 2-10 mg, 4-8 mg, or 4.5-5.5 mg, and 5 mg emulsifying agent (such as polyoxyethylene sorbitan monooleate) or below, for example between 0.1-5 mg, 0.2-5 mg, 0.3-5 mg, 0.4-5 mg, 0.5-4 mg, 1-2 mg or 2-3 mg per dose of the vaccine. Suitably tocol (e.g. alpha-tocopherol) where present is 12 mg or below, for example between 0.5-12 mg, 10-11 mg, 1-11 mg, 2-10 mg, 4-9 mg, or 5-7 mg per human vaccine dose.

By the term "vaccine human dose" is meant a dose which is in a volume suitable for human use. Generally this is between 0.25 and 1.5 ml. In one aspect of the disclosure, a human dose is 0.5 ml. In a further aspect, a human dose is higher than 0.5 ml, for example 0.6, 0.7, 0.8, 0.9 or 1 ml. In a still further aspect, a human dose is between 1 ml and 1.5 ml. In still further aspect, in particular when the immunogenic composition is for the paediatric population, a human dose may be less than 0.5 ml such as between 0.25 and 0.5 ml.

An immunogenic composition typically contains an immunoprotective quantity (or a fractional dose thereof) of the antigen and can be prepared by conventional techniques. Preparation of immunogenic compositions, such as vaccines, including those for administration to human subjects, is generally described in Pharmaceutical Biotechnology, Vol.61 Vaccine Design-the subunit and adjuvant approach, edited by Powell and Newman, Plenurn Press, 1995. New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al.*,* U.S. Patent 4,474,757. Typically, the amount of protein in each dose of the immunogenic composition is selected as an amount which induces an immunoprotective response without significant, adverse side effects in the typical subject. Immunoprotective in this context does not necessarily mean completely protective against infection; it means protection against symptoms or disease, especially severe disease associated with the virus. The amount of antigen can vary depending upon which specific immunogen is employed. Generally, it is expected that each human dose will comprise 1-1000 µg of protein, such as from about 1 µg to about 100 µg, for example, from about 1 µg to about 50 µg, such as about 1 µg, about 2 µg, about 5 µg, about 10 µg, about 15 µg, about 20 µg, about 25 µg, about 30 µg, about 40 µg, or about 50 µg. The amount utilized in an immunogenic composition is selected based on the subject population (e.g., infant or elderly). An optimal amount for a particular composition can be ascertained by standard studies involving observation of antibody titres and other responses in subjects. Following an initial vaccination, subjects can receive a boost in about 4 weeks.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds), The Encyclopedia of Molecular Biology, published by Blackwell Science Ltd; 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a", "an", and "the" include plural referents unless context clearly indicates otherwise. It is further to be understood that all base sizes or amino acid sizes, and all molecular weight or molecular mass values, given for nucleic acids or polypeptides are approximate and are provided for description. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, are intended to be approximate. Thus, where a concentration is indicated to be at least (for example) 200 pg, it is intended that the concentration be understood to be at least approximately (or "about" or "∼") 200 pg.

Although methods and materials similar or equivalent to those described herein can be used in the practice or testing of this disclosure, suitable methods and materials are described below. The term "comprises" means "includes". Thus, unless the context requires otherwise, the word "comprises", and variations such as "comprise" and "comprising" will be understood to imply the inclusion of a stated compound or composition (*e.g*., nucleic acid, polypeptide, antigen) or step, or group of compounds or steps, but not to the exclusion of any other compounds, composition, steps, or group thereof. The abbreviation, "*e.g*." is derived from the Latin exempli gratia, and is used herein to indicate a non-limiting example. Thus, the abbreviation "*e.g*." is synonymous with the term "for example."

The following aspects are described in the present disclosure:
a) A cytomegalovirus (CMV) gB polypeptide comprising, in an N-terminal to C-terminal orientation: at least a portion of an extracellular domain comprising a fusion loop 1 (FL1) domain and a fusion loop 2 (FL2) domain, optionally at least a portion of a transmembrane (TM) domain, and at least a portion of a cytoplasmic domain, wherein at least one amino acid of the FL1 domain is substituted or deleted, and wherein the TM domain or portion thereof, if present, is non-functional.
b) The CMV gB polypeptide of the aspect a), wherein the TM domain is made non-functional by deleting the amino acids at position 701-775 relative to the sequence as set forth in SEQ ID NO:1, or at a corresponding position in other CMV gB polypeptides.
c) The CMV gB polypeptide of the aspect a) or b), further comprising at least one amino acid substitution, or deletion, in the fusion loop FL2 domain.
d) The CMV gB polypeptide of any of the aspects a) to c), further comprising at least the deletion of one hydrophobic amino acid region in the cytoplasmic domain of the polypeptide.
e) The CMV gB polypeptide of any one of the aspects a) to d), wherein the one amino acid substitution in the fusion loop FL1 domain consists in the substitution of at least one amino acid among Y.I.Y as located at position 155-157 relative to the sequence as set forth in SEQ ID NO:1, or at a corresponding position in other CMV gB polypeptides, with a polar amino acid which is not an aromatic.
f) The CMV gB polypeptide of the aspect e), wherein the substitution consists in the substitution of at least two amino acids among Y.I.Y as located at position 155-157 relative to the sequence as set forth in SEQ ID NO:1, or at a corresponding position in other CMV gB polypeptides, with a polar amino acid which is not an aromatic.
g) The CMV gB polypeptide of the aspect e) or f), wherein the polar amino acid is selected from the group of positively charged amino acids consisting of lysine (K), histidine (H) and arginine (R).
h) The CMV gB polypeptide of the aspect g), wherein the isoleucine (I) located at position 156 relative to the sequence as set forth in SEQ ID NO:1, or at a corresponding position in other CMV gB polypeptides, is substituted with a histidine (H).
i) The CMV gB polypeptide of the aspect g) or claim h), wherein the tyrosine (Y) located at position 157 relative to the sequence as set forth in SEQ ID NO:1, or at a corresponding position in other CMV gB polypeptides, is substituted with an arginine (R).
j) The CMV gB polypeptide of any of the aspects e) to i), wherein the tyrosine (Y) located at position 155 relative to the sequence as set forth in SEQ ID NO:1, or at a corresponding position in other CMV gB polypeptides, is substituted with a glycine (G).
k) The CMV gB polypeptide of any of the aspects a) to d), wherein the amino acids Y.I.Y as located at position 155-157 in the fusion loop FL1 domain relative to the sequence as set forth in SEQ ID NO:1, or at a corresponding position in other CMV gB polypeptides, are substituted with amino acids G.H.R, respectively.
I) The CMV gB polypeptide of any of the aspects a) to d), wherein the stretch of amino acids Y.I.Y as located at position 155-157 in the fusion loop FL1 domain relative to the sequence as set forth in SEQ ID NO:1, or at a corresponding position in other CMV gB polypeptides, having a hydrophobicity score of + 1.9 as measured by the Kyte and Doolittle scale, is substituted with a stretch of three amino acids having a hydrophobicity score of less than -3, less than - 7, less than -8, as measured by the Kyte and Doolittle scale.
m) The CMV gB polypeptide of any of the aspectsc) to I), wherein the amino acid substitution in the fusion loop FL2 domain consists in the substitution of at least one amino acid among W.L.Y as located at position 240-242 relative to the sequence as set forth in SEQ ID NO:1, or at a corresponding position in other CMV gB polypeptides, with a positively charged amino acid selected from the group consisting of lysine (K), histidine (H) and arginine (R).
n) The CMV gB polypeptide of the aspect m), wherein the amino acid among W.L.Y as located at position 240-242 relative to the sequence as set forth in SEQ ID NO:1, or at a corresponding position in other CMV gB polypeptides, is substituted with a histidine (H).
o) The CMV gB polypeptide of the aspect m) or n), wherein at least the tyrosine (Y) located at position 242 relative to the sequence as set forth in SEQ ID NO:1, or at a corresponding position in other CMV gB polypeptides, is substituted with a histidine (H).
p) The CMV gB polypeptide of any one of the aspects c) to n), wherein the amino acids W.L.Y as located at position 240-242 in the fusion loop FL2 domain relative to the sequence as set forth in SEQ ID NO:1, or at a corresponding position in other CMV gB polypeptides, are substituted with amino acids A.F.H, respectively.
q) The CMV gB polypeptide of any one of the aspects d) to p), wherein the deletion in the cytoplasmic domain corresponds to the region of amino acids 825 to 877 relative to the sequence as set forth in SEQ ID NO:1.
r) The CMV gB polypeptide of any one of the aspects a) to q), wherein said polypeptide is mutated at an endoproteolytic site
s) The CMV gB polypeptide of the aspect r), wherein at least one of the amino acid substitutions is performed which is selected from the group consisting of: R⁴⁵⁶ substituted with T⁴⁵⁶, R⁴⁵⁸ substituted with Q⁴⁵⁸, and R⁴⁵⁹ substituted with T⁴⁵⁹, relative to the sequence as set forth in SEQ ID NO:1, or at a corresponding position in other CMV gB polypeptides.
t) The CMV gB polypeptide of the aspect s), wherein R⁴⁵⁶, R⁴⁵⁸ and R⁴⁵⁹, relative to the sequence as set forth in SEQ ID NO:1, or at a corresponding position in other CMV gB polypeptides, are substituted with T⁴⁵⁶, Q⁴⁵⁸, and T⁴⁵⁹, respectively.
u) The CMV gB polypeptide of any of the aspects a) to t), wherein the arginine (R³⁵⁷) relative to the sequence as set forth in SEQ ID NO:1 is substituted with a serine (S³⁵⁷).
v) The CMV gB polypeptide of any of the aspects a) to u), wherein the arginine (R⁵⁰) relative to the sequence as set forth in SEQ ID NO:1 is substituted with a serine (S⁵⁰).
w) The CMV gB polypeptide of any of the aspects a) to v), wherein the cysteine (C⁷⁷⁸) relative to the sequence as set forth in SEQ ID NO:1 is substituted with an alanine (A⁷⁷⁸).
x) The CMV polypeptide of the sequence as set forth in SEQ ID NO:3.
y) The CMV polypeptide of the sequence as set forth SEQ ID NO:4.
z) The CMV polypeptide of the sequence as set forth SEQ ID NO:5.
aa) A preparation comprising a CMV gB polypeptide, wherein more than 50% of said polypeptide is in a trimeric form, as measured by AUC (Analytical UltraCentrifugation).
bb) An immunogenic composition comprising the CMV gB polypeptide of any of one of the aspects a) to z) admixed with a suitable pharmaceutical carrier.
cc) The immunogenic composition of the aspect bb), further comprising an adjuvant.
dd) A polynucleotide that encodes the CMV gB polypeptide of any one of the aspects a)-z).
ee) A recombinant vector comprising the polynucleotide of the aspect dd).
ff) A host cell transformed with the recombinant vector of the aspect ee).
gg) The CMV gB polypeptide of any of the aspects a) to z), for use in the prevention and/or treatment of a CMV infection.
hh) Use of the CMV gB polypeptide of any one of the aspects a) to z) in the preparation of a medicament for preventing and/or treating CMV infection.
ii) A method for eliciting an immune response against CMV comprising the step of administering to a subject an immunologically effective amount of a composition comprising the CMV gB polypeptide of any of the aspects a) to z).

The invention will be further described by reference to the following, non-limiting, examples.

### Example 1: CMV gB polypeptides

All the gB polypeptide variants (see Figure 1 and Figure 2 for a schematic representation) designated below originate from the amino acid sequence of gB from the CMV strain AD169. Accordingly, the numbering of amino acids when specifying the position of mutations is relative to the sequence of AD169 CMV gB set forth in SEQ ID NO:1. Also, in addition to the specific mutations they each contain, as described below, the following constructs are (i) all deleted from the transmembrane domain (deletion of amino acids 701 to 775) and (ii) all comprise the following point mutations: amino acids R⁵⁰ and R³⁵⁷ substituted with amino acid S.

While the below variants comprised a histidine tag at the C-terminal end of the polypeptide for transient transfection, the sequence of said tag is not included in the SEQ ID disclosed therein.

### 1.1 gB-SLP12

The gB-SLP12 variant comprises 2 series of point mutations targeting the putative fusion loops of gB, FL1 and FL2, respectively. FL1 was mutated by substituting the amino acids ¹⁵⁵Y.I.Y¹⁵⁷ with the amino acids ¹⁵⁵G.H.R¹⁵⁷, while FL2 was mutated by substituting the amino acids ²⁴⁰W.L.Y²⁴² with amino acids ²⁴⁰A.F.H²⁴². This construct codes for a 830 amino acids long protein. The complete amino acid sequence of gB-SLP12 is given in SEQ ID NO:3.

### 1.1.1 Construction of the expression vector pMax-AD169-gB-SLP12

The gB-SLP12 gene sequence (SEQ ID NO:6) was synthesized by GeneArt company. The coding sequence was codon-optimized for expression in CHO cell. *HindIII* and *BamHI* restriction sites were introduced at the 5' end (in front of the start codon) and the 3' end (just after the stop codon), respectively. The codon-optimized gene sequence carries mutations in both the first putative fusion loop (¹⁵⁵Y.I.Y¹⁵⁷ replaced with ¹⁵⁵G.H.R¹⁵⁷) and the second putative fusion loop (²⁴⁰W.L.Y²⁴² replaced with ²⁴⁰A.F.H²⁴²). The synthetic DNA fragment was received as a 2558 bp long DNA insert cloned into pMA vector (GeneArt proprietary plasmid) using *KpnI* and *SacI* cloning sites, generating AD169-gB-SLP12-pMA plasmid. AD169-gB-SLP12-pMA plasmid was restricted with *HindIII* and *BamHI.* The 2530 bp DNA fragment containing gB-SLP12 gene was gel purified and ligated to the mammalian expression vector pMax, a modified version of the pMaxCloning vector of Amaxa. The pMaxCloning vector backbone has been modified in order to eliminate an ATG start codon present in multiple cloning site of the Amaxa commercial vector (between *KpnI* and *HindIII*). After sequence verification by automated DNA sequencing, pMax-AD169-gB-SLP12 recombinant plasmid was used to transiently transfect CHO-S cells.

### 1.2 gB-SLP12-Del2

The deletion of one hydrophobic region encompassing amino acids Pro⁸²⁵ to Lys⁸⁷⁷ was introduced into the gB-SLP12 variant, generating the gB-SLP12-Del2 variant. This construct codes for a 777 amino acids long protein. The complete amino acid sequence of the gB-SLP12-Del2 variant is given in SEQ ID NO:4. The nucleotide sequence encoding for gB-SLP12-Del2 is given in SEQ ID NO:8.

### 1.2.1 Construction of the expression vector pMax-AD169-gB-SLP12-Del2

A 2159 bp DNA fragment encompassing *HindIII* and *ClaI* unique restriction sites present in the AD169-gB-SLP12 coding sequence was amplified by PCR, using AD169-gB-SLP12-pMA plasmid as template and the primers SLP-Fw (SEQ ID NO:22) (5'-GAAAGCGGGCAGTGAGCGGAAGGC-3') and SLP-Rv (SEQ ID NO:23) (5'-TGTCCTCCACGTACTTCACGCGCTGC-3'). The C-terminal part of AD169-gB-SLP12Del2 gene, was also PCR amplified as a 745 bp fragment encompassing *ClaI* and *SacI* restriction sites, using Del2-pMA vector as template and the primers Del-Fw (SEQ ID NO:24) (5'-CCCGAACGACCGAGCGCAGCGAGTCA-3') and Del-Rv (SEQ ID NO:25) (5'-CCAGCTGGCGAAAGGGGGATGTGC-3'). Del2-pMA vector which carries a synthetic DNA fragment coding for the C-term part of gB-SLP12-Del2 gene was constructed by GeneArt company. After restriction with the appropriate enzymes, the PCR fragments were cloned into pMax vector restricted with *HindIII* and *SacI.* After sequence verification by automated DNA sequencing, pMax-AD169-gB-SLP12 recombinant plasmid was used to transiently transfect CHO-S cells.

### 1.3 gB-SLP1-Del2

The deletion of one hydrophobic region encompassing amino acids Pro⁸²⁵ to Lys⁸⁷⁷ was introduced into the gB-SLP1 variant, generating gB-SLP1-Del2 variant. This variant codes for a 777 amino acids long protein. The complete amino acid sequence of gB-SLP1-Del2 variant is given in SEQ ID NO:5.

### 1.3.1 Construction of the expression vector pMax-AD169-gB-SLP1-Del2

The gB-SLP1 gene was first synthesized by GeneArt. The codon-optimized gene sequence carries mutations in the first putative fusion loop (¹⁵⁵Y.I.Y¹⁵⁷ replaced with ¹⁵⁵G.H.R¹⁵⁷). This synthetic gene was received as a 2558 bp DNA fragment, cloned into pMA vector using *KpnI* and *SacI* cloning sites, generating AD169-gB-SLP1-pMA plasmid. The expression vector pMax-AD169-gB-SLP1-Del2 was constructed from AD169-gB-SLP1-pMA and vector plasmid Del2-pMA. pMA-gB-SLP1 vector was restricted with *HindIII* and *ClaI,* and the 1964 bp DNA fragment coding for the N-terminal part of SLP1-Del-2 protein was isolated by gel purification. The vector plasmid Del2-pMA was digested with *ClaI* and *SacI,* and the 420 bp DNA fragment coding for the C-terminal part of gB-SLP1-Del2 protein was isolated by gel purification. These two fragments were ligated together into pMax expression vector restricted with *HindIII* and *SacI,* generating the final expression vector pMax-AD169-gB-SLP1-Del2. After sequence verification by automated DNA sequencing, pMax-AD169-gB-SLP1-Del2 recombinant plasmid was used to transiently transfect CHO-S cells. The nucleotide sequence encoding for gB-SLP1-Del2 is shown in SEQ ID NO:7.

### 1.4 gB-SLP12-Delta113

The deletion of the C-terminal part of the cytoplasmic domain, starting at the amino acid 793 was performed in the gB-SLP12-Delta113 variant, as well as the deletion of the amino acids 701-775 of the transmembrane domain. This variant codes for a 717 amino acid long protein. The complete amino acid sequence of gB-SLP12-Delta113 variant is given in SEQ ID NO:12.

### 1.4.1 Construction of the vector pTT5-gB-SLP12-Delta113

The strategy used to construct pTT5-gB-SLP12-Delta113 expression vector is as follows. First, pMax-AD169-gB-SLP12 vector (see section 1.1.1) was restricted with *HindIII* and *ClaI.* The resulting 1970-bp gB fragment, corresponding to the N-terminal part of gB-SLP12-Delta113 construct, was isolated by gel purification. The C-terminal end of gB-SLP12-Delta113 construct encompassing *ClaI* and *BamHI* sites (just after the stop codon), was synthesized by Geneart and received as a 230-bp long DNA insert (named new-2) cloned into pMA vector (Geneart proprietary plasmid). New-2-pMA vector was restricted with *ClaI* and *BamHI* and the 230-bp insert was gel purified. The two gel purified DNA fragments were ligated together into pMax expression vector previously restricted with *HindIII* and *BamHI*,generating pMax-AD169-gB-SLP12-Delta113 plasmid. Then, serines at position 50 and 357 of AD169-gB-SLP12-Delta113 construct were both reverted to the native arginine, using "QuickChange Multi Site-Directed Mutagenesis" kit from Stratagene (No. 200513). The resulting plasmid, after sequence verification, was digested with restriction enzymes *HindIII* and *BamHI*. The 2200-bp gB fragment was gel purified and ligated into pTT5 vector (NRC, Canada) previously restricted with *HindIII* and *BamHI*, generating the final pTT5-gB-SLP12-Delta113 expression vector. This vector was used to transiently transfect CHO-S cells. The nucleotide sequence encoding for gB-SLP12-Delta113 is shown in SEQ ID NO:11.

### 1.5 gB-SLP12-Delta725

The deletion of part of the transmembrane domain, starting at the amino acid 725, and of the whole cytoplasmic domain was performed in gB-SLP12-Delta725. This variant codes for a 724 amino acid long protein. The complete amino acid sequence of gB-SLP12-Delta725 variant is given in SEQ ID NO:10

### 1.5.1 Construction of the expression vector pTT5-gB-SLP12-Delta725

First, serine at position 50 and 357 of gB-SLP12-Del2 construct (see section 1.2) were both reverted to the native arginine, using the "QuickChange Multi Site-Directed Mutagenesis" kit from Stratagene (No. 200513). The resulting plasmid, after sequence checking, was digested with restriction enzymes *HindIII* and *ClaI,* and the resulting 1970-bp gB fragment was isolated by gel purification. A PCR fragment encompassing the *ClaI* and *BamHI* restriction sites (SEQ ID NO:26) and comprising the following sequence: *ATCGAT*CCCCTGGAGAACACCGACTTCAGGGTGCTGGAGCTGTACTCCCAGAAGGAACTGAGGT CCAGCAACGTGTTCGACCTGGAGGAAATCATGAGAGAGTTCAACAGCTACAAGCAGCGCGTGAA GTACGTGGAGGACAAGGTGGTGGACCCCCTGC CCCCCTACCT GAAGGGCCTGGACGACCTGA TGAGCGGACTCGGGGCTGCTGGAAAGGCCTGA*GGATCC* was also generated. The 1970-bp *HindIII-ClaI* fragment and the PCR DNA restricted with *ClaI* and *BamHI* were ligated together into pTT5 vector (NRC, Canada), between *HindIII* and *BamHI* cloning sites in order to generate the final pTT5-gB-SLP12-Delta725 expression vector. After sequence checking by automated DNA sequencing, the pTT5-gB-SLP12-Delta725 recombinant plasmid was used to transiently transfect CHO-S cells. The nucleotide sequence encoding for gB-SLP1-Delta725 is shown in SEQ ID NO:9.

### 1.6 gB-SLP12-Delta725-LVL759

This variant codes for a 683 amino acid long protein. The complete amino acid sequence of gB-SLP12-Delta725-LVL759 is given in SEQ ID NO:14.

### 1.6.1 Construction of the expression vector pTTS-AD169-Delta725-LVL759

The pTT5-gB-SLP12-Delta725-RR-leader2 plasmid was mutated using the QuickChange II XL Site-Directed Mutagenesis kit from Stratagene (No. 200522) and the primer CAN1498 5'-gtgcatcgtgtgcctgggatccgaggccgtgagccacagg-3' and CAN 1499 5'-cctgtggctcacggcctcggatcccaggcacacgatgcac-3' resulting in the intermediate plasmid pTT5-694-13. This intermediate plasmid was mutated using the same QuickChange II XL Site-Directed Mutagenesis kit and the primer CAN 1650 5'- gtgaacctgtgcatcgtgtgcctggccctggccagccaccgggccaacgagacaa-3' and CAN 1651 5'- ttgtctcgttggcccggtggctggccagggccaggcacacgatgcacaggttcac-3' resulting in the final expression vector pTT5-gB-SLP12-725-LVL759. The nucleotide sequence encoding for gB-SLP12-Delta725-LVL759 is shown in SEQ ID NO:13.

### 1.7 gB-SLP12-Delta725-LVL776

This variant codes for a 683 amino acid long protein. The complete amino acid sequence of gB-SLP12-Delta725-LVL776 variant is given in SEQ ID NO:16.

### 1.7.1 Construction of the expression vector pTT5-AD169-Delta725-LVL776

The above intermediate plasmid pTT5-694-13 was mutated using using the same QuickChange II XL Site-Directed Mutagenesis kit and the primers CAN1648 5'-cctgtgcatcgtgtgcctgggagccctggccagccaccgggccaacgagacaatc-3' and CAN1649 5'-gattgtctcgttggcccggtggctggccagggctcccaggcacacgatgcacagg-3' resulting in a further intermediate plasmid pTT5- LVL758-1. This intermediate plasmid was mutated using the same QuickChange II XL Site-Directed Mutagenesis kit and the primers CAN1697 5'-tggtgtgcgtgaacctgtgcatcgtgctcctgggagccctggcccaccgggccaacgagacaatctac-3' and CAN1698 5'-gtagattgtctcgttggcccggtgggccagggctcccaggagcacgatgcacaggttcacgcacacca-3' resulting in the final vector expression pTT5-gB-SLP12-Delta725-LVL776. The nucleotide sequence encoding for gB-SLP12-Delta725-LVL759 is shown in SEQ ID NO:15.

### 1.8 gB-SLP12-Delta725-CD33

The gB-SLP12-Delta725-CD33 variant codes for a 677 amino acids long protein. This construct is almost identical to gB-SLP12-Delta725 construct except that the N-terminal part of the gB gene, encompassing nucleotides 1 to 192 (the signal sequence and the first 64 amino acids of mature gB protein), were replaced with the signal sequence of CD33 (Taylor et al. 1999, Vol. 274, No. 17: p11505-11512).The complete amino acid sequence of gB-SLP12Delta725-CD33 variant is given in SEQ ID NO 18.

### 1.8.1 Construction of the expression vector pTT5-gB-SLP12-Delta725-CD33

The gB-SLP12-Delta725-CD33 gene was constructed by PCR amplification using pTT5-gB-SLP12-Delta725 plasmid as a template and the primers CD33 (SEQ ID NO:27) (5'-AATCAAAAGCTTACTAGTGCCGCCACCATGGCCCCCCTGCTGCTTCTGCTGCCCCTGCTTTGGG CAGGGGCCCTGGCCCACCGGGGCAACG-3') and CMO578 (SEQ ID NO:28) (5'-GTAATAGGATCCGGTACCTCATCAGGCCTTTCCAGCAG-3'). The forward primer contains the *HindIII* site and the CD33 signal sequence. The reverse primer contains the stop codon and the *BamHI* site. After restriction with the appropriate enzymes, the PCR fragment was cloned into pEE14 vector restricted with *HindIII* and *BgIII.* The pEE14-gB-SLP12-Delta725-CD33 recombinant plasmid was selected, after sequence verification. Finally, the gB-SLP12-Delta725-CD33 gene was sub-cloned into pTT5 vector as follows. The coding sequence was PCR amplified using pEE14- gB-SLP12-Delta725-CD33 vector as a template and the primers CD33F (SEQ ID NO:29) (5'-AATCAAAAGCTTACTAGTGCCGCCACCATGGCCCCCCTGCTG-3') and Ecto-Rv (SEQ ID NO:30) (5'-GACTTATAGGATCCTCATCAGTGGTGGTGATGATGGTGGCCGCCGGCCTTTCCAGCAGC-3').

Forward and reverse primers contain the *HindIII* and *BamHI* cloning sites, respectively. Amplified DNA and pTT5 vector were restricted with *HindIII* and *BamHI* before ligation. The resulting pTT5-gB-SLP12-Delta725-CD33 expression vector, after sequence verification, was used to transiently transfect CHO-S cells. The nucleotide sequence encoding for gB-SLP12-Delta725-CD33 is shown in SEQ ID NO:17.

### Example 2: Expression of CMV gB polypeptides

The different DNA constructs described above were transiently transfected in CHO (Chinese Hamster Ovary) cells, using the FreeStyle™ MAX CHO expression system from Invitrogen. The construct encoding the polypeptide gB-DeltaTM was used as a control. The amino acid sequence of gB-Delta TM is depicted in SEQ ID NO:2. The expression vector used for expressing gB-DeltaTM was pMax-AD169. FreeStyle™ MAX CHO system uses CHO-S cell line, a separate sub-clone of the common CHO-K1 cell line adapted to suspension and a synthetic cationic lipid-based polymer as transfection reagent. Plasmid DNA for transfection was isolated using Qiagen Maxiprep kit (Qiagen, Valencia, CA) following manufacturer's protocol. The transfection complex was prepared as recommended by Invitrogen. Briefly 37.5 µg of plasmid and 37.5 µl of FreeStyle MAX transfection reagent were diluted separately in 0.6 ml of Opti-Pro™SFM medium. Right after, the diluted FreeStyle MAX transfection reagent was added to the diluted DNA solution. The DNA-FreeStyle MAX mix was incubated for 10 minutes at room temperature before adding to the cell culture. Transfected cultures were incubated at 37°C for 3 days. Then, the cultures were maintained at 29°C for another 3 days period. On day 6 post-transfection, 1 ml of each cell culture comprising the transfected cells in suspension as well as the cell culture medium was collected. The cell culture supernatants were separated from the suspended cells by centrifugation at 12,000 g. Supernatants were collected and cellular pellets were solubilized with 1 ml of lysis buffer (PBS supplemented with 1% Triton X-100). The insoluble material was removed from the cell lysates by centrifugation at 12,000 g at 4°C for 5 min. Aliquots (15 µl) of culture supernatants and solubilized cellular fractions were run on Criterion XT 4-12% Bis Tris gel (Biorad) using MOPS buffer. The proteins were transferred to nitrocellulose membranes and the membranes probed with the anti-gB antibody MAb 27-156 (Spaete *et al.,* 1990). Alternatively, the expression and secretion level were detected by Elisa assay using an anti-gB antibody. The assay was performed in 96 well plates. Plates were coated with a polyclonal anti-gB antibody (the antibody was produced by injecting the polypeptide gB** - described in EP0759995- in rabbits). After a saturation step in Skim Milk 3 % for 1h at 37°C, the antibody was incubated overnight at 4°C. The wells were then washed 4 times. A series of ten 2-fold dilutions of each sample to be tested (supernatant and cellular fractions transfected with given gB constructs) were added to the coated plates in the presence of a monoclonal anti-gB antibody (the antibody was produced by injecting mice with a whole CMV virus) which is biotin-conjugated for 2h at 37°C. After 4 washes with PBS/Tween 20 0.1%, 100 µl of streptavidin-horseradish peroxidase was added and incubated for 30 min at 37°C. Plates were washed 4 times with PBS/Tween 20 0.1% and once with water. Then, the plates were incubated for 10 min at 37°C with 100 µl of Tetra-methyl-benzidine 75% (TMB) in 0.1 M citrate buffer pH 5.8 (25%). This reaction was stopped with 100 µl of H₂SO₄ 0.4N and the colorimetry was read in a spectrophotometer at 450/620 nm. A previous lot of purified gB-DeltaTM whose concentration was known was used as a standard in this experiment. Using the software SoftMax Pro, the concentration of each sample to be tested was quantitated.

### 2.1 Expression and secretion of gB-SLP12, gB-SLP1-Del2 and gB-SLP12-Del2 - Western blot analysis

Shown in FIG.3 is a representative Western blot out of three experiments performed independently - The lanes A, B, C and D represent the cellular fractions of the cultures transfected with gB-DeltaTM, gB-SLP12, gB-SLP1-Del2 and gB-SLP12-Del2, respectively. The level of gB present in said cellular fractions is indicative of the level of gB which is intracellular. The lanes E, F, G and H represent the supernatants of the cultures transfected with gB-DeltaTM, gB-SLP12, gB-SLP1-Del2 and gB-SLP12-Del2, respectively. The level of gB present in said supernatants is indicative of the level of gB which is secreted. If comparing the level of gB present in lanes B, C and D with the level of gB present in lane A, it is noted that the level of intracellular gB remains unchanged when mutating the fusion loops, *i.e*. the polypeptides of the invention, as compared to gB-DeltaTM with intact fusion loops, *i.e.* the polypeptide of the prior art, suggesting that mutations in fusion loops do not affect secretion. Indeed, if comparing the level of gB present in lanes F, G and H with the level of gB present in lane E, it is observed that the expression of the gB polypeptides of the invention (with mutated fusion loops) and their secretion are at least as efficient as the expression and secretion of gB polypeptide of the prior art (gB-DeltaTM with intact fusion loops).

### 2.2 Expression and secretion of gB-SLP12, gB-SLP12-Delta113 and gB-SLP12-Delta725 - Elisa assay

Quantitation of the results obtained by Elisa assay and represented in the form of a graph is shown in FIG. 4 - The black bars represent the cellular fractions of cultures transfected with gB-DeltaTM, gB-SLP12, gB-SLP12-Delta113 and gB-Delta725, respectively, while grey bars represent the supernatants of the same cultures. Accordingly, the sum of the gray bar and of the black bar per expressed variant represents the level of total expression achieved after transfection in CHO cells. With respect to gB-DeltaTM, it was observed that within the population of the expressed polypeptide, around half of the polypeptide is retained intracellularly (gray bar) and around half of the polypeptide is secreted (black bar), Surprisingly, when gB-SLP12 was expressed, it was observed that the level of total expression was significantly higher than for gB-DeltaTM, around 2-fold. Moreover, if comparing the respective level of the intracellular form and the secreted form, it was observed that more than 80% of the polypeptide was secreted, while only 10-20% remained intracellular, suggesting that gB-SLP12 was also more efficiently secreted than gB-DeltaTM. This result suggests that the mutation of the fusion loops has a positive impact on expression and secretion. The higher expression and secretion level over gB-DeltaTM was similarly observed with the polypeptides gB-SLP12-Delta113 and gB-SLP12-Delta725. However, surprisingly, those two polypeptides were even more significantly expressed than gB-SLP12, more than 2-fold and around 1.6-fold, respectively, suggesting that combining the deletion of at least part of the cytoplasmic domain with the fusion loop mutation further improves the expression, and thus, the final yield of the resulting secreted polypeptides..

### 2.3 Expression and secretion of gB-SLP12, gB-SLP12-Delta113, gB-SLP12-Delta725, gB-SLP12-- Role of the fusion loops

Western Blots are shown in FIG. 5 - FIG 5A shows that gB-SLP12 is secreted at least as efficiently as gB-SLP12-Delta725, both of which showing a higher secretion level than gB-DeltaTM (compare lanes F and J with lane B), confirming the results observed by Elisa assay. The lanes M and N represent the cellular fractions and the supernatant of a culture transfected with gB-SLP12-Delta725 having intact fusion loops FL1 and FL2. The absence of detection of gB in the culture supernatant (see lane N) suggests that the good expression/secretion level of gB requires that the fusion loops be mutated - A similar observation was made in FIG. 5B where no gB protein can be detected in the supernatant of a culture transfected with gB-Delta725 having intact fusion loops FL1 and FL2, as compared with the supernatant of a culture transfected with gB-SLP12-Delta725 (compare lanes 6 and 2, respectively). The requirement of the mutation of the fusion loops FL1 and FL2 for achieving a better secretion than gB-DeltaTM was similarly observed for the polypeptides gB-SLP12-Delta113. gB was barely detectable in the supernatant of a culture transfected with gB-SLP12-Delta113 having intact fusion loops FL1 and FL2, as compared with the supernatant of a culture transfected with gB-SLP12-Delta113 (compare lanes 6' and 4', respectively).

### 2.4 Expression and secretion of gB-SLP12-Delta725, gB-SLP12-Delta725-LVL759, and gB-SLP12-Delta725-LVL776

A Western Blot is shown in FIG. 5C - The level of gB present in the supernatant of a culture transfected with gB-SLP12-Delta725-LVL759 is at least as good as the level of gB present in the supernatant of a culture transfected with gB-SLP12-Delta725 (compare lanes f and h with lanes b and d, respectively). The level of gB present in the supernatant of a culture transfected with gB-SLP12-Delta725-LVL776 is only slightly lower than the level of gB present in the supernatant of a culture transfected with gB-SLP12-Delta725 (compare lanes j and l with lanes b and d, respectively). These results indicate that the mutation in the N-terminal part in polypeptides (see FIG. 2) of the invention does not significantly impact the secretion level of said polypeptides.

### Example 3: Product profile of CMV gB polypeptides

gB-DeltaTM, gB-SLP12 and gB-SLP1-Del2 were transiently expressed in CHO cells as described in Example 2, and the culture supernatants were collected at day 6 post-transfection. After clarification, the supernatants were supplemented with 350 mM NaCl and 0.4% Empigen and then 0.22 µm filtrated. Nickel columns were used to purify the transfected and secreted polypeptides from cell corresponding culture supernatants. XK 16 columns (Qiagen) were equilibrated with a buffer comprising 10 mM TRIS-HCl, 350 mM NaCl, 10 mM imidazole and 0.4% Empigen pH 8.0. Cell culture supernatants, previously clarified, supplemented and filtrated, were loaded at a flow rate of 4 ml/min and retained proteins were washed with the equilibration buffer. Elution was performed at a flow rate of 4 ml/min with a buffer comprising 10 mM TRIS HCl, 350 mM NaCl, 350 mM imidazole and 0.4% Empigen pH 8.0. The eluted fractions were then injected at a flow rate of 2 ml/min in XK 26 columns (GE) equilibrated with a buffer comprising 10 mM phosphate (K) buffer and 0.4% Empigen pH 7.2. The first protein peak was harvested for further analysis. The eluted fractions corresponding to this peak were then loaded on cellufine sulphate columns (Chisso Corporation) equilibrated with a buffer comprising 10 mM phosphate (K) and 0.4% Empigen pH 7.2. Elution was performed at a flow rate of 3 ml/min with a buffer comprising 5 mM phosphate (K), 1M NaCl and 0.1% Pluronic F68 pH 7.2. The purified polypeptides resulting from the above purification process were then subjected to the following experiments.

### 3.1 Glutaraldehyde cross-linking experiments

Glutaraldehyde was added directly to 15 µl of each purified polypeptide (corresponding to approximately 7 µg of total protein) so as to obtain a final concentration of glutaraldehyde of 0.5% and 1%. As a control, a sample of each purified polypeptide was left without glutaraldehyde. The mixtures were left incubated for 150 min and, then, the glutaraldehyde reaction was stopped by adding NaBH₄ (Aldrich) and the samples were incubated on ice for 20 min. Samples were then run on Criterion XT 4-12% Bis Tris gel (Biorad) using MOPS buffer. The gel was stained with Coomassie blue R-250 and is shown in FIG. 9.

### Results

If multimers of polypeptides were present in the samples before being treated with glutaraldehyde, then mutimers of any given size, whether dimers, trimers, or any other high molecular weight multimers, were cross-linked and, thus, maintained as such when migrating on a gel in denaturing conditions. Accordingly, said multimers migrated according to their molecular weight. On the contrary, with control samples which were not treated with glutaraldehyde, any multimer present in said sample was denatured into monomers when migrating on a gel in denaturing conditions, so that polypeptides in control samples migrated according to the molecular weight equivalent to a monomer of the polypeptide (see a single band a bit lower than 150 kDa in lanes A, D and G). If considering the multimer profile of gB-DeltaTM (lanes A, B and C), one observes 2 bands of very high molecular weight (indicated by the arrows) in samples treated with glutaraldehyde. One notes also that these two bands are of the same intensity, suggesting that the gB-DeltaTM sample comprises mostly two types of multimers and that the proportion of each mutlimer within the sample should be around the same. On the contrary, when considering the profile of gB-SLP12, in particular, lanes E and F, wherein samples were treated with glutaraldehyde, while two high molecular weight bands were also observed, the intensity of each band is significantly different. Indeed, the intensity of the lower molecular weight band is at least 3-5 times stronger than the highest one, suggesting that the lower molecular weight multimer population within the gB-SLP12 sample is much larger than the population of the higher molecular weight multimer. A similar observation was made when considering the gB-SLP1-Del2 sample (lanes G, H and I), where the intensity of the lower band is around 2-3 times stronger than the intensity of the higher band. This suggests that gB-SLP12 and gB-SLP1-Del2 have a similar multimer profile with an enriched proportion of the lower molecular weight multimers, which is different from gB-DeltaTM, the polypeptide of the prior art. It is to be noted that the gel used in that experiment does not provide a resolution good enough to determine whether the lower molecular weight bands observed in lanes relating to gB-DeltaTM are of the same size as the ones observed in lanes relating to gB-SLP12 and gB-SLP1-Del2. For the same reason, it is not possible to determine precisely the actual size of each band, so that identifying the observed multimers as dimers or trimers, etc... from that experiment is difficult. In order to get this information, the two following experiments were performed with the purified polypeptides.

### 3.2 Analytical Ultracentrifugation-gB-DeltaTM, gB-SLP12, gB-SLP1-Del2

Analytical ultracentrifugation (AUC) was used to determine the homogeneity and size distribution in solution of the different species (e.g. multimers of different sizes of a purified polypeptide which aggregates) within a purified polypetide sample by measuring the rate at which molecules move in response to a centrifugal force. This is based on the calculation of the coefficient of sedimentation of the different species that are obtained by sedimentation velocity experiment, which depend on their molecular shape and mass. After purification, gB-DeltaTM, gB-SLP12 and gB-SLP1-Del2 resuspended in 10 mM sodium phosphate, 1 M NaCl and 0.1% Pluronic, pH 7.2 were spun in a Beckman-Coulter ProteomeLab XL-1 analytical ultracentrifuge at 28 000 rpm after the AN-60Ti rotor had been equilibrated to 15°C. For data collection, 160 scans were recorded at 280nm and 230nm every 5 min. Data analysis was performed using the program SEDFIT for determination of the C(S) distribution. Determination of the partial specific volume of the proteins was performed with the SEDNTERP software from a combination of their amino acid sequence and of their expected glycan composition. SEDNTERP was also used to determine the viscosity and the density of the buffer, by neglecting the contribution of the Pluronic F68, which is not represented in the database of this software. The results are presented in the form of a graph representing the concentration of each species plotted against molecular weight. Determination of the relative abundance of all species has been performed by considering the total area under the curve of the overall distribution as 100% of the sample and by calculating the percentage of this total area represented by the contribution of every species.

### Results

FIG. 10 successively displays the profile of purified gB-DeltaTM, gB-SLP12 and gB-SLP1-Del2 as analysed by analytical ultracentrifugation. The graph corresponding to the gB-DeltaTM sample indicates a heterogeneous distribution represented by a plurality of peaks. In particular, the major population observed in said sample are species of 417 kDa (35%) and 723 kDa (30%), which are likely to represent trimers and hexamers, respectively. Higher molecular weight multimers are also present within said sample. On the contrary, when fusion loops FL1 and FL2 were mutated, the profile of the corresponding purified gB-SLP12 polypeptide indicates a more homogenous population, as the major population is a species of 327 kDa (63%) which is likely to represent trimers. The presence of higher molecular weight multimers is barely noticeable. A similar profile was observed in the gB-SLP1-Del2 sample, wherein the major population is a species of 311 kDa (67%) which is likely to represent trimers also, while the presence of higher molecular weight multimers is only marginal.

In conclusion, the gB polypeptides of the invention (gB-SLP12 and gB-SLP1-Del2) present an improved product profile, as the trimers population is increased of an approximately 2-fold factor, as compared with the trimer population of the gB polypeptide of the prior art (gB-DeltaTM).

### 3.3 Analytical Ultracentrifugation-gB-SLP12-Delta725, gB-SLP12-Delta113

Analytical Ultracentrifugation was performed as described above in section 3.2 to determine the homogeneity and size distribution in solution of the different species within the purified polypeptides gB-SLP12-Delta725 and gB-SLP12-Delta113. The polypeptides were recombinantly expressed as described in Example 2 and purified as described in the first paragraph of the present Example 3. After purification, gB-SLP12-Delta725 and gB-SLP12-Delta113 were each resuspended either in a buffer comprising 0.1 % Pluronic or in a buffer with no Pluronic.

### Results

- FIG. 11A successively displays the profile of purified gB-SLP12-Delta113 polypeptide as analysed by analytical ultracentrifugation in the presence or absence of 0.1 % Pluronic. In the absence of Pluronic, the profile observed was similar to the profile observed for gB-SLP12 and gB-SLP12-Del2 in FIG. 10, with a similar 72% population representing trimers within the population of the purified polypeptide, *i.e.* an improved product profile as compared with the gB polypeptide of the prior art (gB-DeltaTM). However, in the presence of Pluronic, the percentage of trimers was reduced with a concomitant increase of the percentage of monomers (25%) and the presence of multimers of a size around 230 kDa. This tendency of the gB-SLP12-Delta113 polypeptide to monomerize in the presence of Pluronic is indicative of a susceptibility to detergent.
- FIG. 11B successively displays the profile of purified gB-SLP12-Delta725 polypeptide as analysed by analytical ultracentrifugation in the presence or absence of 0.1% Pluronic. The profile observed with this purified polypeptide is similar whether or not Pluronic is present. Indeed, the percentage of trimers in both cases is around 70%, *i.e.* as high as the trimer percentage observed with the above gB-SLP12 and gB-SLP12-Del2 polypeptides, and thus, gB-SLP12-Delta725 also displays an improved product profile as compared with the gB polypeptide of the prior art (gB-DeltaTM).

### Conclusion

While both gB-SLP12-Delta113 and gB-SLP12-Delta725 show an improved product profile, with an enriched proportion of trimers, the results obtained indicate that gB-SLP12-Delta725 presents the additional advantage of having an increased structural stability in solution, as its trimer profile is maintained in the presence or absence of Pluronic. Indeed, a detergent treatment had no observable impact on the sedimentation properties of that polypeptide.

### 3.4 Size-exclusion chromatography

The protein size of purified gB-DeltaTM and gB-SLP12 was also determined by size-exclusion chromatography using a UV-detector at 280 nm and a TSK G5000PWXL column (Tosoh Bioscience). The samples were loaded at room temperature at a flow rate of 0.5ml/min. The elution was performed with PBS + 0.1% Pluronic at the same flow rate at room temperature. Thyroglobulin (669 kDa), ferritin (440 kDa), catalase (232 kDa), aldolase (158 kDa), albumin (68 kDa), conalbumine (75k Da), ovalbumin (43 kDa), carbonic anydrase (29 kDa), and ribonuclease (13.7kDa) were used for calibration.

### Results

As shown on FIG. 13, gB-SLP12 has a size of 357 kDa, as measured by size-exclusion chromatography, which is likely to represent the trimeric form. Two polypeptides of the prior art, gB-DeltaTM and gB-S50-DeltaTM, *i.e.* polypeptides with intact fusion loops, have a similar size of 710 kDa which represent higher molecular weight multimers. These results confirm the above results obtained with analytical ultracentrifugation in that the polypeptides of the invention (gB-SLP12) have a product profile distinct from the polypeptides of the prior art (gB-DeltaTM).

### Example 4: C-terminal clipping of gB-SLP12 polypeptides

### 4.1 Expression and purification

The gB-DeltaTM, gB-SLP12, gB-SLP12-Delta113 and gB-SLP12-Delta725 polypeptides were transiently transfected and expressed as described in Example 2. gB-SLP12, gB-SLP12-Delta113 and gB-SLP12-Delta725 polypeptides were purified as described in the first paragraph of the Example 3. After collection on day 6 post-transfection,the gB-DeltaTM supernatant was supplemented with Bis-Tris and Empigen BB to reach the final concentration of 20 mM and 0.4%, respectively. It was then loaded onto a Q-Sepharose XL column (GE Healthcare) equilibrated in a buffer comprising 20 mM Bis-Tris - 0.4% Empigen BB, pH 6.0. The bound polypeptide was eluted from the column with a mixture of 75% of the buffer comprising 20 mM Bis-Tris - 0.4% Empigen BB, pH 6.0 and 25% of the buffer comprising 20 mM Bis-Tris - 0.4% Empigen BB - 1 M NaCl, pH 6.0. The eluate was then supplemented with sodium phosphate to reach a final concentration of 10 mM and adjusted to pH 6.8. It was then loaded onto a Hydroxyapatite type II column (Bio-Rad) equilibrated in a buffer comprising 10 mM sodium phosphate - 0.4% Empigen BB - 0.25M NaCl, pH 6.8. The polypeptide-containing flow-through was recovered and loaded onto an Cellufine Sulfate column (JNC Corporation) equilibrated in a buffer comprising 10 mM sodium phosphate - 0.4% Empigen BB - 0.25 M NaCl, pH 6.8 buffer. The polypeptide-containing flow-through was recovered, concentrated by ultrafiltration using a 100kD Pellicon XL membrane (Millipore), and loaded onto a Superdex 200 column (GE Healthcare) equilibrated in a buffer comprising 10 mM sodium phosphate - 0.4% Empigen BB, pH 7.2. The eluate was collected, concentrated and diafiltered with 10 mM potassium phosphate, pH 7.2.

### 4.2 Western-Blot analysis

The above purified polypeptides were deglycosylated using the N-Glycosidase F deglycosylation kit (Roche, No. 11836552001) following the manufacturer's instructions. Equivalent amounts of each of the purified deglycosylated polypeptide were run on Criterion XT 4-12% Bis Tris gel (Biorad) using MOPS buffer. The proteins were transferred to nitrocellulose membranes and the membranes probed with either the anti-gB antibody MAb 27-156 (Spaete et al., Virology 167, 207-225 (1988) (FIG. 14A, FIG. 14B, lane 3, FIG. 14C, lanes B, and FIG. 14D, lane D) or the anti-6X His tag antibody (Abcam, No. ab9108) (FIG. 14B, lane 2, FIG. 14C, lanes A, and FIG. 14D, lane C).

### Results

- FIG. 14A shows that the anti-gB antibody recognizes two different forms in the population of purified gB-DeltaTM polypeptide, suggesting that the polypeptide exists in at least two forms of different sizes, 92 kDa and 80 kDa, respectively, in the purified population. Based on the mean value of the molecular weight of an amino acid (110 Da), the molecular weight of gB-DeltaTM, which comprises 830 amino acids, should be around 91 kDa. Therefore, the 92 kDa band is likely to correspond to the full length gB-DeltaTM polypeptide, while the 80 kDa band is likely to correspond to a shorter version of the polypeptide, suggesting that within the population, some polypeptides are cleaved and clipped products of around 80 kDa are produced.
- FIG. 14B shows that, upon recombinant expression of the gB-SLP12 polypeptide in cells, the anti-gB antibody (lane 3) provides an identical profile recognizing two forms of similar sizes, 92 kDa and 80 kDa, respectively, suggesting that a similar cleavage occurred in this polypeptide. It is noted here that the expected molecular weight of gB-SLP12 is also around 91 kDa, as the number of amino acids is the same as for gB-DeltaTM. When probing the membrane with an anti-His tag antibody (lane 2), only the band at around 92 kDa was detected. As the His-tag is located at the C-terminal end of the polypeptide, these data suggest that the cleavage occurs in the C-terminal part of the polypeptide, and generates thus a fragment of 80 kDa missing the C-terminal end.

- FIG. 14C shows that, upon recombinant expression of the gB-SLP12-Delta113 polypeptide in cells, the anti-gB antibody (lane B) only recognizes one band (at around 80 kDa). As gB-SLP12-Delta113 comprises 717 amino acids, then its expected mean molecular weight should be around 79 kDa. Therefore, the observation of one band only at around 80 kDa indicates that the population of this purified polypeptide is made of the full length polypeptide only, suggesting that the previously observed cleavage in the C-terminal part no longer occurs in this population of polypeptides. This was confirmed by probing the membrane with an anti-His tag antibody (lane A) and observing an identical band at around 80 kDa, indicating that the C-terminal end of the polypeptide is present.
- A similar conclusion can be drawn from the Western blot presented in FIG. 14D relating to the polypeptide gB-SLP12-Delta725. Lane D represents the membrane probed with an anti-gB antibody which recognizes only one band (at around 80 kDa). The expected mean molecular weight of this polypeptide comprising 824 amino acids should be around 79 kDa also. Lane C represents the membrane probed with an anti-His tag antibody and similarly recognizes only one band at around 80 kDa, indicating that the C-terminal end of the polypeptide is present
- Polypeptides may precipitate in the course of deglycosylation, which can result in an incomplete deglycosylation. When the deglycosylation is incomplete, some polypeptides remain in their glycosylated form, which forms typically migrate in the gel in a diffuse manner, such as observed for the polypeptides gB-SLP12-Delta113 and gB-SLP12-Delta725 in the Western blots presented in FIG. 14C and 14D.

### Conclusion

The data presented in FIG. 14 show that the clipping occurring with the polypeptides gB-DeltaTM and gB-SLP12 in the C-terminal part of the polypeptide does not occur when at least part of the cytoplasmic domain is deleted.

### Example 5: N-terminal heterogeneity of gB-SLP12-Delta725 of polypeptides

As shown in FIG. 12A, the CMV gB-SLP12-Delta725 polypeptide, when recombinantly expressed in cells results in a population of mature polypeptides which are heterogeneous at the the N-terminal end. Indeed, the signal peptidase happens to cleave off the signal sequence at different amino acid positions, as indicated. This phenomenon is also referred to as signal peptidase "woobling".

### 5.1 Expression and purification

The His-tag-containing gB-SLP12-Delta725, gB-SLP12-Delta725-LVL759, gB-SLP12-Delta725-LVL776 and gB-SLP12-Delta725-CD33 polypeptides, as described in Example 1, were recombinantly expressed as described in Example 2. On day 6 post-transfection, the culture supernatants were collected. An EDTA-free protease inhibitor cocktail (Roche, No. 11873580001) was added to the supernatants, and the NaCl concentration was adjusted to 500 mM, and the final pH to 8.3. IMAC sepharose 6 FF (GE Healthcare) was packed into 5 cm diameter glass columns (Waters). The columns were equilibrated with 5 column volumes of binding buffer (10 mM Tris, 500 mM NaCl, 10 mM Imidazole, pH 8.3). The supernatants were loaded on the equilibrated columns at a flow rate of 10 ml/min. Unbound material was washed with two column volumes of the above binding buffer. Fractions of 50 ml were harvested. Columns were further washed with an additional two column volumes of the above binding buffer. Fractions of 10 ml were harvested. The proteins bound to the columns were eluted with 6 column volumes of elution buffer (Tris, 500 mM NaCl, 350 mM, Imidazole, pH 8.30). Fractions of 10 ml were harvested. The fractions were loaded on a SDS-PAGE gel and positive fractions were pooled and concentrated with Centricon Ultrafiltration device (Millipore). The concentrated protein was quantified by RCDC modified Lowry colorimetric assay (Biorad). 5 to 10 µg of the purified protein was deglycosylated using the N-Glycosidase F deglycosylation kit (Roche, No. 11836552001) following the manufacturer's instructions. The deglycosylated protein was precipitated by RCDC precipitation reactant (BioRad), resuspended in SDS-PAGE reducing loading buffer and loaded on SDS-PAGE. The protein band migrating at the position corresponding to the deglycosylated protein was cut for tryptic digestion and MS/MS peptide mapping analysis.

### 5.2 Trypsin digestion

The gel pieces containing the purified protein were dehydrated with acetonitrile (ACN). Proteins were reduced by adding the reduction buffer (10 mM DTT, 100 mM ammonium bicarbonate) for 30 min at 40°C, and alkylated by adding the alkylation buffer (55 mM iodoacetamide, 100 mM ammonium bicarbonate) for 20 min at 40°C. Gel pieces were then further dehydrated and washed at 40 °C by adding ACN for 5 min before discarding all the reagents. Gel pieces were then dried for 5 min at 40 °C and then re-hydrated at 4°C for 40 min with the trypsin solution (6 ng/µl of trypsin sequencing grade from Promega, 25 mM ammonium bicarbonate). Protein digestion was performed at 58 °Cfor 1h and stopped with 15 µl of of a mixture of 1% formic acid and 2% ACN. Supernatant was transferred into a 96-well plate and the extraction of the trypsin-digested peptides was performed with two 30-min extraction steps at room temperature using the extraction buffer (1% formic acid and 50% ACN). All extracted peptides were pooled into the 96-well plate and then completely dried in vacuum centrifuge. The plate was sealed and stored at -20°C until further processed to the LC-MS/MS analysis.

### 5.3 Mass spectrometry

Prior to LC-MS/MS analysis, extracted peptides were re-solubilized under agitation for 15 min in 12 µl of 0.2% formic acid and then centrifuged at 2000 rpm for 1 min. The LC column is a C18 reversed phase column packed with a high-pressure packing cell. A 75 lm i.d. fused silica capillary of 100 mm long was packed with the C18 Jupiter 5 lm300 Å reverse-phase material (Phenomenex). This column was installed on the nano LC-2D system (Eksigent) and coupled to the LTQ Orbitrap (ThermoFisher Scientific). The buffers used for chromatography were 0.2% formic acid (buffer A) and 100% ACN/0.2% formic acid (buffer B). During the first 12 min, 5 µl of sample are loaded on column with a flow of 650nl/min and, subsequently, the gradient goes from 2-80% buffer B in 20 min and then come back to 2% buffer B for 10 min. LC-MS/MS data acquisition was accomplished using a four scan event cycle comprised of a full scan MS for scan event 1 acquired in the Orbitrap.

### Results

- FIG. 12 shows the result of the quantitative evaluation of the signal peptidase "woobling" occurring at the cleavage site of the signal sequence of the different polypeptides tested. Woobling of the signal peptidase leads to the generation of various mature polypeptide forms starting with a different amino acid at their N-terminal end within each population of the indicated polypeptide.
- FIG. 12A shows that, after tryptic digestion of the total protein extract, followed by the relative quantification of the resulting peptides comprising the N-terminal end by MS/MS, it was observed that the population of gB-SLP12-Delta725 polypeptide, after cleavage of the signal sequence, was mainly present in solution as five different polypeptide forms in a relatively equivalent proportion (ranging from about 10% to about 30%), each one starting with a different amino acid, as indicated.
- FIG. 12B and 12C show a similar analysis of the MS/MS relative quantification of the different forms of the polypeptides present in the population of gB-SLP12-Delta725-LVL759 and gB-SLP12-Delta725-LVL776, respectively. It was observed that the population of those two polypeptides, after cleavage of the signal sequence, was mainly present in solution as one major form of polypeptide in a percentage higher than 99%, starting with the amino acid at position 23 of the sequence set forth in SEQ ID NO: 14, and with the amino acid at position 24 of the sequence set forth in SEQ ID NO:16, respectively. The two alternative cleavage sites cleaved by the signal peptidase and generating two additional polypeptide forms having a distinct amino acid at the N-terminal end represented as little as less than 1% within the respective population of polypeptides.
- FIG. 12D show a similar analysis of the MS/MS relative quantification of the different forms of the polypeptides present in the population of gB-SLP12-Delta725-CD33 polypeptide. It was observed that this population, after cleavage of the signal sequence, was mainly present in solution as one major form of polypeptide in a percentage higher than 99%, starting with the amino acid at position 18 of the sequence set forth in SEQ ID NO: 18. The other cleavage site cleaved by the signal peptidase and generating one additional polypeptide form having a distinct amino acid at the N-terminal end represented as little as less than 1% within the population of gB-SLP12-Delta725-CD33 polypeptide.

### Conclusion

Those results indicate that the woobling no longer occurred in gB-SLP12-Delta725-LVL759, gB-SLP12-Delta725-LVL776, and gB-SLP12-Delta725-CD33 polypeptides, as the signal peptidase consistently recognized one major cleavage site only. This suggests that modifications made to the signal sequence and to the leader sequence of the gB polypeptide has a significant positive impact on the heterogeneity of the mature polypeptide population, rendering said populations homogeneous to an extent greater than 99%.

### Example 6: Immunogenicity of immunogenic compositions containing exemplary CMV gB polypeptides of the invention

The immunogenicity of the immunogenic compositions containing the following polypeptides which comprise a C-terminal His tag and which were expressed and purified as described in section, gB-DeltaTM, gB-SLP12, gB-SLP12-Delta113, gB-SLP12-Delta725, LVL759, LVL776 and CD33, was tested as follows.

### 6.1 Experiment design

Groups of ten C57BL/6 mice have been vaccinated with two doses at 21 days of interval with the above exemplary immunogenic compositions, as indicated in Table 1. Immunizations have been done intramuscularly (1.5 µg of each gB polypeptide adjuvanted with the adjuvant system ASA comprising 2.5 µg of 3D-MPL and 2.5 µg of QS21 in a liposomal formulation in a total volume of 50 µl).

**Table 1 - Study groups**

| *Groups*/*Polypeptides* | *Concentration (µg*/*ml)* | *Arginine (mM)* |
|---|---|---|
| 1. gB-DeltaTM* | 442 µg/ml | 346 |
| 2. gB-SLP12* | 987 µg/ml | 600 |
| 3. gB-SLP12-Delta113* | 304 µg/ml | 321 |
| 4. gB-SLP12-Delta725* | 718 µg/ml | 366 |
| 5. CD33* | 256 µg/ml | 370 |
| 6. LVL759** | 460 µg/ml | 348 |
| 7. LVL776** | 460 µg/ml | 348 |

| | | |
|---|---|---|
| * Polypeptide buffer: 10 mM Phosphate K/K2 Buffer - 500 mM NaCl - pH8 + Arginine ** Polypeptide buffer: 10 mM Tris- 500 mM NaCl - pH8.3 + Arginine | | |

Immunogenicity was evaluated by analyzing the humoral immune responses to vaccination, as measured by neutralization (NT) assay and ELISA (Enzyme-Linked immuno Sorbent Assay) 21 days post-immunization 1 and 14 days post-immunization 2 (see section 6.3 and 6.4, respectively).

### 6.2 Statistical analysis

Groups of 10 C57BL/6 mice have been designed in order to obtain 90% of power with a 2-fold difference for the primary read-out. Statistical analysis was performed on days 14 post-immunization 2 data using UNISTAT. The protocol applied for analysis of variance can be briefly described as follows: Log transformation of data; Shapiro-Wilk test on each population (group) in order to verify the normality; Cochran test in order to verify the homogeneity of variance between different populations (group): Analysis of variance on selected data (ANOVA 1 or 2); Tuckey-HSD test for multiple comparisons.

### 6.3 Neutralization assay

Prior to the assay, MRC5 cells were seeded into 96-well plates and incubated for 3 days at 37°C. Serial dilutions of heat-inactivated sera were incubated with a fixed amount of human CMV AD169 virus for 1h at 37°C. After incubation, the mixture of sera and virus was added into 96-well plates containing MRC5 cells. The plates were centrifuged at 2000 rpm for 1h at 37°C. After an overnight incubation at 37°C, the plates were fixed with a solution of acetone 80% (20-30 min at +4°C). The acetone solution was removed and the CMV-positive wells were detected using a specific monoclonal biotin-conjugated anti-immediate early I (IE-I) antibody for 1h at 37°C. The plates were washed 3 times with PBS. After washing, streptavidin-horseradish peroxidase was added for an additional 30 min at 37°C. Plates were washed 3 times and incubated for 10 min with a solution of True-Blue. Specific colored signals were recorded by visual examination under microscope. Neutralizing titers were expressed as the reciprocal of the highest dilution of serum giving 50% reduction in the number of CMV-positive wells when compared to the virus dose control without serum.

### Results

The neutralizing antibody response was measured on sera samples collected 21 days after the first immunization and 14 days after the second immunization. Samples were tested for their neutralizing activity against human CMV AD169 virus. Results are shown in FIG. 15A. All tested polypeptides induced a significant response 14 days post-immunization 2 (see the grey bars). gB-SLP12, gB-SLP12-Delta113, gB SLP12-Delta725 and LVL759 induced titres that were non-significantly* different from gB-DeltaTM. CD33 (p-value: 0.0006) and LVL776 (p-value: 0.0116) induced titers significantly lower than gB-DeltaTM.
* p-value ≥ 0.05 → No statistical difference (with 95% of confidence)
** p-value < 0.05 → Statistical difference

### 6.4 ELISA assay

Quantification of anti-gB antibodies was performed by ELISA using a gB polypeptide called gB** (described in EP0759995) as a coating antigen. The antigen was diluted at a final concentration of 4 µg/ml in PBS (100 µl/well) and incubated overnight at 4°C in 96-well plates. The plates were then saturated for 1h at 37°C with 200 µl of PBS containing 1% Bovine Serum Albumin. Two-fold serial dilutions of sera were added into wells (100 µl/well) and incubated 1h 30 min at 37°C. The plates were washed 4 times with PBS/Tween 20 0.1%. 100 µl of biotin-conjugated anti-mouse Ig was added to each well and incubated for 1h at 37°C. After 4 washes with PBS/Tween 20 0.1%, 100 µl of streptavidin-horseradish peroxidase was added for an additional 30 min incubation at 37°C. Plates were washed 4 times with PBS/Tween 20 0.1% and once with water. Then, the plates were incubated for 10 min at 22°C with 100 µl of Tetra-methyl-benzidine 75% in 0.1 M citrate buffer pH 5.8. This reaction was stopped with 100 µl of H₂SO₄ 0.4 N and the colorimetry was read in a spectrophotometer at 450/620 nm. A previous lot of purified gB-DeltaTM whose concentration was known was used as a standard in this experiment. Using the software SoftMax Pro, ELISA titers were determined and they are expressed in ELISA Unit/ml.

### Results

The ELISA titre was measured on sera samples collected 21 days after the first immunization and 14 days after the second immunization. Results are shown in FIG. 15B. All tested polypeptides induced a significant response 14 days post-immunization 2 (see the grey bars). gB-SLP12-Delta113 and gB-SLP12-Delta725 induced titers that were non-significantly* different from the titer induced by gB-DeltaTM. gB-SLP12, CD33, LVL759 and LVL776 induced titers that were non-significantly* different. gB-DeltaTM induced significantly higher titres** than gB-SLP12 (p-value: 0.0002), CD33 (p-value: 0.0226), LVL759 (p-value: 0.0002) and LVL776 (p-value: 0.0001).
* p-value ≥ 0.05 → No statistical difference (with 95% of confidence)
** p-value < 0.05 → Statistical difference

## Claims

1. A cytomegalovirus (CMV) gB polypeptide comprising a non-functional transmembrane (TM) domain, wherein at least 50%, at least 70%, at least 80%, or at least 90% of the amino acids of the TM domain is deleted, and at least a portion of a gB protein extracellular domain comprising a fusion loop 1 (FL1) domain defined by amino acids Y.I.Y located at position 155-157 of the sequence set forth in SEQ ID NO:1, or at a corresponding position in other CMV gB polypeptides, and a fusion loop 2 (FL2) domain defined by amino acids W.L.Y located at position 240-242 of the sequence set forth in SEQ ID NO:1, or at a corresponding position in other CMV gB polypeptides, wherein at least the FL1 domain comprises the substitution of at least one amino acid selected from Y.I.Y located at position 155-157 of the sequence set forth in SEQ ID NO:1, or at a corresponding position in other CMV gB polypeptides, with a polar amino acid other than an aromatic amino acid.

2. The CMV gB polypeptide of claim 1, wherein the TM domain is made non-functional by deleting amino acid amino acids 701 to 775 of the sequence set forth in SEQ ID NO:1, or at a corresponding position in other CMV gB polypeptides.

3. The CMV gB polypeptide of claim 1 or claim 2, comprising a deletion of at least 80%, at least 90%, at least 95% of the amino acids of the cytoplasmic domain, or a deletion of the entire cytoplasmic domain.

4. The CMV gB polypeptide of claims 1 to 3, wherein the polar amino acid is selected from the group of positively charged amino acids consisting of lysine (K), histidine (H) and arginine (R).

5. The CMV gB polypeptide of any of claims 1 to 4, wherein the amino acids Y.I.Y located at position 155-157 in the fusion loop FL1 domain of the sequence set forth in SEQ ID NO:1, or at a corresponding position in other CMV gB polypeptides, are substituted with amino acids G.H.R, respectively.

6. The CMV gB polypeptide of any one of claims 1 to 5, wherein the amino acids Y.I.Y located at position 155-157 in the fusion loop FL1 domain of the sequence set forth in SEQ ID NO:1, or at a corresponding position in other CMV gB polypeptides, are substituted with a stretch of amino acids having a hydrophobicity score of less than -3, less than - 7, less than -8, as measured by the Kyte and Doolittle scale.

7. The CMV gB polypeptide of any one of claims 1 to 6, wherein the fusion loop FL2 domain comprises the substitution of at least one amino acid selected from W.L.Y located at position 240-242 of the sequence set forth in SEQ ID NO:1, or at a corresponding position in other CMV gB polypeptides, with a positively charged amino acid selected from the group consisting of lysine (K), histidine (H) and arginine (R).

8. The CMV gB polypeptide of any one of claims 1 to 7, wherein the amino acids W.L.Y located at position 240-242 in the fusion loop FL2 domain of the sequence set forth in SEQ ID NO:1, or at a corresponding position in other CMV gB polypeptides, are substituted with amino acid A.F.H, respectively.

9. The CMV gB polypeptide of any one of claims of 1 to 8, comprising a deletion of at least 40%, at least 50%, at least 70%, at least 80%, at least 90% of the amino acids of the leader sequence, or of the entire leader sequence.

10. A CMV gB polypeptide having the sequence selected from the group consisting of the sequences set forth in: SEQ ID NO:3, SEQ ID NO:4, SEQ ID NO:5, SEQ ID NO:10, SEQ ID NO:12, SEQ ID NO:14, SEQ ID NO:16, SEQ ID NO:18, SEQ ID NO:19, SEQ ID NO:20 and SEQ ID NO:21.

11. An immunogenic composition comprising the CMV gB polypeptide of any of one of claims 1 to 9 admixed with a suitable pharmaceutical carrier.

12. The immunogenic composition of claim 11, further comprising an adjuvant comprising 3D-MPL and QS21 in a liposomal formulation.

13. A polynucleotide that encodes the CMV gB polypeptide of any one of claims 1 to 9.

14. A polynucleotide having the sequence selected from the group consisting of the sequences set forth in: SEQ ID NO:6, SEQ ID NO:7, SEQ ID NO:8, SEQ ID NO:9, SEQ ID NO:11, SEQ ID NO:13, SEQ ID NO:15, and SEQ ID NO:17.

15. A recombinant vector comprising the polynucleotide of claim 13 or claim 14.

16. A host cell transformed with the recombinant vector of claim 15.

17. The CMV gB polypeptide of any of claims 1 to 9 or the composition of any of claims 11 and 12, for use in the prevention and/or treatment of a CMV infection.

18. The CMV gB polypeptide of claim 17 for use in the prevention of congenital CMV infection in a newborn.

## Patentansprüche

1. Cytomegalovirus (CMV)-gB-Polypeptid, umfassend eine nicht-funktionale Transmembran- (TM-) Domäne, wobei wenigstens 50%, wenigstens 70%, wenigstens 80% oder wenigstens 90% der Aminosäuren der TM-Domäne deletiert sind, und wenigstens ein Teil einer gB-Proteinextrazellulären Domäne eine Fusionsschleife 1- (FL1-) Domäne, die definiert ist durch die Aminosäuren Y.I.Y, die an Position 155-157 der in SEQ ID NO: 1 ausgeführten Sequenz oder an einer entsprechenden Position in anderen CMV-gB-Polypeptiden liegen, und eine Fusionsschleife 2-(FL2-) Domäne, die definiert ist durch die Aminosäuren W.L.Y, die an Position 240-242 der in SEQ ID NO: 1 ausgeführten Sequenz oder an einer entsprechenden Position in anderen CMV-gB-Polypeptiden liegen, umfasst, wobei wenigstens die FL1-Domäne die Substitution wenigstens einer Aminosäure, ausgewählt aus Y.I.Y, die an Position 155-157 der in SEQ ID NO: 1 ausgeführten Sequenz oder an einer entsprechenden Position in anderen CMV-gB-Polypeptiden liegen, mit einer polaren Aminosäure, die keine aromatische Aminosäure ist, umfasst.

2. CMV-gB-Polypeptid gemäß Anspruch 1, wobei die TM-Domäne durch Deletieren der Aminosäure Aminosäuren 701 bis 775 der in SEQ ID NO: 1 ausgeführten Sequenz oder einer entsprechenden Position in anderen CMV-gB-Polypeptiden nicht-funktional gemacht wird.

3. CMV-gB-Polypeptid gemäß Anspruch 1 oder Anspruch 2, umfassend eine Deletion von wenigstens 80%, wenigstens 90%, wenigstens 95% der Aminosäuren der zytoplasmatischen Domäne, oder eine Deletion der gesamten zytoplasmatischen Domäne.

4. CMV-gB-Polypeptid gemäß den Ansprüchen 1 bis 3, wobei die polare Aminosäure aus der Gruppe von positiv geladenen Aminosäuren, bestehend aus Lysin (K), Histidin (H) und Arginin (R), ausgewählt ist.

5. CMV-gB-Polypeptid gemäß irgendeinem der Ansprüche 1 bis 4, wobei die Aminosäuren Y.I.Y, die an Position 155-157 in der Fusionsschleife-FL1-Domäne der in SEQ ID NO: 1 ausgeführten Sequenz oder an einer entsprechenden Position in anderen CMV-gB-Polypeptiden liegen, entsprechend mit den Aminosäuren G.H.R substituiert sind.

6. CMV-gB-Polypeptid gemäß irgendeinem der Ansprüche 1 bis 5, wobei die Aminosäuren Y.I.Y, die an Position 155-157 in der Fusionsschleife-FL1-Domäne der in SEQ ID NO: 1 ausgeführten Sequenz oder an einer entsprechenden Position in anderen CMV-gB-Polypeptiden liegen, mit einem Abschnitt von Aminosäuren substituiert sind, die einen Hydrophobizitätsscore von weniger als -3, weniger als -7, weniger als -8 haben, gemessen durch die Kyte-und-Doolittle-Skala.

7. CMV-gB-Polypeptid gemäß irgendeinem der Ansprüche 1 bis 6, wobei die Fusionsschleife-FL2-Domäne die Substitution wenigstens einer Aminosäure, ausgewählt aus W.L.Y, die an Position 240-242 der in SEQ ID NO: 1 ausgeführten Sequenz oder an einer entsprechenden Position in anderen CMV-gB-Polypeptiden liegen, mit einer positiv geladenen Aminosäure, ausgewählt aus der Gruppe, bestehend aus Lysin (K), Histidin (H) und Arginin (R), umfasst.

8. CMV-gB-Polypeptid gemäß irgendeinem der Ansprüche 1 bis 7, wobei die Aminosäuren W.L.Y, die an Position 240-242 in der Fusionsschleife-FL2-Domäne der in SEQ ID NO: 1 ausgeführten Sequenz oder an einer entsprechenden Position in anderen CMV-gB-Polypeptiden liegen, entsprechend mit der Aminosäure A.F.H substituiert sind.

9. CMV-gB-Polypeptid gemäß irgendeinem der Ansprüche 1 bis 8, umfassend eine Deletion von wenigstens 40%, wenigstens 50%, wenigstens 70%, wenigstens 80%, wenigstens 90% der Aminosäuren der Leadersequenz oder der kompletten Leadersequenz.

10. CMV-gB-Polypeptid, das die Sequenz hat, ausgewählt aus der Gruppe, bestehend aus den Sequenzen, die ausgeführt sind in: SEQ ID NO: 3, SEQ ID NO: 4, SEQ ID NO: 5, SEQ ID NO: 10, SEQ ID NO: 12, SEQ ID NO: 14, SEQ ID NO: 16, SEQ ID NO: 18, SEQ ID NO: 19, SEQ ID NO: 20 und SEQ ID NO: 21.

11. Immunogene Zusammensetzung, die das CMV-gB-Polypeptid gemäß irgendeinem der Ansprüche 1 bis 9 im Gemisch mit einem geeigneten pharmazeutischen Träger umfasst.

12. Immunogene Zusammensetzung gemäß Anspruch 11, die weiterhin ein Adjuvans, umfassend 3D-MPL und QS21 in einer liposomalen Formulierung, umfasst.

13. Polynukleotid, welches das CMV-gB-Polypeptid gemäß irgendeinem der Ansprüche 1 bis 9 codiert.

14. Polynukleotid, das die Sequenz hat, ausgewählt aus der Gruppe, bestehend aus den Sequenzen, die ausgeführt sind in: SEQ ID NO: 6, SEQ ID NO: 7, SEQ ID NO: 8, SEQ ID NO: 9, SEQ ID NO: 11, SEQ ID NO: 13, SEQ ID NO: 15 und SEQ ID NO: 17.

15. Rekombinanter Vektor, welcher das Polynukleotid gemäß Anspruch 13 oder Anspruch 14 umfasst.

16. Wirtszelle, die mit dem rekombinanten Vektor gemäß Anspruch 15 transformiert ist.

17. CMV-gB-Polypeptid gemäß irgendeinem der Ansprüche 1 bis 9 oder Zusammensetzung gemäß irgendeinem der Ansprüche 11 und 12 zur Verwendung bei der Prävention und/oder Behandlung einer CMV-Infektion.

18. CMV-gB-Polypeptid gemäß Anspruch 17 zur Verwendung bei der Prävention von kongenitaler CMV-Infektion in einem Neugeborenen.

## Revendications

1. Polypeptide gB du cytomégalovirus (CMV) comprenant un domaine transmembranaire (TM) non fonctionnel, dans lequel au moins 50 %, au moins 70 %, au moins 80 %, ou au moins 90 % des acides aminés du domaine TM sont délétés, et au moins une partie du domaine extracellulaire de la protéine gB comprenant un domaine de boucle de fusion 1 (FL1) défini par les acides aminés Y.I.Y localisés au niveau des positions 155 à 157 de la séquence représentée par SEQ ID NO : 1, ou au niveau d'une position correspondante dans d'autres polypeptides gB du CMV, et un domaine de boucle de fusion 2 (FL2) défini par les acides aminés W.L.Y localisés au niveau des positions 240 à 242 de la séquence représentée par SEQ ID NO : 1, ou au niveau d'une position correspondante dans d'autres polypeptides gB du cytomégalovirus, dans lequel au moins le domaine FL1 comprend la substitution d'au moins un acide aminé choisi parmi Y.I.Y localisés au niveau des positions 155 à 157 de la séquence représentée par SEQ ID NO : 1, ou au niveau d'une position correspondante dans d'autres polypeptides gB du CMV, par un acide aminé polaire autre qu'un acide aminé aromatique.

2. Polypeptide gB du CMV selon la revendication 1, dans lequel le domaine TM est rendu non fonctionnel par délétion des acide aminés acides aminés 701 à 775 de la séquence représentée par SEQ ID NO : 1, ou au niveau d'une position correspondante dans d'autres polypeptides gB du CMV.

3. Polypeptide gB du CMV selon la revendication 1 ou la revendication 2, comprenant une délétion d'au moins 80 %, d'au moins 90 %, d'au moins 95 % des acides aminés du domaine cytoplasmique, ou une délétion du domaine cytoplasmique entier.

4. Polypeptide gB du CMV selon les revendications 1 à 3, dans lequel l'acide aminé polaire est choisi dans le groupe des acides aminés chargés positivement constitué de la lysine (K), l'histidine (H) et l'arginine (R).

5. Polypeptide gB du CMV selon l'une quelconque des revendications 1 à 4, dans lequel les acides aminés Y.I.Y localisés au niveau des positions 155 à 157 dans le domaine de boucle de fusion FL1 de la séquence représentée par SEQ ID NO : 1, ou au niveau d'une position correspondante dans d'autres polypeptides gB du CMV, sont substitués par les acides aminés G.H.R, respectivement.

6. Polypeptide gB du CMV selon l'une quelconque des revendications 1 à 5, dans lequel les acides aminés Y.I.Y localisés au niveau des positions 155 à 157 dans le domaine de boucle de fusion FL1 de la séquence représentée par SEQ ID NO : 1, ou au niveau d'une position correspondante dans d'autres polypeptides gB du CMV, sont substitués par une séquence d'acides aminés présentant un score d'hydrophobicité inférieur à -3, inférieur à -7, inférieur à -8, mesuré par l'échelle de Kyte et Doolittle.

7. Polypeptide gB du CMV selon l'une quelconque des revendications 1 à 6, dans lequel le domaine de boucle de fusion FL2 comprend la substitution d'au moins un acide aminé choisi parmi W.L.Y localisés au niveau des positions 240 à 242 de la séquence représentée par SEQ ID NO : 1, ou au niveau d'une position correspondante dans d'autres polypeptides gB du CMV, par un acide aminé chargé positivement choisi dans le groupe constitué de la lysine (K), l'histidine (H) et l'arginine (R).

8. Polypeptide gB du CMV selon l'une quelconque des revendications 1 à 7, dans lequel les acides aminés W.L.Y localisés au niveau des positions 240 à 242 dans le domaine de boucle de fusion FL2 de la séquence représentée par SEQ ID NO : 1, ou au niveau d'une position correspondante dans d'autres polypeptides gB du CMV, sont substitués par les acides aminés A.F.H, respectivement.

9. Polypeptide gB du CMV selon l'une quelconque des revendications 1 à 8, comprenant une délétion d'au moins 40 %, d'au moins 50 %, d'au moins 70 %, d'au moins 80 %, d'au moins 90 % des acides aminés de la séquence de tête, ou de la séquence de tête entière.

10. Polypeptide gB du CMV ayant la séquence choisie dans le groupe constitué des séquences représentées par : SEQ ID NO : 3, SEQ ID NO : 4, SEQ ID NO : 5, SEQ ID NO : 10, SEQ ID NO : 12, SEQ ID NO : 14, SEQ ID NO : 16, SEQ ID NO : 18, SEQ ID NO : 19, SEQ ID NO : 20 et SEQ ID NO : 21.

11. Composition immunogène comprenant le polypeptide gB du CMV selon l'une quelconque des revendications 1 à 9 mélangé avec un support pharmaceutique approprié.

12. Composition immunogène selon la revendication 11, comprenant en outre un adjuvant comprenant du 3D-MPL et du QS21 dans une formulation liposomique.

13. Polynucléotide qui code pour le polypeptide gB du CMV selon l'une quelconque des revendications 1 à 9.

14. Polynucléotide ayant la séquence choisie dans le groupe constitué des séquences représentées par : SEQ ID NO : 6, SEQ ID NO : 7, SEQ ID NO : 8, SEQ ID NO : 9, SEQ ID NO : 11, SEQ ID NO : 13, SEQ ID NO : 15, et SEQ ID NO : 17.

15. Vecteur recombinant comprenant le polynucléotide selon la revendication 13 ou la revendication 14.

16. Cellule hôte transformée par le vecteur recombinant selon la revendication 15.

17. Polypeptide gB du CMV selon l'une quelconque des revendications 1 à 9 ou la composition selon l'une quelconque des revendications 11 et 12, pour une utilisation dans la prévention et/ou le traitement d'une infection par le CMV.

18. Polypeptide gB du CMV selon la revendication 17 pour une utilisation dans la prévention d'une infection congénitale par le CMV chez un nouveau-né.


### SEQUENCE LISTING

<110> Blais, Normand
   Baudoux, Guy
   Marchand, Martine
<120> Novel Antigen
<130> VB63857
<160> 38
<170> FastSEQ for Windows Version 4.0
<210> 1
   <211> 906
   <212> PRT
   <213> AD169 Cytomegalovirus
<220>
   <223> AD169 Cytomegalovirus
<400> 1
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Arg Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Tyr Ile Tyr Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Trp
   225                 230                 235                 240
   Leu Tyr Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Arg Ser Glu Ala Glu Asp Ser Tyr His Phe Ser Ser
           355                 360                 365
   Ala Lys Met Thr Ala Thr Phe Leu Ser Lys Lys Gln Glu Val Asn Met
       370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Arg Thr Arg Arg Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
       610                 615                 620
   Tyr Glu Tyr Val Asp Tyr Leu Phe Lys Arg Met Ile Asp Leu Ser Ser
   625                 630                 635                 640
   Ile Ser Thr Val Asp Ser Met Ile Ala Leu Asp Ile Asp Pro Leu Glu
                   645                 650                 655
   Asn Thr Asp Phe Arg Val Leu Glu Leu Tyr Ser Gln Lys Glu Leu Arg
               660                 665                 670
   Ser Ser Asn Val Phe Asp Leu Glu Glu Ile Met Arg Glu Phe Asn Ser
           675                 680                 685
   Tyr Lys Gln Arg Val Lys Tyr Val Glu Asp Lys Val Val Asp Pro Leu
       690                 695                 700
   Pro Pro Tyr Leu Lys Gly Leu Asp Asp Leu Met Ser Gly Leu Gly Ala
   705                 710                 715                 720
   Ala Gly Lys Ala Val Gly Val Ala Ile Gly Ala Val Gly Gly Ala Val
                   725                 730                 735
   Ala Ser Val Val Glu Gly Val Ala Thr Phe Leu Lys Asn Pro Phe Gly
               740                 745                 750
   Ala Phe Thr Ile Ile Leu Val Ala Ile Ala Val Val Ile Ile Thr Tyr
                       755                 760                 765
   Leu Ile Tyr Thr Arg Gln Arg Arg Leu Cys Thr Gln Pro Leu Gln Asn
           770                 775                 780
   Leu Phe Pro Tyr Leu Val Ser Ala Asp Gly Thr Thr Val Thr Ser Gly
   785                 790                 795                 800
   Ser Thr Lys Asp Thr Ser Leu Gln Ala Pro Pro Ser Tyr Glu Glu Ser
                   805                 810                 815
   Val Tyr Asn Ser Gly Arg Lys Gly Pro Gly Pro Pro Ser Ser Asp Ala
               820                 825                 830
   Ser Thr Ala Ala Pro Pro Tyr Thr Asn Glu Gln Ala Tyr Gln Met Leu
           835                 840                 845
   Leu Ala Leu Ala Arg Leu Asp Ala Glu Gln Arg Ala Gln Gln Asn Gly
       850                 855                 860
   Thr Asp Ser Leu Asp Gly Gln Thr Gly Thr Gln Asp Lys Gly Gln Lys
   865                 870                 875                 880
   Pro Asn Leu Leu Asp Arg Leu Arg His Arg Lys Asn Gly Tyr Arg His
                   885                 890                 895
   Leu Lys Asp Ser Asp Glu Glu Glu Asn Val
               900                 905
<210> 2
   <211> 830
   <212> PRT
   <213> Artificial Sequence
<220>
   <223> AD169 Cytomegalovirus
<400> 2
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Arg Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Tyr Ile Tyr Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Trp
   225                 230                 235                 240
   Leu Tyr Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Arg Ser Glu Ala Glu Asp Ser Tyr His Phe Ser Ser
           355                 360                 365
   Ala Lys Met Thr Ala Thr Phe Leu Ser Lys Lys Gln Glu Val Asn Met
       370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Thr Thr Gln Thr Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
       610                 615                 620
   Tyr Glu Tyr Val Asp Tyr Leu Phe Lys Arg Met Ile Asp Leu Ser Ser
   625                 630                 635                 640
   Ile Ser Thr Val Asp Ser Met Ile Ala Leu Asp Ile Asp Pro Leu Glu
                   645                 650                 655
   Asn Thr Asp Phe Arg Val Leu Glu Leu Tyr Ser Gln Lys Glu Leu Arg
               660                 665                 670
   Ser Ser Asn Val Phe Asp Leu Glu Glu Ile Met Arg Glu Phe Asn Ser
           675                 680                 685
   Tyr Lys Gln Arg Val Lys Tyr Val Glu Asp Lys Arg Leu Cys Thr Gln
       690                 695                 700
   Pro Leu Gln Asn Leu Phe Pro Tyr Leu Val Ser Ala Asp Gly Thr Thr
   705                 710                 715                 720
   Val Thr Ser Gly Ser Thr Lys Asp Thr Ser Leu Gln Ala Pro Pro Ser
                   725                 730                 735
   Tyr Glu Glu Ser Val Tyr Asn Ser Gly Arg Lys Gly Pro Gly Pro Pro
               740                 745                 750
   Ser Ser Asp Ala Ser Thr Ala Ala Pro Pro Tyr Thr Asn Glu Gln Ala
           755                 760                 765
   Tyr Gln Met Leu Leu Ala Leu Ala Arg Leu Asp Ala Glu Gln Arg Ala
       770                 775                 780
   Gln Gln Asn Gly Thr Asp Ser Leu Asp Gly Gln Thr Gly Thr Gln Asp
   785                 790                 795                 800
   Lys Gly Gln Lys Pro Asn Leu Leu Asp Arg Leu Arg His Arg Lys Asn
                   805                 810                 815
   Gly Tyr Arg His Leu Lys Asp Ser Asp Glu Glu Glu Asn Val
               820                 825                 830
<210> 3
   <211> 830
   <212> PRT
   <213> Artificial Sequence
<220>
   <223> AD169 Cytomegalovirus
<400> 3
   Met Glu Ser Arg Ile Trp Cys Leu Val Val Cys Val Asn Leu Cys Ile
   1 5 10 15
   Val Cys Leu Gly Ala Ala Val Ser Ser Ser Ser Thr Ser His Ala Thr
   20 25 30
   Ser Ser Thr His Asn Gly Ser His Thr Ser Arg Thr Thr Ser Ala Gln
   35 40 45
   Thr Ser Ser Val Tyr Ser Gln His Val Thr Ser Ser Glu Ala Val Ser
   50 55 60
   His Arg Ala Asn Glu Thr Ile Tyr Asn Thr Thr Leu Lys Tyr Gly Asp
   65 70 75 80
   Val Val Gly Val Asn Thr Thr Lys Tyr Pro Tyr Arg Val Cys Ser Met
   85 90 95
   Ala Gln Gly Thr Asp Leu Ile Arg Phe Glu Arg Asn Ile Ile Cys Thr
   100 105 110
   Ser Met Lys Pro Ile Asn Glu Asp Leu Asp Glu Gly Ile Met Val Val
   115 120 125
   Tyr Lys Arg Asn Ile Val Ala His Thr Phe Lys Val Arg Val Tyr Gln
   130 135 140
   Lys Val Leu Thr Phe Arg Arg Ser Tyr Ala Gly His Arg Thr Thr Tyr
   145 150 155 160
   Leu Leu Gly Ser Asn Thr Glu Tyr Val Ala Pro Pro Met Trp Glu Ile
   165 170 175
   His His Ile Asn Lys Phe Ala Gln Cys Tyr Ser Ser Tyr Ser Arg Val
   180 185 190
   Ile Gly Gly Thr Val Phe Val Ala Tyr His Arg Asp Ser Tyr Glu Asn
   195 200 205
   Lys Thr Met Gln Leu Ile Pro Asp Asp Tyr Ser Asn Thr His Ser Thr
   210 215 220
   Arg Tyr Val Thr Val Lys Asp Gln Trp His Ser Arg Gly Ser Thr Ala
   225 230 235 240
   Phe His Arg Glu Thr Cys Asn Leu Asn Cys Met Leu Thr Ile Thr Thr
   245 250 255
   Ala Arg Ser Lys Tyr Pro Tyr His Phe Phe Ala Thr Ser Thr Gly Asp
   260 265 270
   Val Val Tyr Ile Ser Pro Phe Tyr Asn Gly Thr Asn Arg Asn Ala Ser
   275 280 285
   Tyr Phe Gly Glu Asn Ala Asp Lys Phe Phe Ile Phe Pro Asn Tyr Thr
   290 295 300
   Ile Val Ser Asp Phe Gly Arg Pro Asn Ala Ala Pro Glu Thr His Arg
   305 310 315 320
   Leu Val Ala Phe Leu Glu Arg Ala Asp Ser Val Ile Ser Trp Asp Ile
   325 330 335
   Gln Asp Glu Lys Asn Val Thr Cys Gln Leu Thr Phe Trp Glu Ala Ser
   340 345 350
   Glu Arg Thr Ile Ser Ser Glu Ala Glu Asp Ser Tyr His Phe Ser Ser
   355 360 365
   Ala Lys Met Thr Ala Thr Phe Leu Ser Lys Lys Gln Glu Val Asn Met
   370 375 380
   Ser Asp Ser Ala Leu Asp Cys Val Arg Asp Glu Ala Ile Asn Lys Leu
   385 390 395 400
   Gln Gln Ile Phe Asn Thr Ser Tyr Asn Gln Thr Tyr Glu Lys Tyr Gly
   405 410 415
   Asn Val Ser Val Phe Glu Thr Ser Gly Gly Leu Val Val Phe Trp Gln
   420 425 430
   Gly Ile Lys Gln Lys Ser Leu Val Glu Leu Glu Arg Leu Ala Asn Arg
   435 440 445
   Ser Ser Leu Asn Ile Thr His Thr Thr Gln Thr Ser Thr Ser Asp Asn
   450 455 460
   Asn Thr Thr His Leu Ser Ser Met Glu Ser Val His Asn Leu Val Tyr
   465 470 475 480
   Ala Gln Leu Gln Phe Thr Tyr Asp Thr Leu Arg Gly Tyr Ile Asn Arg
   485 490 495
   Ala Leu Ala Gln Ile Ala Glu Ala Trp Cys Val Asp Gln Arg Arg Thr
   500 505 510
   Leu Glu Val Phe Lys Glu Leu Ser Lys Ile Asn Pro Ser Ala Ile Leu
   515 520 525
   Ser Ala Ile Tyr Asn Lys Pro Ile Ala Ala Arg Phe Met Gly Asp Val
   530 535 540
   Leu Gly Leu Ala Ser Cys Val Thr Ile Asn Gln Thr Ser Val Lys Val
   545 550 555 560
   Leu Arg Asp Met Asn Val Lys Glu Ser Pro Gly Arg Cys Tyr Ser Arg
   565 570 575
   Pro Val Val Ile Phe Asn Phe Ala Asn Ser Ser Tyr Val Gln Tyr Gly
   580 585 590
   Gln Leu Gly Glu Asp Asn Glu Ile Leu Leu Gly Asn His Arg Thr Glu
   595 600 605
   Glu Cys Gln Leu Pro Ser Leu Lys Ile Phe Ile Ala Gly Asn Ser Ala
   610 615 620
   Tyr Glu Tyr Val Asp Tyr Leu Phe Lys Arg Met Ile Asp Leu Ser Ser
   625 630 635 640
   Ile Ser Thr Val Asp Ser Met Ile Ala Leu Asp Ile Asp Pro Leu Glu
   645 650 655
   Asn Thr Asp Phe Arg Val Leu Glu Leu Tyr Ser Gln Lys Glu Leu Arg
   660 665 670
   Ser Ser Asn Val Phe Asp Leu Glu Glu Ile Met Arg Glu Phe Asn Ser
   675 680 685
   Tyr Lys Gln Arg Val Lys Tyr Val Glu Asp Lys Arg Leu Cys Thr Gln
   690 695 700
   Pro Leu Gln Asn Leu Phe Pro Tyr Leu Val Ser Ala Asp Gly Thr Thr
   705 710 715 720
   Val Thr Ser Gly Ser Thr Lys Asp Thr Ser Leu Gln Ala Pro Pro Ser
   725 730 735
   Tyr Glu Glu Ser Val Tyr Asn Ser Gly Arg Lys Gly Pro Gly Pro Pro
   740 745 750
   Ser Ser Asp Ala Ser Thr Ala Ala Pro Pro Tyr Thr Asn Glu Gln Ala
               755                 760                 765
   Tyr Gln Met Leu Leu Ala Leu Ala Arg Leu Asp Ala Glu Gln Arg Ala
           770                 775                 780
   Gln Gln Asn Gly Thr Asp Ser Leu Asp Gly Gln Thr Gly Thr Gln Asp
   785                 790                 795                 800
   Lys Gly Gln Lys Pro Asn Leu Leu Asp Arg Leu Arg His Arg Lys Asn
                   805                 810                 815
   Gly Tyr Arg His Leu Lys Asp Ser Asp Glu Glu Glu Asn Val
               820                 825                 830
<210> 4
   <211> 777
   <212> PRT
   <213> Artificial Sequence
<220>
   <223> AD169 Cytomegalovirus
<400> 4
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Ser Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Gly His Arg Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Ala
   225                 230                 235                 240
   Phe His Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Ser Ser Glu Ala Glu Asp Ser Tyr His Phe Ser Ser
           355                 360                 365
   Ala Lys Met Thr Ala Thr Phe Leu Ser Lys Lys Gln Glu Val Asn Met
       370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Thr Thr Gln Thr Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
       610                 615                 620
   Tyr Glu Tyr Val Asp Tyr Leu Phe Lys Arg Met Ile Asp Leu Ser Ser
   625                 630                 635                 640
   Ile Ser Thr Val Asp Ser Met Ile Ala Leu Asp Ile Asp Pro Leu Glu
                   645                 650                 655
   Asn Thr Asp Phe Arg Val Leu Glu Leu Tyr Ser Gln Lys Glu Leu Arg
               660                 665                 670
   Ser Ser Asn Val Phe Asp Leu Glu Glu Ile Met Arg Glu Phe Asn Ser
           675                 680                 685
   Tyr Lys Gln Arg Val Lys Tyr Val Glu Asp Lys Arg Leu Ala Thr Gln
       690                 695                 700
   Pro Leu Gln Asn Leu Phe Pro Tyr Leu Val Ser Ala Asp Gly Thr Thr
   705                 710                 715                 720
   Val Thr Ser Gly Ser Thr Lys Asp Thr Ser Leu Gln Ala Pro Pro Ser
                   725                 730                 735
   Tyr Glu Glu Ser Val Tyr Asn Ser Gly Arg Lys Gly Gly Gln Lys Pro
               740                 745                 750
   Asn Leu Leu Asp Arg Leu Arg His Arg Lys Asn Gly Tyr Arg His Leu
           755                 760                 765
   Lys Asp Ser Asp Glu Glu Glu Asn Val
       770                 775
<210> 5
   <211> 777
   <212> PRT
   <213> Artificial Sequence
<220>
   <223> AD169 Cytomegalovirus
<400> 5
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Ser Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Gly His Arg Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Trp
   225                 230                 235                 240
   Leu Tyr Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Ser Ser Glu Ala Glu Asp Ser Tyr His Phe Ser Ser
           355                 360                 365
   Ala Lys Met Thr Ala Thr Phe Leu Ser Lys Lys Gln Glu Val Asn Met
       370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Thr Thr Gln Thr Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
       610                 615                 620
   Tyr Glu Tyr Val Asp Tyr Leu Phe Lys Arg Met Ile Asp Leu Ser Ser
   625                 630                 635                 640
   Ile Ser Thr Val Asp Ser Met Ile Ala Leu Asp Ile Asp Pro Leu Glu
                   645                 650                 655
   Asn Thr Asp Phe Arg Val Leu Glu Leu Tyr Ser Gln Lys Glu Leu Arg
               660                 665                 670
   Ser Ser Asn Val Phe Asp Leu Glu Glu Ile Met Arg Glu Phe Asn Ser
           675                 680                 685
   Tyr Lys Gln Arg Val Lys Tyr Val Glu Asp Lys Arg Leu Ala Thr Gln
       690                 695                 700
   Pro Leu Gln Asn Leu Phe Pro Tyr Leu Val Ser Ala Asp Gly Thr Thr
   705                 710                 715                 720
   Val Thr Ser Gly Ser Thr Lys Asp Thr Ser Leu Gln Ala Pro Pro Ser
                   725                 730                 735
   Tyr Glu Glu Ser Val Tyr Asn Ser Gly Arg Lys Gly Gly Gln Lys Pro
               740                 745                 750
   Asn Leu Leu Asp Arg Leu Arg His Arg Lys Asn Gly Tyr Arg His Leu
           755                 760                 765
   Lys Asp Ser Asp Glu Glu Glu Asn Val
       770                 775
<210> 6
   <211> 2490
   <212> DNA
   <213> Artificial Sequence
<220>
   <223> AD169 Cytomegalovirus
<400> 6
   atggagagca ggatctggtg cctggtggtg tgcgtgaacc tgtgcatcgt gtgcctggga 60
   gccgccgtga gcagcagcag cacctcccac gccacctcca gcacccacaa cggcagccac 120
   accagcagga ccacctccgc ccagaccagc agcgtgtaca gccagcacgt gacctcttcc 180
   gaggccgtga gccacagggc caacgagaca atctacaaca ccaccctgaa gtacggcgac 240
   gtggtgggag tgaacaccac caagtacccc tacagagtgt gcagcatggc ccagggcacc 300
   gacctgatca gattcgagcg gaacatcatc tgcaccagca tgaagcccat caacgaggac 360
   ctggacgagg gcatcatggt ggtgtacaag aggaacatcg tggcccacac cttcaaggtc 420
   cgcgtgtacc agaaggtgct gaccttcagg cggagctacg ccggccacag gaccacctac 480
   ctgctgggca gcaacaccga gtacgtggcc ccacccatgt gggagatcca ccacatcaac 540
   aagttcgccc agtgctacag cagctacagc cgcgtgatcg gcggcaccgt gttcgtggcc 600
   taccacaggg acagctacga gaacaagacc atgcagctga tccccgacga ctacagcaac 660
   acccacagca ccagatacgt gaccgtgaag gaccagtggc acagcagggg cagcaccgcc 720
   ttccacagag agacatgcaa cctgaactgc atgctgacca tcaccaccgc cagaagcaag 780
   tacccttacc acttcttcgc cacctctacc ggggacgtgg tgtacatcag ccccttctac 840
   aacggcacca acaggaacgc cagctacttc ggcgagaacg ccgacaagtt cttcatcttc 900
   cccaactaca ccatcgtgtc cgacttcggc aggcccaacg ctgcccccga gacacacaga 960
   ctggtggcct ttctggagag ggccgacagc gtgatcagct gggacatcca ggacgagaag 1020
   aacgtgacct gccagctgac cttctgggag gccagcgaga ggaccatcag cagcgaggcc 1080
   gaggacagct accacttcag cagcgccaag atgaccgcca ccttcctgag caagaaacaa 1140
   gaggtgaaca tgagcgacag cgccctggac tgcgtgaggg acgaggccat caacaagctg 1200
   cagcagatct tcaacaccag ctacaaccag acctacgaga agtacggaaa cgtgtccgtg 1260
   ttcgagacaa gcggcggact ggtggtgttc tggcagggca tcaagcagaa gtccctggtc 1320
   gagctggaga ggctggccaa caggtccagc ctgaacatca cccacaccac ccagaccagc 1380
   accagcgaca acaacaccac acacctgagc agcatggaaa gcgtgcacaa cctggtgtat 1440
   gcccagctgc agttcaccta cgacaccctg aggggctaca tcaacagagc cctggcccag 1500
   atcgccgagg cttggtgcgt ggaccagaga aggaccctgg aggtgttcaa ggaactgtcc 1560
   aagatcaacc ccagcgccat cctgagcgcc atctacaaca agccaatcgc cgccagattc 1620
   atgggcgacg tgctgggcct ggccagctgc gtgaccatca accagaccag cgtgaaggtg 1680
   ctgcgggaca tgaacgtgaa ggaaagccca ggccgctgct actccaggcc cgtggtgatc 1740
   ttcaacttcg ccaacagctc ctacgtgcag tacggccagc tgggcgagga caacgagatc 1800
   ctgctgggaa accacaggac cgaggaatgc cagctgccca gcctgaagat ctttatcgcc 1860
   ggcaactccg cctacgagta cgtggactac ctgttcaaga ggatgatcga cctgtccagc 1920
   atcagcaccg tggacagcat gatcgccctg gatatcgatc ccctggagaa caccgacttc 1980
   agggtgctgg agctgtactc ccagaaggaa ctgaggtcca gcaacgtgtt cgacctggag 2040
   gaaatcatga gagagttcaa cagctacaag cagcgcgtga agtacgtgga ggacaagagg 2100
   ctgtgcaccc agcccctgca gaacctgttc ccctacctgg tgtccgccga cggcaccacc 2160
   gtgaccagcg gctccaccaa ggacaccagc ctgcaggccc ctcccagcta cgaggaaagc 2220
   gtgtacaaca gcggcaggaa gggcccaggc cccccaagca gcgacgccag cacagccgcc 2280
   ccaccctaca ccaacgagca ggcctaccag atgctgctgg ccctggcccg gctggacgcc 2340
   gagcagaggg cccagcagaa cggcaccgac agcctggacg gccagaccgg cacccaggac 2400
   aagggccaga agcccaacct gctggacagg ctgaggcaca ggaagaacgg ctacaggcac 2460
   ctgaaggaca gcgacgagga agaaaacgtg                                  2490
<210> 7
   <211> 2331
   <212> DNA
   <213> Artificial Sequence
<220>
   <223> AD169 Cytomegalovirus
<400> 7
   atggagagca ggatctggtg cctggtggtg tgcgtgaacc tgtgcatcgt gtgcctggga 60
   gccgccgtga gcagcagcag cacctcccac gccacctcca gcacccacaa cggcagccac 120
   accagcagga ccacctccgc ccagaccagc agcgtgtaca gccagcacgt gacctcttcc 180
   gaggccgtga gccacagggc caacgagaca atctacaaca ccaccctgaa gtacggcgac 240
   gtggtgggag tgaacaccac caagtacccc tacagagtgt gcagcatggc ccagggcacc 300
   gacctgatca gattcgagcg gaacatcatc tgcaccagca tgaagcccat caacgaggac 360
   ctggacgagg gcatcatggt ggtgtacaag aggaacatcg tggcccacac cttcaaggtc 420
   cgcgtgtacc agaaggtgct gaccttcagg cggagctacg ccggccacag gaccacctac 480
   ctgctgggca gcaacaccga gtacgtggcc ccacccatgt gggagatcca ccacatcaac 540
   aagttcgccc agtgctacag cagctacagc cgcgtgatcg gcggcaccgt gttcgtggcc 600
   taccacaggg acagctacga gaacaagacc atgcagctga tccccgacga ctacagcaac 660
   acccacagca ccagatacgt gaccgtgaag gaccagtggc acagcagggg cagcacctgg 720
   ctgtacagag agacatgcaa cctgaactgc atgctgacca tcaccaccgc cagaagcaag 780
   tacccttacc acttcttcgc cacctctacc ggggacgtgg tgtacatcag ccccttctac 840
   aacggcacca acaggaacgc cagctacttc ggcgagaacg ccgacaagtt cttcatcttc 900
   cccaactaca ccatcgtgtc cgacttcggc aggcccaacg ctgcccccga gacacacaga 960
   ctggtggcct ttctggagag ggccgacagc gtgatcagct gggacatcca ggacgagaag 1020
   aacgtgacct gccagctgac cttctgggag gccagcgaga ggaccatcag cagcgaggcc 1080
   gaggacagct accacttcag cagcgccaag atgaccgcca ccttcctgag caagaaacaa 1140
   gaggtgaaca tgagcgacag cgccctggac tgcgtgaggg acgaggccat caacaagctg 1200
   cagcagatct tcaacaccag ctacaaccag acctacgaga agtacggaaa cgtgtccgtg 1260
   ttcgagacaa gcggcggact ggtggtgttc tggcagggca tcaagcagaa gtccctggtc 1320
   gagctggaga ggctggccaa caggtccagc ctgaacatca cccacaccac ccagaccagc 1380
   accagcgaca acaacaccac acacctgagc agcatggaaa gcgtgcacaa cctggtgtat 1440
   gcccagctgc agttcaccta cgacaccctg aggggctaca tcaacagagc cctggcccag 1500
   atcgccgagg cttggtgcgt ggaccagaga aggaccctgg aggtgttcaa ggaactgtcc 1560
   aagatcaacc ccagcgccat cctgagcgcc atctacaaca agccaatcgc cgccagattc 1620
   atgggcgacg tgctgggcct ggccagctgc gtgaccatca accagaccag cgtgaaggtg 1680
   ctgcgggaca tgaacgtgaa ggaaagccca ggccgctgct actccaggcc cgtggtgatc 1740
   ttcaacttcg ccaacagctc ctacgtgcag tacggccagc tgggcgagga caacgagatc 1800
   ctgctgggaa accacaggac cgaggaatgc cagctgccca gcctgaagat ctttatcgcc 1860
   ggcaactccg cctacgagta cgtggactac ctgttcaaga ggatgatcga cctgtccagc 1920
   atcagcaccg tggacagcat gatcgccctg gatatcgatc ccctggagaa caccgacttc 1980
   agggtgctgg agctgtactc ccagaaggaa ctgaggtcca gcaacgtgtt cgacctggag 2040
   gaaatcatga gagagttcaa cagctacaag cagcgcgtga agtacgtgga ggacaagagg 2100
   ctggccaccc agcccctgca gaacctgttc ccctacctgg tgtccgccga cggcaccacc 2160
   gtgaccagcg gctccaccaa ggacaccagc ctgcaggccc ctcccagcta cgaggaaagc 2220
   gtgtacaaca gcggcaggaa gggcggccag aagcccaacc tgctggacag gctgaggcac 2280
   aggaagaacg gctacaggca cctgaaggac agcgacgagg aagaaaacgt g          2331
<210> 8
   <211> 2331
   <212> DNA
   <213> Artificial Sequence
<220>
   <223> AD169 Cytomegalovirus
<400> 8
   atggagagca ggatctggtg cctggtggtg tgcgtgaacc tgtgcatcgt gtgcctggga 60
   gccgccgtga gcagcagcag cacctcccac gccacctcca gcacccacaa cggcagccac 120
   accagcagga ccacctccgc ccagaccagc agcgtgtaca gccagcacgt gacctcttcc 180
   gaggccgtga gccacagggc caacgagaca atctacaaca ccaccctgaa gtacggcgac 240
   gtggtgggag tgaacaccac caagtacccc tacagagtgt gcagcatggc ccagggcacc 300
   gacctgatca gattcgagcg gaacatcatc tgcaccagca tgaagcccat caacgaggac 360
   ctggacgagg gcatcatggt ggtgtacaag aggaacatcg tggcccacac cttcaaggtc 420
   cgcgtgtacc agaaggtgct gaccttcagg cggagctacg ccggccacag gaccacctac 480
   ctgctgggca gcaacaccga gtacgtggcc ccacccatgt gggagatcca ccacatcaac 540
   aagttcgccc agtgctacag cagctacagc cgcgtgatcg gcggcaccgt gttcgtggcc 600
   taccacaggg acagctacga gaacaagacc atgcagctga tccccgacga ctacagcaac 660
   acccacagca ccagatacgt gaccgtgaag gaccagtggc acagcagggg cagcaccgcc 720
   ttccacagag agacatgcaa cctgaactgc atgctgacca tcaccaccgc cagaagcaag 780
   tacccttacc acttcttcgc cacctctacc ggggacgtgg tgtacatcag ccccttctac 840
   aacggcacca acaggaacgc cagctacttc ggcgagaacg ccgacaagtt cttcatcttc 900
   cccaactaca ccatcgtgtc cgacttcggc aggcccaacg ctgcccccga gacacacaga 960
   ctggtggcct ttctggagag ggccgacagc gtgatcagct gggacatcca ggacgagaag 1020
   aacgtgacct gccagctgac cttctgggag gccagcgaga ggaccatcag cagcgaggcc 1080
   gaggacagct accacttcag cagcgccaag atgaccgcca ccttcctgag caagaaacaa 1140
   gaggtgaaca tgagcgacag cgccctggac tgcgtgaggg acgaggccat caacaagctg 1200
   cagcagatct tcaacaccag ctacaaccag acctacgaga agtacggaaa cgtgtccgtg 1260
   ttcgagacaa gcggcggact ggtggtgttc tggcagggca tcaagcagaa gtccctggtc 1320
   gagctggaga ggctggccaa caggtccagc ctgaacatca cccacaccac ccagaccagc 1380
   accagcgaca acaacaccac acacctgagc agcatggaaa gcgtgcacaa cctggtgtat 1440
   gcccagctgc agttcaccta cgacaccctg aggggctaca tcaacagagc cctggcccag 1500
   atcgccgagg cttggtgcgt ggaccagaga aggaccctgg aggtgttcaa ggaactgtcc 1560
   aagatcaacc ccagcgccat cctgagcgcc atctacaaca agccaatcgc cgccagattc 1620
   atgggcgacg tgctgggcct ggccagctgc gtgaccatca accagaccag cgtgaaggtg 1680
   ctgcgggaca tgaacgtgaa ggaaagccca ggccgctgct actccaggcc cgtggtgatc 1740
   ttcaacttcg ccaacagctc ctacgtgcag tacggccagc tgggcgagga caacgagatc 1800
   ctgctgggaa accacaggac cgaggaatgc cagctgccca gcctgaagat ctttatcgcc 1860
   ggcaactccg cctacgagta cgtggactac ctgttcaaga ggatgatcga cctgtccagc 1920
   atcagcaccg tggacagcat gatcgccctg gatatcgatc ccctggagaa caccgacttc 1980
   agggtgctgg agctgtactc ccagaaggaa ctgaggtcca gcaacgtgtt cgacctggag 2040
   gaaatcatga gagagttcaa cagctacaag cagcgcgtga agtacgtgga ggacaagagg 2100
   ctggccaccc agcccctgca gaacctgttc ccctacctgg tgtccgccga cggcaccacc 2160
   gtgaccagcg gctccaccaa ggacaccagc ctgcaggccc ctcccagcta cgaggaaagc 2220
   gtgtacaaca gcggcaggaa gggcggccag aagcccaacc tgctggacag gctgaggcac 2280
   aggaagaacg gctacaggca cctgaaggac agcgacgagg aagaaaacgt g          2331
<210> 9
   <211> 2172
   <212> DNA
   <213> Artificial Sequence
<220>
   <223> AD169 Cytomegalovirus
<400> 9
   atggagagca ggatctggtg cctggtggtg tgcgtgaacc tgtgcatcgt gtgcctggga 60
   gccgccgtga gcagcagcag cacctcccac gccacctcca gcacccacaa cggcagccac 120
   accagcagga ccacctccgc ccagaccaga agcgtgtaca gccagcacgt gacctcttcc 180
   gaggccgtga gccacagggc caacgagaca atctacaaca ccaccctgaa gtacggcgac 240
   gtggtgggag tgaacaccac caagtacccc tacagagtgt gcagcatggc ccagggcacc 300
   gacctgatca gattcgagcg gaacatcatc tgcaccagca tgaagcccat caacgaggac 360
   ctggacgagg gcatcatggt ggtgtacaag aggaacatcg tggcccacac cttcaaggtc 420
   cgcgtgtacc agaaggtgct gaccttcagg cggagctacg ccggccacag gaccacctac 480
   ctgctgggca gcaacaccga gtacgtggcc ccacccatgt gggagatcca ccacatcaac 540
   aagttcgccc agtgctacag cagctacagc cgcgtgatcg gcggcaccgt gttcgtggcc 600
   taccacaggg acagctacga gaacaagacc atgcagctga tccccgacga ctacagcaac 660
   acccacagca ccagatacgt gaccgtgaag gaccagtggc acagcagggg cagcaccgcc 720
   ttccacagag agacatgcaa cctgaactgc atgctgacca tcaccaccgc cagaagcaag 780
   tacccttacc acttcttcgc cacctctacc ggggacgtgg tgtacatcag ccccttctac 840
   aacggcacca acaggaacgc cagctacttc ggcgagaacg ccgacaagtt cttcatcttc 900
   cccaactaca ccatcgtgtc cgacttcggc aggcccaacg ctgcccccga gacacacaga 960
   ctggtggcct ttctggagag ggccgacagc gtgatcagct gggacatcca ggacgagaag 1020
   aacgtgacct gccagctgac cttctgggag gccagcgaga ggaccatcag aagcgaggcc 1080
   gaggacagct accacttcag cagcgccaag atgaccgcca ccttcctgag caagaaacaa 1140
   gaggtgaaca tgagcgacag cgccctggac tgcgtgaggg acgaggccat caacaagctg 1200
   cagcagatct tcaacaccag ctacaaccag acctacgaga agtacggaaa cgtgtccgtg 1260
   ttcgagacaa gcggcggact ggtggtgttc tggcagggca tcaagcagaa gtccctggtc 1320
   gagctggaga ggctggccaa caggtccagc ctgaacatca cccacaccac ccagaccagc 1380
   accagcgaca acaacaccac acacctgagc agcatggaaa gcgtgcacaa cctggtgtat 1440
   gcccagctgc agttcaccta cgacaccctg aggggctaca tcaacagagc cctggcccag 1500
   atcgccgagg cttggtgcgt ggaccagaga aggaccctgg aggtgttcaa ggaactgtcc 1560
   aagatcaacc ccagcgccat cctgagcgcc atctacaaca agccaatcgc cgccagattc 1620
   atgggcgacg tgctgggcct ggccagctgc gtgaccatca accagaccag cgtgaaggtg 1680
   ctgcgggaca tgaacgtgaa ggaaagccca ggccgctgct actccaggcc cgtggtgatc 1740
   ttcaacttcg ccaacagctc ctacgtgcag tacggccagc tgggcgagga caacgagatc 1800
   ctgctgggaa accacaggac cgaggaatgc cagctgccca gcctgaagat ctttatcgcc 1860
   ggcaactccg cctacgagta cgtggactac ctgttcaaga ggatgatcga cctgtccagc 1920
   atcagcaccg tggacagcat gatcgccctg gatatcgatc ccctggagaa caccgacttc 1980
   agggtgctgg agctgtactc ccagaaggaa ctgaggtcca gcaacgtgtt cgacctggag 2040
   gaaatcatga gagagttcaa cagctacaag cagcgcgtga agtacgtgga ggacaaggtg 2100
   gtggaccccc tgccccccta cctgaagggc ctggacgacc tgatgagcgg actcggggct 2160
   gctggaaagg cc 2172
<210> 10
   <211> 724
   <212> PRT
   <213> Artificial Sequence
<220>
   <223> AD169 Cytomegalovirus
<400> 10
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Arg Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Gly His Arg Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Ala
   225                 230                 235                 240
   Phe His Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Arg Ser Glu Ala Glu Asp Ser Tyr His Phe Ser Ser
               355                 360                 365
   Ala Lys Met Thr Ala Thr Phe Leu Ser Lys Lys Gln Glu Val Asn Met
           370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Thr Thr Gln Thr Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
       610                 615                 620
   Tyr Glu Tyr Val Asp Tyr Leu Phe Lys Arg Met Ile Asp Leu Ser Ser
   625                 630                 635                 640
   Ile Ser Thr Val Asp Ser Met Ile Ala Leu Asp Ile Asp Pro Leu Glu
                   645                 650                 655
   Asn Thr Asp Phe Arg Val Leu Glu Leu Tyr Ser Gln Lys Glu Leu Arg
               660                 665                 670
   Ser Ser Asn Val Phe Asp Leu Glu Glu Ile Met Arg Glu Phe Asn Ser
           675                 680                 685
   Tyr Lys Gln Arg Val Lys Tyr Val Glu Asp Lys Val Val Asp Pro Leu
       690                 695                 700
   Pro Pro Tyr Leu Lys Gly Leu Asp Asp Leu Met Ser Gly Leu Gly Ala
   705                 710                 715                 720
   Ala Gly Lys Ala
<210> 11
   <211> 2151
   <212> DNA
   <213> Artificial Sequence
<220>
   <223> AD169 Cytomegalovirus
<400> 11
   atggagagca ggatctggtg cctggtggtg tgcgtgaacc tgtgcatcgt gtgcctggga 60
   gccgccgtga gcagcagcag cacctcccac gccacctcca gcacccacaa cggcagccac 120
   accagcagga ccacctccgc ccagaccaga agcgtgtaca gccagcacgt gacctcttcc 180
   gaggccgtga gccacagggc caacgagaca atctacaaca ccaccctgaa gtacggcgac 240
   gtggtgggag tgaacaccac caagtacccc tacagagtgt gcagcatggc ccagggcacc 300
   gacctgatca gattcgagcg gaacatcatc tgcaccagca tgaagcccat caacgaggac 360
   ctggacgagg gcatcatggt ggtgtacaag aggaacatcg tggcccacac cttcaaggtc 420
   cgcgtgtacc agaaggtgct gaccttcagg cggagctacg ccggccacag gaccacctac 480
   ctgctgggca gcaacaccga gtacgtggcc ccacccatgt gggagatcca ccacatcaac 540
   aagttcgccc agtgctacag cagctacagc cgcgtgatcg gcggcaccgt gttcgtggcc 600
   taccacaggg acagctacga gaacaagacc atgcagctga tccccgacga ctacagcaac 660
   acccacagca ccagatacgt gaccgtgaag gaccagtggc acagcagggg cagcaccgcc 720
   ttccacagag agacatgcaa cctgaactgc atgctgacca tcaccaccgc cagaagcaag 780
   tacccttacc acttcttcgc cacctctacc ggggacgtgg tgtacatcag ccccttctac 840
   aacggcacca acaggaacgc cagctacttc ggcgagaacg ccgacaagtt cttcatcttc 900
   cccaactaca ccatcgtgtc cgacttcggc aggcccaacg ctgcccccga gacacacaga 960
   ctggtggcct ttctggagag ggccgacagc gtgatcagct gggacatcca ggacgagaag 1020
   aacgtgacct gccagctgac cttctgggag gccagcgaga ggaccatcag aagcgaggcc 1080
   gaggacagct accacttcag cagcgccaag atgaccgcca ccttcctgag caagaaacaa 1140
   gaggtgaaca tgagcgacag cgccctggac tgcgtgaggg acgaggccat caacaagctg 1200
   cagcagatct tcaacaccag ctacaaccag acctacgaga agtacggaaa cgtgtccgtg 1260
   ttcgagacaa gcggcggact ggtggtgttc tggcagggca tcaagcagaa gtccctggtc 1320
   gagctggaga ggctggccaa caggtccagc ctgaacatca cccacaccac ccagaccagc 1380
   accagcgaca acaacaccac acacctgagc agcatggaaa gcgtgcacaa cctggtgtat 1440
   gcccagctgc agttcaccta cgacaccctg aggggctaca tcaacagagc cctggcccag 1500
   atcgccgagg cttggtgcgt ggaccagaga aggaccctgg aggtgttcaa ggaactgtcc 1560
   aagatcaacc ccagcgccat cctgagcgcc atctacaaca agccaatcgc cgccagattc 1620
   atgggcgacg tgctgggcct ggccagctgc gtgaccatca accagaccag cgtgaaggtg 1680
   ctgcgggaca tgaacgtgaa ggaaagccca ggccgctgct actccaggcc cgtggtgatc 1740
   ttcaacttcg ccaacagctc ctacgtgcag tacggccagc tgggcgagga caacgagatc 1800
   ctgctgggaa accacaggac cgaggaatgc cagctgccca gcctgaagat ctttatcgcc 1860
   ggcaactccg cctacgagta cgtggactac ctgttcaaga ggatgatcga cctgtccagc 1920
   atcagcaccg tggacagcat gatcgccctg gatatcgatc ccctggagaa caccgacttc 1980
   agggtgctgg agctgtactc ccagaaggaa ctgaggtcca gcaacgtgtt cgacctggag 2040
   gaaatcatga gagagttcaa cagctacaag cagcgcgtga agtacgtgga ggacaagagg 2100
   ctggccaccc agcccctgca gaacctgttc ccctacctgg tgtccgccga c          2151
<210> 12
   <211> 717
   <212> PRT
   <213> Artificial Sequence
<220>
   <223> AD169 Cytomegalovirus
<400> 12
   Met Glu Ser Arg Ile Trp Cys Leu Val Val Cys Val Asn Leu Cys Ile
    1               5                  10                  15
   Val Cys Leu Gly Ala Ala Val Ser Ser Ser Ser Thr Ser His Ala Thr
               20                  25                  30
   Ser Ser Thr His Asn Gly Ser His Thr Ser Arg Thr Thr Ser Ala Gln
           35                  40                  45
   Thr Arg Ser Val Tyr Ser Gln His Val Thr Ser Ser Glu Ala Val Ser
       50                  55                  60
   His Arg Ala Asn Glu Thr Ile Tyr Asn Thr Thr Leu Lys Tyr Gly Asp
   65                  70                  75                  80
   Val Val Gly Val Asn Thr Thr Lys Tyr Pro Tyr Arg Val Cys Ser Met
                   85                  90                  95
   Ala Gln Gly Thr Asp Leu Ile Arg Phe Glu Arg Asn Ile Ile Cys Thr
               100                 105                 110
   Ser Met Lys Pro Ile Asn Glu Asp Leu Asp Glu Gly Ile Met Val Val
           115                 120                 125
   Tyr Lys Arg Asn Ile Val Ala His Thr Phe Lys Val Arg Val Tyr Gln
       130                 135                 140
   Lys Val Leu Thr Phe Arg Arg Ser Tyr Ala Gly His Arg Thr Thr Tyr
   145                 150                 155                 160
   Leu Leu Gly Ser Asn Thr Glu Tyr Val Ala Pro Pro Met Trp Glu Ile
                   165                 170                 175
   His His Ile Asn Lys Phe Ala Gln Cys Tyr Ser Ser Tyr Ser Arg Val
               180                 185                 190
   Ile Gly Gly Thr Val Phe Val Ala Tyr His Arg Asp Ser Tyr Glu Asn
           195                 200                 205
   Lys Thr Met Gln Leu Ile Pro Asp Asp Tyr Ser Asn Thr His Ser Thr
       210                 215                 220
   Arg Tyr Val Thr Val Lys Asp Gln Trp His Ser Arg Gly Ser Thr Ala
   225                 230                 235                 240
   Phe His Arg Glu Thr Cys Asn Leu Asn Cys Met Leu Thr Ile Thr Thr
                   245                 250                 255
   Ala Arg Ser Lys Tyr Pro Tyr His Phe Phe Ala Thr Ser Thr Gly Asp
               260                 265                 270
   Val Val Tyr Ile Ser Pro Phe Tyr Asn Gly Thr Asn Arg Asn Ala Ser
           275                 280                 285
   Tyr Phe Gly Glu Asn Ala Asp Lys Phe Phe Ile Phe Pro Asn Tyr Thr
       290                 295                 300
   Ile Val Ser Asp Phe Gly Arg Pro Asn Ala Ala Pro Glu Thr His Arg
   305                 310                 315                 320
   Leu Val Ala Phe Leu Glu Arg Ala Asp Ser Val Ile Ser Trp Asp Ile
                   325                 330                 335
   Gln Asp Glu Lys Asn Val Thr Cys Gln Leu Thr Phe Trp Glu Ala Ser
               340                 345                 350
   Glu Arg Thr Ile Arg Ser Glu Ala Glu Asp Ser Tyr His Phe Ser Ser
           355                 360                 365
   Ala Lys Met Thr Ala Thr Phe Leu Ser Lys Lys Gln Glu Val Asn Met
       370                 375                 380
   Ser Asp Ser Ala Leu Asp Cys Val Arg Asp Glu Ala Ile Asn Lys Leu
   385                 390                 395                 400
   Gln Gln Ile Phe Asn Thr Ser Tyr Asn Gln Thr Tyr Glu Lys Tyr Gly
                   405                 410                 415
   Asn Val Ser Val Phe Glu Thr Ser Gly Gly Leu Val Val Phe Trp Gln
               420                 425                 430
   Gly Ile Lys Gln Lys Ser Leu Val Glu Leu Glu Arg Leu Ala Asn Arg
           435                 440                 445
   Ser Ser Leu Asn Ile Thr His Thr Thr Gln Thr Ser Thr Ser Asp Asn
       450                 455                 460
   Asn Thr Thr His Leu Ser Ser Met Glu Ser Val His Asn Leu Val Tyr
   465                 470                 475                 480
   Ala Gln Leu Gln Phe Thr Tyr Asp Thr Leu Arg Gly Tyr Ile Asn Arg
                   485                 490                 495
   Ala Leu Ala Gln Ile Ala Glu Ala Trp Cys Val Asp Gln Arg Arg Thr
               500                 505                 510
   Leu Glu Val Phe Lys Glu Leu Ser Lys Ile Asn Pro Ser Ala Ile Leu
           515                 520                 525
   Ser Ala Ile Tyr Asn Lys Pro Ile Ala Ala Arg Phe Met Gly Asp Val
       530                 535                 540
   Leu Gly Leu Ala Ser Cys Val Thr Ile Asn Gln Thr Ser Val Lys Val
   545                 550                 555                 560
   Leu Arg Asp Met Asn Val Lys Glu Ser Pro Gly Arg Cys Tyr Ser Arg
                   565                 570                 575
   Pro Val Val Ile Phe Asn Phe Ala Asn Ser Ser Tyr Val Gln Tyr Gly
               580                 585                 590
   Gln Leu Gly Glu Asp Asn Glu Ile Leu Leu Gly Asn His Arg Thr Glu
           595                 600                 605
   Glu Cys Gln Leu Pro Ser Leu Lys Ile Phe Ile Ala Gly Asn Ser Ala
   610 615 620
   Tyr Glu Tyr Val Asp Tyr Leu Phe Lys Arg Met Ile Asp Leu Ser Ser
   625 630 635 640
   Ile Ser Thr Val Asp Ser Met Ile Ala Leu Asp Ile Asp Pro Leu Glu
   645 650 655
   Asn Thr Asp Phe Arg Val Leu Glu Leu Tyr Ser Gln Lys Glu Leu Arg
   660 665 670
   Ser Ser Asn Val Phe Asp Leu Glu Glu Ile Met Arg Glu Phe Asn Ser
   675 680 685
   Tyr Lys Gln Arg Val Lys Tyr Val Glu Asp Lys Arg Leu Ala Thr Gln
   690 695 700
   Pro Leu Gln Asn Leu Phe Pro Tyr Leu Val Ser Ala Asp
   705 710 715
<210> 13
   <211> 2049
   <212> DNA
   <213> Artificial Sequence
<220>
   <223> AD169 Cytomegalovirus
<400> 13
   atggaatccc ggatctggtg cctggtggtg tgcgtgaacc tgtgcatcgt gtgcctggcc 60
   ctggccagcc accgggccaa cgagacaatc tacaacacca ccctgaagta cggcgacgtg 120
   gtgggagtga acaccaccaa gtacccctac agagtgtgct ccatggccca gggcaccgac 180
   ctgatcagat tcgagcggaa catcatctgc accagcatga agcccatcaa cgaggacctg 240
   gacgagggca tcatggtggt gtacaagagg aacatcgtgg cccacacctt caaggtccgc 300
   gtgtaccaga aggtgctgac cttcaggcgg agctacgccg gccacaggac cacctacctg 360
   ctgggcagca acaccgagta cgtggcccca cccatgtggg agatccacca catcaacaag 420
   ttcgcccagt gctacagcag ctacagccgc gtgatcggcg gcaccgtgtt cgtggcctac 480
   cacagggaca gctacgagaa caagaccatg cagctgatcc ccgacgacta cagcaacacc 540
   cacagcacca gatacgtgac cgtgaaggac cagtggcaca gcaggggcag caccgccttc 600
   cacagagaga catgcaacct gaactgcatg ctgaccatca ccaccgccag aagcaagtac 660
   ccttaccact tcttcgccac ctctaccggg gacgtggtgt acatcagccc cttctacaac 720
   ggcaccaaca ggaacgccag ctacttcggc gagaacgccg acaagttctt catcttcccc 780
   aactacacca tcgtgtccga cttcggcagg cccaacgctg cccccgagac acacagactg 840
   gtggcctttc tggagagggc cgacagcgtg atcagctggg acatccagga cgagaagaac 900
   gtgacctgcc agctgacctt ctgggaggcc agcgagagga ccatcagaag cgaggccgag 960
   gacagctacc acttcagcag cgccaagatg accgccacct tcctgagcaa gaaacaagag 1020
   gtgaacatga gcgacagcgc cctggactgc gtgagggacg aggccatcaa caagctgcag 1080
   cagatcttca acaccagcta caaccagacc tacgagaagt acggaaacgt gtccgtgttc 1140
   gagacaagcg gcggactggt ggtgttctgg cagggcatca agcagaagtc cctggtcgag 1200
   ctggagaggc tggccaacag gtccagcctg aacatcaccc acaccaccca gaccagcacc 1260
   agcgacaaca acaccacaca cctgagcagc atggaaagcg tgcacaacct ggtgtatgcc 1320
   cagctgcagt tcacctacga caccctgagg ggctacatca acagagccct ggcccagatc 1380
   gccgaggctt ggtgcgtgga ccagagaagg accctggagg tgttcaagga actgtccaag 1440
   atcaacccca gcgccatcct gagcgccatc tacaacaagc caatcgccgc cagattcatg 1500
   ggcgacgtgc tgggcctggc cagctgcgtg accatcaacc agaccagcgt gaaggtgctg 1560
   cgggacatga acgtgaagga aagcccaggc cgctgctact ccaggcccgt ggtgatcttc 1620
   aacttcgcca acagctccta cgtgcagtac ggccagctgg gcgaggacaa cgagatcctg 1680
   ctgggaaacc acaggaccga ggaatgccag ctgcccagcc tgaagatctt tatcgccggc 1740
   aactccgcct acgagtacgt ggactacctg ttcaagagga tgatcgacct gtccagcatc 1800
   agcaccgtgg acagcatgat cgccctggat atcgatcccc tggagaacac cgacttcagg 1860
   gtgctggagc tgtactccca gaaggaactg aggtccagca acgtgttcga cctggaggaa 1920
   atcatgagag agttcaacag ctacaagcag cgcgtgaagt acgtggagga caaggtggtg 1980
   gaccccctgc ccccctacct gaagggcctg gacgacctga tgagcggact cggggctgct 2040
   ggaaaggcc 2049
<210> 14
   <211> 683
   <212> PRT
   <213> Artificial Sequence
<220>
   <223> AD169 Cytomegalovirus
<400> 14
   Met Glu Ser Arg Ile Trp Cys Leu Val Val Cys Val Asn Leu Cys Ile
    1               5                  10                  15
   Val Cys Leu Ala Leu Ala Ser His Arg Ala Asn Glu Thr Ile Tyr Asn
               20                  25                  30
   Thr Thr Leu Lys Tyr Gly Asp Val Val Gly Val Asn Thr Thr Lys Tyr
           35                  40                  45
   Pro Tyr Arg Val Cys Ser Met Ala Gln Gly Thr Asp Leu Ile Arg Phe
       50                  55                  60
   Glu Arg Asn Ile Ile Cys Thr Ser Met Lys Pro Ile Asn Glu Asp Leu
   65                  70                  75                  80
   Asp Glu Gly Ile Met Val Val Tyr Lys Arg Asn Ile Val Ala His Thr
                   85                  90                  95
   Phe Lys Val Arg Val Tyr Gln Lys Val Leu Thr Phe Arg Arg Ser Tyr
               100                 105                 110
   Ala Gly His Arg Thr Thr Tyr Leu Leu Gly Ser Asn Thr Glu Tyr Val
           115                 120                 125
   Ala Pro Pro Met Trp Glu Ile His His Ile Asn Lys Phe Ala Gln Cys
       130                 135                 140
   Tyr Ser Ser Tyr Ser Arg Val Ile Gly Gly Thr Val Phe Val Ala Tyr
   145                 150                 155                 160
   His Arg Asp Ser Tyr Glu Asn Lys Thr Met Gln Leu Ile Pro Asp Asp
                   165                 170                 175
   Tyr Ser Asn Thr His Ser Thr Arg Tyr Val Thr Val Lys Asp Gln Trp
               180                 185                 190
   His Ser Arg Gly Ser Thr Ala Phe His Arg Glu Thr Cys Asn Leu Asn
           195                 200                 205
   Cys Met Leu Thr Ile Thr Thr Ala Arg Ser Lys Tyr Pro Tyr His Phe
       210                 215                 220
   Phe Ala Thr Ser Thr Gly Asp Val Val Tyr Ile Ser Pro Phe Tyr Asn
   225                 230                 235                 240
   Gly Thr Asn Arg Asn Ala Ser Tyr Phe Gly Glu Asn Ala Asp Lys Phe
                   245                 250                 255
   Phe Ile Phe Pro Asn Tyr Thr Ile Val Ser Asp Phe Gly Arg Pro Asn
               260                 265                 270
   Ala Ala Pro Glu Thr His Arg Leu Val Ala Phe Leu Glu Arg Ala Asp
           275                 280                 285
   Ser Val Ile Ser Trp Asp Ile Gln Asp Glu Lys Asn Val Thr Cys Gln
       290                 295                 300
   Leu Thr Phe Trp Glu Ala Ser Glu Arg Thr Ile Arg Ser Glu Ala Glu
   305                 310                 315                 320
   Asp Ser Tyr His Phe Ser Ser Ala Lys Met Thr Ala Thr Phe Leu Ser
                   325                 330                 335
   Lys Lys Gln Glu Val Asn Met Ser Asp Ser Ala Leu Asp Cys Val Arg
               340                 345                 350
   Asp Glu Ala Ile Asn Lys Leu Gln Gln Ile Phe Asn Thr Ser Tyr Asn
           355                 360                 365
   Gln Thr Tyr Glu Lys Tyr Gly Asn Val Ser Val Phe Glu Thr Ser Gly
       370                 375                 380
   Gly Leu Val Val Phe Trp Gln Gly Ile Lys Gln Lys Ser Leu Val Glu
   385                 390                 395                 400
   Leu Glu Arg Leu Ala Asn Arg Ser Ser Leu Asn Ile Thr His Thr Thr
                   405                 410                 415
   Gln Thr Ser Thr Ser Asp Asn Asn Thr Thr His Leu Ser Ser Met Glu
               420                 425                 430
   Ser Val His Asn Leu Val Tyr Ala Gln Leu Gln Phe Thr Tyr Asp Thr
           435                 440                 445
   Leu Arg Gly Tyr Ile Asn Arg Ala Leu Ala Gln Ile Ala Glu Ala Trp
       450                 455                 460
   Cys Val Asp Gln Arg Arg Thr Leu Glu Val Phe Lys Glu Leu Ser Lys
   465                 470                 475                 480
   Ile Asn Pro Ser Ala Ile Leu Ser Ala Ile Tyr Asn Lys Pro Ile Ala
                   485                 490                 495
   Ala Arg Phe Met Gly Asp Val Leu Gly Leu Ala Ser Cys Val Thr Ile
               500                 505                 510
   Asn Gln Thr Ser Val Lys Val Leu Arg Asp Met Asn Val Lys Glu Ser
           515                 520                 525
   Pro Gly Arg Cys Tyr Ser Arg Pro Val Val Ile Phe Asn Phe Ala Asn
       530                 535                 540
   Ser Ser Tyr Val Gln Tyr Gly Gln Leu Gly Glu Asp Asn Glu Ile Leu
   545                 550                 555                 560
   Leu Gly Asn His Arg Thr Glu Glu Cys Gln Leu Pro Ser Leu Lys Ile
                   565                 570                 575
   Phe Ile Ala Gly Asn Ser Ala Tyr Glu Tyr Val Asp Tyr Leu Phe Lys
               580                 585                 590
   Arg Met Ile Asp Leu Ser Ser Ile Ser Thr Val Asp Ser Met Ile Ala
           595                 600                 605
   Leu Asp Ile Asp Pro Leu Glu Asn Thr Asp Phe Arg Val Leu Glu Leu
       610                 615                 620
   Tyr Ser Gln Lys Glu Leu Arg Ser Ser Asn Val Phe Asp Leu Glu Glu
   625                 630                 635                 640
   Ile Met Arg Glu Phe Asn Ser Tyr Lys Gln Arg Val Lys Tyr Val Glu
                   645                 650                 655
   Asp Lys Val Val Asp Pro Leu Pro Pro Tyr Leu Lys Gly Leu Asp Asp
               660                 665                 670
   Leu Met Ser Gly Leu Gly Ala Ala Gly Lys Ala
           675                 680
<210> 15
   <211> 2049
   <212> DNA
   <213> Artificial Sequence
<220>
   <223> AD169 Cytomegalovirus
<400> 15
   atggaatccc ggatctggtg cctggtggtg tgcgtgaacc tgtgcatcgt gctcctggga 60
   gccctggccc accgggccaa cgagacaatc tacaacacca ccctgaagta cggcgacgtg 120
   gtgggagtga acaccaccaa gtacccctac agagtgtgct ccatggccca gggcaccgac 180
   ctgatcagat tcgagcggaa catcatctgc accagcatga agcccatcaa cgaggacctg 240
   gacgagggca tcatggtggt gtacaagagg aacatcgtgg cccacacctt caaggtccgc 300
   gtgtaccaga aggtgctgac cttcaggcgg agctacgccg gccacaggac cacctacctg 360
   ctgggcagca acaccgagta cgtggcccca cccatgtggg agatccacca catcaacaag 420
   ttcgcccagt gctacagcag ctacagccgc gtgatcggcg gcaccgtgtt cgtggcctac 480
   cacagggaca gctacgagaa caagaccatg cagctgatcc ccgacgacta cagcaacacc 540
   cacagcacca gatacgtgac cgtgaaggac cagtggcaca gcaggggcag caccgccttc 600
   cacagagaga catgcaacct gaactgcatg ctgaccatca ccaccgccag aagcaagtac 660
   ccttaccact tcttcgccac ctctaccggg gacgtggtgt acatcagccc cttctacaac 720
   ggcaccaaca ggaacgccag ctacttcggc gagaacgccg acaagttctt catcttcccc 780
   aactacacca tcgtgtccga cttcggcagg cccaacgctg cccccgagac acacagactg 840
   gtggcctttc tggagagggc cgacagcgtg atcagctggg acatccagga cgagaagaac 900
   gtgacctgcc agctgacctt ctgggaggcc agcgagagga ccatcagaag cgaggccgag 960
   gacagctacc acttcagcag cgccaagatg accgccacct tcctgagcaa gaaacaagag 1020
   gtgaacatga gcgacagcgc cctggactgc gtgagggacg aggccatcaa caagctgcag 1080
   cagatcttca acaccagcta caaccagacc tacgagaagt acggaaacgt gtccgtgttc 1140
   gagacaagcg gcggactggt ggtgttctgg cagggcatca agcagaagtc cctggtcgag 1200
   ctggagaggc tggccaacag gtccagcctg aacatcaccc acaccaccca gaccagcacc 1260
   agcgacaaca acaccacaca cctgagcagc atggaaagcg tgcacaacct ggtgtatgcc 1320
   cagctgcagt tcacctacga caccctgagg ggctacatca acagagccct ggcccagatc 1380
   gccgaggctt ggtgcgtgga ccagagaagg accctggagg tgttcaagga actgtccaag 1440
   atcaacccca gcgccatcct gagcgccatc tacaacaagc caatcgccgc cagattcatg 1500
   ggcgacgtgc tgggcctggc cagctgcgtg accatcaacc agaccagcgt gaaggtgctg 1560
   cgggacatga acgtgaagga aagcccaggc cgctgctact ccaggcccgt ggtgatcttc 1620
   aacttcgcca acagctccta cgtgcagtac ggccagctgg gcgaggacaa cgagatcctg 1680
   ctgggaaacc acaggaccga ggaatgccag ctgcccagcc tgaagatctt tatcgccggc 1740
   aactccgcct acgagtacgt ggactacctg ttcaagagga tgatcgacct gtccagcatc 1800
   agcaccgtgg acagcatgat cgccctggat atcgatcccc tggagaacac cgacttcagg 1860
   gtgctggagc tgtactccca gaaggaactg aggtccagca acgtgttcga cctggaggaa 1920
   atcatgagag agttcaacag ctacaagcag cgcgtgaagt acgtggagga caaggtggtg 1980
   gaccccctgc ccccctacct gaagggcctg gacgacctga tgagcggact cggggctgct 2040
   ggaaaggcc                                                         2049
<210> 16
   <211> 683
   <212> PRT
   <213> Artificial Sequence
<220>
   <223> AD169 Cytomegalovirus
<400> 16
   Met Glu Ser Arg Ile Trp Cys Leu Val Val Cys Val Asn Leu Cys Ile
    1               5                  10                  15
   Val Leu Leu Gly Ala Leu Ala His Arg Ala Asn Glu Thr Ile Tyr Asn
               20                  25                  30
   Thr Thr Leu Lys Tyr Gly Asp Val Val Gly Val Asn Thr Thr Lys Tyr
           35                  40                  45
   Pro Tyr Arg Val Cys Ser Met Ala Gln Gly Thr Asp Leu Ile Arg Phe
       50                  55                  60
   Glu Arg Asn Ile Ile Cys Thr Ser Met Lys Pro Ile Asn Glu Asp Leu
   65                  70                  75                  80
   Asp Glu Gly Ile Met Val Val Tyr Lys Arg Asn Ile Val Ala His Thr
                   85                  90                  95
   Phe Lys Val Arg Val Tyr Gln Lys Val Leu Thr Phe Arg Arg Ser Tyr
               100                 105                 110
   Ala Gly His Arg Thr Thr Tyr Leu Leu Gly Ser Asn Thr Glu Tyr Val
           115                 120                 125
   Ala Pro Pro Met Trp Glu Ile His His Ile Asn Lys Phe Ala Gln Cys
       130                 135                 140
   Tyr Ser Ser Tyr Ser Arg Val Ile Gly Gly Thr Val Phe Val Ala Tyr
   145                 150                 155                 160
   His Arg Asp Ser Tyr Glu Asn Lys Thr Met Gln Leu Ile Pro Asp Asp
                   165                 170                 175
   Tyr Ser Asn Thr His Ser Thr Arg Tyr Val Thr Val Lys Asp Gln Trp
               180                 185                 190
   His Ser Arg Gly Ser Thr Ala Phe His Arg Glu Thr Cys Asn Leu Asn
           195                 200                 205
   Cys Met Leu Thr Ile Thr Thr Ala Arg Ser Lys Tyr Pro Tyr His Phe
       210                 215                 220
   Phe Ala Thr Ser Thr Gly Asp Val Val Tyr Ile Ser Pro Phe Tyr Asn
   225 230 235 240
   Gly Thr Asn Arg Asn Ala Ser Tyr Phe Gly Glu Asn Ala Asp Lys Phe
   245 250 255
   Phe Ile Phe Pro Asn Tyr Thr Ile Val Ser Asp Phe Gly Arg Pro Asn
   260 265 270
   Ala Ala Pro Glu Thr His Arg Leu Val Ala Phe Leu Glu Arg Ala Asp
   275 280 285
   Ser Val Ile Ser Trp Asp Ile Gln Asp Glu Lys Asn Val Thr Cys Gln
   290 295 300
   Leu Thr Phe Trp Glu Ala Ser Glu Arg Thr Ile Arg Ser Glu Ala Glu
   305 310 315 320
   Asp Ser Tyr His Phe Ser Ser Ala Lys Met Thr Ala Thr Phe Leu Ser
   325 330 335
   Lys Lys Gln Glu Val Asn Met Ser Asp Ser Ala Leu Asp Cys Val Arg
   340 345 350
   Asp Glu Ala Ile Asn Lys Leu Gln Gln Ile Phe Asn Thr Ser Tyr Asn
   355 360 365
   Gln Thr Tyr Glu Lys Tyr Gly Asn Val Ser Val Phe Glu Thr Ser Gly
   370 375 380
   Gly Leu Val Val Phe Trp Gln Gly Ile Lys Gln Lys Ser Leu Val Glu
   385 390 395 400
   Leu Glu Arg Leu Ala Asn Arg Ser Ser Leu Asn Ile Thr His Thr Thr
   405 410 415
   Gln Thr Ser Thr Ser Asp Asn Asn Thr Thr His Leu Ser Ser Met Glu
   420 425 430
   Ser Val His Asn Leu Val Tyr Ala Gln Leu Gln Phe Thr Tyr Asp Thr
   435 440 445
   Leu Arg Gly Tyr Ile Asn Arg Ala Leu Ala Gln Ile Ala Glu Ala Trp
   450 455 460
   Cys Val Asp Gln Arg Arg Thr Leu Glu Val Phe Lys Glu Leu Ser Lys
   465 470 475 480
   Ile Asn Pro Ser Ala Ile Leu Ser Ala Ile Tyr Asn Lys Pro Ile Ala
   485 490 495
   Ala Arg Phe Met Gly Asp Val Leu Gly Leu Ala Ser Cys Val Thr Ile
   500 505 510
   Asn Gln Thr Ser Val Lys Val Leu Arg Asp Met Asn Val Lys Glu Ser
   515 520 525
   Pro Gly Arg Cys Tyr Ser Arg Pro Val Val Ile Phe Asn Phe Ala Asn
   530 535 540
   Ser Ser Tyr Val Gln Tyr Gly Gln Leu Gly Glu Asp Asn Glu Ile Leu
   545 550 555 560
   Leu Gly Asn His Arg Thr Glu Glu Cys Gln Leu Pro Ser Leu Lys Ile
   565 570 575
   Phe Ile Ala Gly Asn Ser Ala Tyr Glu Tyr Val Asp Tyr Leu Phe Lys
   580 585 590
   Arg Met Ile Asp Leu Ser Ser Ile Ser Thr Val Asp Ser Met Ile Ala
   595 600 605
   Leu Asp Ile Asp Pro Leu Glu Asn Thr Asp Phe Arg Val Leu Glu Leu
   610 615 620
   Tyr Ser Gln Lys Glu Leu Arg Ser Ser Asn Val Phe Asp Leu Glu Glu
   625 630 635 640
   Ile Met Arg Glu Phe Asn Ser Tyr Lys Gln Arg Val Lys Tyr Val Glu
   645 650 655
   Asp Lys Val Val Asp Pro Leu Pro Pro Tyr Leu Lys Gly Leu Asp Asp
   660 665 670
   Leu Met Ser Gly Leu Gly Ala Ala Gly Lys Ala
   675 680
<210> 17
   <211> 2031
   <212> DNA
   <213> Artificial Sequence
<220>
   <223> AD169 Cytomegalovirus
<400> 17
   atggcccccc tgctgcttct gctgcccctg ctttgggcag gggccctggc ccaccgggcc 60
   aacgagacaa tctacaacac caccctgaag tacggcgacg tggtgggagt gaacaccacc 120
   aagtacccct acagagtgtg ctccatggcc cagggcaccg acctgatcag attcgagcgg 180
   aacatcatct gcaccagcat gaagcccatc aacgaggacc tggacgaggg catcatggtg 240
   gtgtacaaga ggaacatcgt ggcccacacc ttcaaggtcc gcgtgtacca gaaggtgctg 300
   accttcaggc ggagctacgc cggccacagg accacctacc tgctgggcag caacaccgag 360
   tacgtggccc cacccatgtg ggagatccac cacatcaaca agttcgccca gtgctacagc 420
   agctacagcc gcgtgatcgg cggcaccgtg ttcgtggcct accacaggga cagctacgag 480
   aacaagacca tgcagctgat ccccgacgac tacagcaaca cccacagcac cagatacgtg 540
   accgtgaagg accagtggca cagcaggggc agcaccgcct tccacagaga gacatgcaac 600
   ctgaactgca tgctgaccat caccaccgcc agaagcaagt acccttacca cttcttcgcc 660
   acctctaccg gggacgtggt gtacatcagc cccttctaca acggcaccaa caggaacgcc 720
   agctacttcg gcgagaacgc cgacaagttc ttcatcttcc ccaactacac catcgtgtcc 780
   gacttcggca ggcccaacgc tgcccccgag acacacagac tggtggcctt tctggagagg 840
   gccgacagcg tgatcagctg ggacatccag gacgagaaga acgtgacctg ccagctgacc 900
   ttctgggagg ccagcgagag gaccatcaga agcgaggccg aggacagcta ccacttcagc 960
   agcgccaaga tgaccgccac cttcctgagc aagaaacaag aggtgaacat gagcgacagc 1020
   gccctggact gcgtgaggga cgaggccatc aacaagctgc agcagatctt caacaccagc 1080
   tacaaccaga cctacgagaa gtacggaaac gtgtccgtgt tcgagacaag cggcggactg 1140
   gtggtgttct ggcagggcat caagcagaag tccctggtcg agctggagag gctggccaac 1200
   aggtccagcc tgaacatcac ccacaccacc cagaccagca ccagcgacaa caacaccaca 1260
   cacctgagca gcatggaaag cgtgcacaac ctggtgtatg cccagctgca gttcacctac 1320
   gacaccctga ggggctacat caacagagcc ctggcccaga tcgccgaggc ttggtgcgtg 1380
   gaccagagaa ggaccctgga ggtgttcaag gaactgtcca agatcaaccc cagcgccatc 1440
   ctgagcgcca tctacaacaa gccaatcgcc gccagattca tgggcgacgt gctgggcctg 1500
   gccagctgcg tgaccatcaa ccagaccagc gtgaaggtgc tgcgggacat gaacgtgaag 1560
   gaaagcccag gccgctgcta ctccaggccc gtggtgatct tcaacttcgc caacagctcc 1620
   tacgtgcagt acggccagct gggcgaggac aacgagatcc tgctgggaaa ccacaggacc 1680
   gaggaatgcc agctgcccag cctgaagatc tttatcgccg gcaactccgc ctacgagtac 1740
   gtggactacc tgttcaagag gatgatcgac ctgtccagca tcagcaccgt ggacagcatg 1800
   atcgccctgg atatcgatcc cctggagaac accgacttca gggtgctgga gctgtactcc 1860
   cagaaggaac tgaggtccag caacgtgttc gacctggagg aaatcatgag agagttcaac 1920
   agctacaagc agcgcgtgaa gtacgtggag gacaaggtgg tggaccccct gcccccctac 1980
   ctgaagggcc tggacgacct gatgagcgga ctcggggctg ctggaaaggc c          2031
<210> 18
   <211> 677
   <212> PRT
   <213> Artificial Sequence
<220>
   <223> AD169 Cytomegalovirus
<400> 18
   Met Ala Pro Leu Leu Leu Leu Leu Pro Leu Leu Trp Ala Gly Ala Leu
    1               5                  10                  15
   Ala His Arg Ala Asn Glu Thr Ile Tyr Asn Thr Thr Leu Lys Tyr Gly
               20                  25                  30
   Asp Val Val Gly Val Asn Thr Thr Lys Tyr Pro Tyr Arg Val Cys Ser
           35                  40                  45
   Met Ala Gln Gly Thr Asp Leu Ile Arg Phe Glu Arg Asn Ile Ile Cys
       50                  55                  60
   Thr Ser Met Lys Pro Ile Asn Glu Asp Leu Asp Glu Gly Ile Met Val
   65                  70                  75                  80
   Val Tyr Lys Arg Asn Ile Val Ala His Thr Phe Lys Val Arg Val Tyr
                   85                  90                  95
   Gln Lys Val Leu Thr Phe Arg Arg Ser Tyr Ala Gly His Arg Thr Thr
               100                 105                 110
   Tyr Leu Leu Gly Ser Asn Thr Glu Tyr Val Ala Pro Pro Met Trp Glu
           115                 120                 125
   Ile His His Ile Asn Lys Phe Ala Gln Cys Tyr Ser Ser Tyr Ser Arg
       130                 135                 140
   Val Ile Gly Gly Thr Val Phe Val Ala Tyr His Arg Asp Ser Tyr Glu
   145                 150                 155                 160
   Asn Lys Thr Met Gln Leu Ile Pro Asp Asp Tyr Ser Asn Thr His Ser
                   165                 170                 175
   Thr Arg Tyr Val Thr Val Lys Asp Gln Trp His Ser Arg Gly Ser Thr
               180                 185                 190
   Ala Phe His Arg Glu Thr Cys Asn Leu Asn Cys Met Leu Thr Ile Thr
           195                 200                 205
   Thr Ala Arg Ser Lys Tyr Pro Tyr His Phe Phe Ala Thr Ser Thr Gly
       210                 215                 220
   Asp Val Val Tyr Ile Ser Pro Phe Tyr Asn Gly Thr Asn Arg Asn Ala
   225                 230                 235                 240
   Ser Tyr Phe Gly Glu Asn Ala Asp Lys Phe Phe Ile Phe Pro Asn Tyr
                   245                 250                 255
   Thr Ile Val Ser Asp Phe Gly Arg Pro Asn Ala Ala Pro Glu Thr His
               260                 265                 270
   Arg Leu Val Ala Phe Leu Glu Arg Ala Asp Ser Val Ile Ser Trp Asp
           275                 280                 285
   Ile Gln Asp Glu Lys Asn Val Thr Cys Gln Leu Thr Phe Trp Glu Ala
       290                 295                 300
   Ser Glu Arg Thr Ile Arg Ser Glu Ala Glu Asp Ser Tyr His Phe Ser
   305                 310                 315                 320
   Ser Ala Lys Met Thr Ala Thr Phe Leu Ser Lys Lys Gln Glu Val Asn
                   325                 330                 335
   Met Ser Asp Ser Ala Leu Asp Cys Val Arg Asp Glu Ala Ile Asn Lys
               340                 345                 350
   Leu Gln Gln Ile Phe Asn Thr Ser Tyr Asn Gln Thr Tyr Glu Lys Tyr
           355                 360                 365
   Gly Asn Val Ser Val Phe Glu Thr Ser Gly Gly Leu Val Val Phe Trp
       370                 375                 380
   Gln Gly Ile Lys Gln Lys Ser Leu Val Glu Leu Glu Arg Leu Ala Asn
   385                 390                 395                 400
   Arg Ser Ser Leu Asn Ile Thr His Thr Thr Gln Thr Ser Thr Ser Asp
                   405                 410                 415
   Asn Asn Thr Thr His Leu Ser Ser Met Glu Ser Val His Asn Leu Val
               420                 425                 430
   Tyr Ala Gln Leu Gln Phe Thr Tyr Asp Thr Leu Arg Gly Tyr Ile Asn
           435                 440                 445
   Arg Ala Leu Ala Gln Ile Ala Glu Ala Trp Cys Val Asp Gln Arg Arg
       450                 455                 460
   Thr Leu Glu Val Phe Lys Glu Leu Ser Lys Ile Asn Pro Ser Ala Ile
   465                 470                 475                 480
   Leu Ser Ala Ile Tyr Asn Lys Pro Ile Ala Ala Arg Phe Met Gly Asp
                   485                 490                 495
   Val Leu Gly Leu Ala Ser Cys Val Thr Ile Asn Gln Thr Ser Val Lys
               500                 505                 510
   Val Leu Arg Asp Met Asn Val Lys Glu Ser Pro Gly Arg Cys Tyr Ser
           515                 520                 525
   Arg Pro Val Val Ile Phe Asn Phe Ala Asn Ser Ser Tyr Val Gln Tyr
   530 535 540
   Gly Gln Leu Gly Glu Asp Asn Glu Ile Leu Leu Gly Asn His Arg Thr
   545 550 555 560
   Glu Glu Cys Gln Leu Pro Ser Leu Lys Ile Phe Ile Ala Gly Asn Ser
   565 570 575
   Ala Tyr Glu Tyr Val Asp Tyr Leu Phe Lys Arg Met Ile Asp Leu Ser
   580 585 590
   Ser Ile Ser Thr Val Asp Ser Met Ile Ala Leu Asp Ile Asp Pro Leu
   595 600 605
   Glu Asn Thr Asp Phe Arg Val Leu Glu Leu Tyr Ser Gln Lys Glu Leu
   610 615 620
   Arg Ser Ser Asn Val Phe Asp Leu Glu Glu Ile Met Arg Glu Phe Asn
   625 630 635 640
   Ser Tyr Lys Gln Arg Val Lys Tyr Val Glu Asp Lys Val Val Asp Pro
   645 650 655
   Leu Pro Pro Tyr Leu Lys Gly Leu Asp Asp Leu Met Ser Gly Leu Gly
   660 665 670
   Ala Ala Gly Lys Ala
   675
<210> 19
   <211> 661
   <212> PRT
   <213> Artificial Sequence
<220>
   <223> AD169 Cytomegalovirus
<400> 19
   Ser His Arg Ala Asn Glu Thr Ile Tyr Asn Thr Thr Leu Lys Tyr Gly
    1               5                  10                  15
   Asp Val Val Gly Val Asn Thr Thr Lys Tyr Pro Tyr Arg Val Cys Ser
               20                  25                  30
   Met Ala Gln Gly Thr Asp Leu Ile Arg Phe Glu Arg Asn Ile Ile Cys
           35                  40                  45
   Thr Ser Met Lys Pro Ile Asn Glu Asp Leu Asp Glu Gly Ile Met Val
       50                  55                  60
   Val Tyr Lys Arg Asn Ile Val Ala His Thr Phe Lys Val Arg Val Tyr
   65                  70                  75                  80
   Gln Lys Val Leu Thr Phe Arg Arg Ser Tyr Ala Gly His Arg Thr Thr
                   85                  90                  95
   Tyr Leu Leu Gly Ser Asn Thr Glu Tyr Val Ala Pro Pro Met Trp Glu
               100                 105                 110
   Ile His His Ile Asn Lys Phe Ala Gln Cys Tyr Ser Ser Tyr Ser Arg
           115                 120                 125
   Val Ile Gly Gly Thr Val Phe Val Ala Tyr His Arg Asp Ser Tyr Glu
       130                 135                 140
   Asn Lys Thr Met Gln Leu Ile Pro Asp Asp Tyr Ser Asn Thr His Ser
   145                 150                 155                 160
   Thr Arg Tyr Val Thr Val Lys Asp Gln Trp His Ser Arg Gly Ser Thr
                   165                 170                 175
   Ala Phe His Arg Glu Thr Cys Asn Leu Asn Cys Met Leu Thr Ile Thr
               180                 185                 190
   Thr Ala Arg Ser Lys Tyr Pro Tyr His Phe Phe Ala Thr Ser Thr Gly
           195                 200                 205
   Asp Val Val Tyr Ile Ser Pro Phe Tyr Asn Gly Thr Asn Arg Asn Ala
       210                 215                 220
   Ser Tyr Phe Gly Glu Asn Ala Asp Lys Phe Phe Ile Phe Pro Asn Tyr
   225                 230                 235                 240
   Thr Ile Val Ser Asp Phe Gly Arg Pro Asn Ala Ala Pro Glu Thr His
                   245                 250                 255
   Arg Leu Val Ala Phe Leu Glu Arg Ala Asp Ser Val Ile Ser Trp Asp
               260                 265                 270
   Ile Gln Asp Glu Lys Asn Val Thr Cys Gln Leu Thr Phe Trp Glu Ala
           275                 280                 285
   Ser Glu Arg Thr Ile Arg Ser Glu Ala Glu Asp Ser Tyr His Phe Ser
       290                 295                 300
   Ser Ala Lys Met Thr Ala Thr Phe Leu Ser Lys Lys Gln Glu Val Asn
   305                 310                 315                 320
   Met Ser Asp Ser Ala Leu Asp Cys Val Arg Asp Glu Ala Ile Asn Lys
                   325                 330                 335
   Leu Gln Gln Ile Phe Asn Thr Ser Tyr Asn Gln Thr Tyr Glu Lys Tyr
               340                 345                 350
   Gly Asn Val Ser Val Phe Glu Thr Ser Gly Gly Leu Val Val Phe Trp
           355                 360                 365
   Gln Gly Ile Lys Gln Lys Ser Leu Val Glu Leu Glu Arg Leu Ala Asn
       370                 375                 380
   Arg Ser Ser Leu Asn Ile Thr His Thr Thr Gln Thr Ser Thr Ser Asp
   385                 390                 395                 400
   Asn Asn Thr Thr His Leu Ser Ser Met Glu Ser Val His Asn Leu Val
                   405                 410                 415
   Tyr Ala Gln Leu Gln Phe Thr Tyr Asp Thr Leu Arg Gly Tyr Ile Asn
               420                 425                 430
   Arg Ala Leu Ala Gln Ile Ala Glu Ala Trp Cys Val Asp Gln Arg Arg
           435                 440                 445
   Thr Leu Glu Val Phe Lys Glu Leu Ser Lys Ile Asn Pro Ser Ala Ile
       450                 455                 460
   Leu Ser Ala Ile Tyr Asn Lys Pro Ile Ala Ala Arg Phe Met Gly Asp
   465                 470                 475                 480
   Val Leu Gly Leu Ala Ser Cys Val Thr Ile Asn Gln Thr Ser Val Lys
                   485                 490                 495
   Val Leu Arg Asp Met Asn Val Lys Glu Ser Pro Gly Arg Cys Tyr Ser
               500                 505                 510
   Arg Pro Val Val Ile Phe Asn Phe Ala Asn Ser Ser Tyr Val Gln Tyr
           515                 520                 525
   Gly Gln Leu Gly Glu Asp Asn Glu Ile Leu Leu Gly Asn His Arg Thr
       530                 535                 540
   Glu Glu Cys Gln Leu Pro Ser Leu Lys Ile Phe Ile Ala Gly Asn Ser
   545                 550                 555                 560
   Ala Tyr Glu Tyr Val Asp Tyr Leu Phe Lys Arg Met Ile Asp Leu Ser
                   565                 570                 575
   Ser Ile Ser Thr Val Asp Ser Met Ile Ala Leu Asp Ile Asp Pro Leu
               580                 585                 590
   Glu Asn Thr Asp Phe Arg Val Leu Glu Leu Tyr Ser Gln Lys Glu Leu
           595                 600                 605
   Arg Ser Ser Asn Val Phe Asp Leu Glu Glu Ile Met Arg Glu Phe Asn
       610                 615                 620
   Ser Tyr Lys Gln Arg Val Lys Tyr Val Glu Asp Lys Val Val Asp Pro
   625                 630                 635                 640
   Leu Pro Pro Tyr Leu Lys Gly Leu Asp Asp Leu Met Ser Gly Leu Gly
                   645                 650                 655
   Ala Ala Gly Lys Ala
               660
<210> 20
   <211> 660
   <212> PRT
   <213> Artificial Sequence
<220>
   <223> AD169 Cytomegalovirus
<400> 20
   His Arg Ala Asn Glu Thr Ile Tyr Asn Thr Thr Leu Lys Tyr Gly Asp
    1               5                  10                  15
   Val Val Gly Val Asn Thr Thr Lys Tyr Pro Tyr Arg Val Cys Ser Met
               20                  25                  30
   Ala Gln Gly Thr Asp Leu Ile Arg Phe Glu Arg Asn Ile Ile Cys Thr
           35                  40                  45
   Ser Met Lys Pro Ile Asn Glu Asp Leu Asp Glu Gly Ile Met Val Val
       50                  55                  60
   Tyr Lys Arg Asn Ile Val Ala His Thr Phe Lys Val Arg Val Tyr Gln
   65                  70                  75                  80
   Lys Val Leu Thr Phe Arg Arg Ser Tyr Ala Gly His Arg Thr Thr Tyr
                   85                  90                  95
   Leu Leu Gly Ser Asn Thr Glu Tyr Val Ala Pro Pro Met Trp Glu Ile
               100                 105                 110
   His His Ile Asn Lys Phe Ala Gln Cys Tyr Ser Ser Tyr Ser Arg Val
           115                 120                 125
   Ile Gly Gly Thr Val Phe Val Ala Tyr His Arg Asp Ser Tyr Glu Asn
       130                 135                 140
   Lys Thr Met Gln Leu Ile Pro Asp Asp Tyr Ser Asn Thr His Ser Thr
   145                 150                 155                 160
   Arg Tyr Val Thr Val Lys Asp Gln Trp His Ser Arg Gly Ser Thr Ala
                   165                 170                 175
   Phe His Arg Glu Thr Cys Asn Leu Asn Cys Met Leu Thr Ile Thr Thr
               180                 185                 190
   Ala Arg Ser Lys Tyr Pro Tyr His Phe Phe Ala Thr Ser Thr Gly Asp
           195                 200                 205
   Val Val Tyr Ile Ser Pro Phe Tyr Asn Gly Thr Asn Arg Asn Ala Ser
       210                 215                 220
   Tyr Phe Gly Glu Asn Ala Asp Lys Phe Phe Ile Phe Pro Asn Tyr Thr
   225                 230                 235                 240
   Ile Val Ser Asp Phe Gly Arg Pro Asn Ala Ala Pro Glu Thr His Arg
                   245                 250                 255
   Leu Val Ala Phe Leu Glu Arg Ala Asp Ser Val Ile Ser Trp Asp Ile
               260                 265                 270
   Gln Asp Glu Lys Asn Val Thr Cys Gln Leu Thr Phe Trp Glu Ala Ser
           275                 280                 285
   Glu Arg Thr Ile Arg Ser Glu Ala Glu Asp Ser Tyr His Phe Ser Ser
       290                 295                 300
   Ala Lys Met Thr Ala Thr Phe Leu Ser Lys Lys Gln Glu Val Asn Met
   305                 310                 315                 320
   Ser Asp Ser Ala Leu Asp Cys Val Arg Asp Glu Ala Ile Asn Lys Leu
                   325                 330                 335
   Gln Gln Ile Phe Asn Thr Ser Tyr Asn Gln Thr Tyr Glu Lys Tyr Gly
               340                 345                 350
   Asn Val Ser Val Phe Glu Thr Ser Gly Gly Leu Val Val Phe Trp Gln
           355                 360                 365
   Gly Ile Lys Gln Lys Ser Leu Val Glu Leu Glu Arg Leu Ala Asn Arg
       370                 375                 380
   Ser Ser Leu Asn Ile Thr His Thr Thr Gln Thr Ser Thr Ser Asp Asn
   385                 390                 395                 400
   Asn Thr Thr His Leu Ser Ser Met Glu Ser Val His Asn Leu Val Tyr
                   405                 410                 415
   Ala Gln Leu Gln Phe Thr Tyr Asp Thr Leu Arg Gly Tyr Ile Asn Arg
               420                 425                 430
   Ala Leu Ala Gln Ile Ala Glu Ala Trp Cys Val Asp Gln Arg Arg Thr
           435                 440                 445
   Leu Glu Val Phe Lys Glu Leu Ser Lys Ile Asn Pro Ser Ala Ile Leu
       450                 455                 460
   Ser Ala Ile Tyr Asn Lys Pro Ile Ala Ala Arg Phe Met Gly Asp Val
   465                 470                 475                 480
   Leu Gly Leu Ala Ser Cys Val Thr Ile Asn Gln Thr Ser Val Lys Val
                   485                 490                 495
   Leu Arg Asp Met Asn Val Lys Glu Ser Pro Gly Arg Cys Tyr Ser Arg
               500                 505                 510
   Pro Val Val Ile Phe Asn Phe Ala Asn Ser Ser Tyr Val Gln Tyr Gly
           515                 520                 525
   Gln Leu Gly Glu Asp Asn Glu Ile Leu Leu Gly Asn His Arg Thr Glu
       530                 535                 540
   Glu Cys Gln Leu Pro Ser Leu Lys Ile Phe Ile Ala Gly Asn Ser Ala
   545                 550                 555                 560
   Tyr Glu Tyr Val Asp Tyr Leu Phe Lys Arg Met Ile Asp Leu Ser Ser
                   565                 570                 575
   Ile Ser Thr Val Asp Ser Met Ile Ala Leu Asp Ile Asp Pro Leu Glu
               580                 585                 590
   Asn Thr Asp Phe Arg Val Leu Glu Leu Tyr Ser Gln Lys Glu Leu Arg
           595                 600                 605
   Ser Ser Asn Val Phe Asp Leu Glu Glu Ile Met Arg Glu Phe Asn Ser
       610                 615                 620
   Tyr Lys Gln Arg Val Lys Tyr Val Glu Asp Lys Val Val Asp Pro Leu
   625                 630                 635                 640
   Pro Pro Tyr Leu Lys Gly Leu Asp Asp Leu Met Ser Gly Leu Gly Ala
                   645                 650                 655
   Ala Gly Lys Ala
               660
<210> 21
   <211> 660
   <212> PRT
   <213> Artificial Sequence
<220>
   <223> AD169 Cytomegalovirus
<400> 21
   His Arg Ala Asn Glu Thr Ile Tyr Asn Thr Thr Leu Lys Tyr Gly Asp
    1               5                  10                  15
   Val Val Gly Val Asn Thr Thr Lys Tyr Pro Tyr Arg Val Cys Ser Met
               20                  25                  30
   Ala Gln Gly Thr Asp Leu Ile Arg Phe Glu Arg Asn Ile Ile Cys Thr
           35                  40                  45
   Ser Met Lys Pro Ile Asn Glu Asp Leu Asp Glu Gly Ile Met Val Val
       50                  55                  60
   Tyr Lys Arg Asn Ile Val Ala His Thr Phe Lys Val Arg Val Tyr Gln
   65                  70                  75                  80
   Lys Val Leu Thr Phe Arg Arg Ser Tyr Ala Gly His Arg Thr Thr Tyr
                   85                  90                  95
   Leu Leu Gly Ser Asn Thr Glu Tyr Val Ala Pro Pro Met Trp Glu Ile
               100                 105                 110
   His His Ile Asn Lys Phe Ala Gln Cys Tyr Ser Ser Tyr Ser Arg Val
           115                 120                 125
   Ile Gly Gly Thr Val Phe Val Ala Tyr His Arg Asp Ser Tyr Glu Asn
       130                 135                 140
   Lys Thr Met Gln Leu Ile Pro Asp Asp Tyr Ser Asn Thr His Ser Thr
   145                 150                 155                 160
   Arg Tyr Val Thr Val Lys Asp Gln Trp His Ser Arg Gly Ser Thr Ala
   165 170 175
   Phe His Arg Glu Thr Cys Asn Leu Asn Cys Met Leu Thr Ile Thr Thr
   180 185 190
   Ala Arg Ser Lys Tyr Pro Tyr His Phe Phe Ala Thr Ser Thr Gly Asp
   195 200 205
   Val Val Tyr Ile Ser Pro Phe Tyr Asn Gly Thr Asn Arg Asn Ala Ser
   210 215 220
   Tyr Phe Gly Glu Asn Ala Asp Lys Phe Phe Ile Phe Pro Asn Tyr Thr
   225 230 235 240
   Ile Val Ser Asp Phe Gly Arg Pro Asn Ala Ala Pro Glu Thr His Arg
   245 250 255
   Leu Val Ala Phe Leu Glu Arg Ala Asp Ser Val Ile Ser Trp Asp Ile
   260 265 270
   Gln Asp Glu Lys Asn Val Thr Cys Gln Leu Thr Phe Trp Glu Ala Ser
   275 280 285
   Glu Arg Thr Ile Arg Ser Glu Ala Glu Asp Ser Tyr His Phe Ser Ser
   290 295 300
   Ala Lys Met Thr Ala Thr Phe Leu Ser Lys Lys Gln Glu Val Asn Met
   305 310 315 320
   Ser Asp Ser Ala Leu Asp Cys Val Arg Asp Glu Ala Ile Asn Lys Leu
   325 330 335
   Gln Gln Ile Phe Asn Thr Ser Tyr Asn Gln Thr Tyr Glu Lys Tyr Gly
   340 345 350
   Asn Val Ser Val Phe Glu Thr Ser Gly Gly Leu Val Val Phe Trp Gln
   355 360 365
   Gly Ile Lys Gln Lys Ser Leu Val Glu Leu Glu Arg Leu Ala Asn Arg
   370 375 380
   Ser Ser Leu Asn Ile Thr His Thr Thr Gln Thr Ser Thr Ser Asp Asn
   385 390 395 400
   Asn Thr Thr His Leu Ser Ser Met Glu Ser Val His Asn Leu Val Tyr
   405 410 415
   Ala Gln Leu Gln Phe Thr Tyr Asp Thr Leu Arg Gly Tyr Ile Asn Arg
   420 425 430
   Ala Leu Ala Gln Ile Ala Glu Ala Trp Cys Val Asp Gln Arg Arg Thr
   435 440 445
   Leu Glu Val Phe Lys Glu Leu Ser Lys Ile Asn Pro Ser Ala Ile Leu
   450 455 460
   Ser Ala Ile Tyr Asn Lys Pro Ile Ala Ala Arg Phe Met Gly Asp Val
   465 470 475 480
   Leu Gly Leu Ala Ser Cys Val Thr Ile Asn Gln Thr Ser Val Lys Val
   485 490 495
   Leu Arg Asp Met Asn Val Lys Glu Ser Pro Gly Arg Cys Tyr Ser Arg
   500 505 510
   Pro Val Val Ile Phe Asn Phe Ala Asn Ser Ser Tyr Val Gln Tyr Gly
   515 520 525
   Gln Leu Gly Glu Asp Asn Glu Ile Leu Leu Gly Asn His Arg Thr Glu
   530 535 540
   Glu Cys Gln Leu Pro Ser Leu Lys Ile Phe Ile Ala Gly Asn Ser Ala
   545 550 555 560
   Tyr Glu Tyr Val Asp Tyr Leu Phe Lys Arg Met Ile Asp Leu Ser Ser
   565 570 575
   Ile Ser Thr Val Asp Ser Met Ile Ala Leu Asp Ile Asp Pro Leu Glu
   580 585 590
   Asn Thr Asp Phe Arg Val Leu Glu Leu Tyr Ser Gln Lys Glu Leu Arg
   595 600 605
   Ser Ser Asn Val Phe Asp Leu Glu Glu Ile Met Arg Glu Phe Asn Ser
   610 615 620
   Tyr Lys Gln Arg Val Lys Tyr Val Glu Asp Lys Val Val Asp Pro Leu
   625 630 635 640
   Pro Pro Tyr Leu Lys Gly Leu Asp Asp Leu Met Ser Gly Leu Gly Ala
   645 650 655
   Ala Gly Lys Ala
   660
<210> 22
   <211> 24
   <212> DNA
   <213> Artificial Sequence
<220>
   <223> AD169 Cytomegalovirus
<400> 22
   gaaagcgggc agtgagcgga aggc 24
<210> 23
   <211> 26
   <212> DNA
   <213> Artificial Sequence
<220>
   <223> AD169 Cytomegalovirus
<400> 23
   tgtcctccac gtacttcacg cgctgc 26
<210> 24
   <211> 26
   <212> DNA
   <213> Artificial Sequence
<220>
   <223> AD169 Cytomegalovirus
<400> 24
   cccgaacgac cgagcgcagc gagtca 26
<210> 25
   <211> 24
   <212> DNA
   <213> Artificial Sequence
<220>
   <223> AD169 Cytomegalovirus
<400> 25
   ccagctggcg aaagggggat gtgc 24
<210> 26
   <211> 228
   <212> DNA
   <213> Artificial Sequence
<220>
   <223> AD169 Cytomegalovirus
<400> 26
   atcgatcccc tggagaacac cgacttcagg gtgctggagc tgtactccca gaaggaactg 60
   aggtccagca acgtgttcga cctggaggaa atcatgagag agttcaacag ctacaagcag 120
   cgcgtgaagt acgtggagga caaggtggtg gaccccctgc ccccctacct gaagggcctg 180
   gacgacctga tgagcggact cggggctgct ggaaaggcct gaggatcc              228
<210> 27
   <211> 91
   <212> DNA
   <213> Artificial Sequence
<220>
   <223> AD169 Cytomegalovirus
<400> 27
   aatcaaaagc ttactagtgc cgccaccatg gcccccctgc tgcttctgct gcccctgctt 60
   tgggcagggg ccctggccca ccggggcaac g                                 91
<210> 28
   <211> 38
   <212> DNA
   <213> Artificial Sequence
<220>
   <223> AD169 Cytomegalovirus
<400> 28
   gtaataggat ccggtacctc atcaggcctt tccagcag 38
<210> 29
   <211> 42
   <212> DNA
   <213> Artificial Sequence
<220>
   <223> AD169 Cytomegalovirus
<400> 29
   aatcaaaagc ttactagtgc cgccaccatg gcccccctgc tg 42
<210> 30
   <211> 59
   <212> DNA
   <213> Artificial Sequence
<220>
   <223> AD169 Cytomegalovirus
<400> 30
   gacttatagg atcctcatca gtggtggtga tgatggtggc cgccggcctt tccagcagc 59
<210> 31
   <211> 40
   <212> DNA
   <213> Artificial Sequence
<220>
   <223> AD169 Cytomegalovirus
<400> 31
   gtgcatcgtg tgcctgggat ccgaggccgt gagccacagg 40
<210> 32
   <211> 40
   <212> DNA
   <213> Artificial Sequence
<220>
   <223> AD169 Cytomegalovirus
<400> 32
   cctgtggctc acggcctcgg atcccaggca cacgatgcac 40
<210> 33
   <211> 55
   <212> DNA
   <213> Artificial Sequence
<220>
   <223> AD169 Cytomegalovirus
<400> 33
   gtgaacctgt gcatcgtgtg cctggccctg gccagccacc gggccaacga gacaa 55
<210> 34
   <211> 55
   <212> DNA
   <213> Artificial Sequence
<220>
   <223> AD169 Cytomegalovirus
<400> 34
   ttgtctcgtt ggcccggtgg ctggccaggg ccaggcacac gatgcacagg ttcac 55
<210> 35
   <211> 55
   <212> DNA
   <213> Artificial Sequence
<220>
   <223> AD169 Cytomegalovirus
<400> 35
   cctgtgcatc gtgtgcctgg gagccctggc cagccaccgg gccaacgaga caatc 55
<210> 36
   <211> 55
   <212> DNA
   <213> Artificial Sequence
<220>
   <223> AD169 Cytomegalovirus
<400> 36
   gattgtctcg ttggcccggt ggctggccag ggctcccagg cacacgatgc acagg 55
<210> 37
   <211> 68
   <212> DNA
   <213> Artificial Sequence
<220>
   <223> AD169 Cytomegalovirus
<400> 37
   tggtgtgcgt gaacctgtgc atcgtgctcc tgggagccct ggcccaccgg gccaacgaga 60
   caatctac 68
<210> 38
   <211> 68
   <212> DNA
   <213> Artificial Sequence
<220>
   <223> AD169 Cytomegalovirus
<400> 38
   gtagattgtc tcgttggccc ggtgggccag ggctcccagg agcacgatgc acaggttcac 60
   gcacacca 68